# EUROPEAN PATENT SPECIFICATION

(11) **EP 3 841 097 B1**
(45) Date of publication and mention of the grant of the patent: **20.05.2026**
(21) Application number: 19762730.0
(22) Date of filing: 23.08.2019
(51) Int. Cl.: C07D 401/14, C07D 213/81, A61K 31/4439, A61P 35/00

(54) **2,6-BIS(((1H-BENZO[D]IMIDAZOL-2-YL)THIO)METHYL)PYRIDINE AND N2,N6-DIBENZYLPYRIDINE-2,6-DICARBOXAMIDE DERIVATIVES AND RELATED COMPOUNDS AS PHOSPHOINOSITIDE 3-KINASE (PI3K) INHIBITORS FOR TREATING CANCER**
2,6-BIS(((1H-BENZO[D]IMIDAZOL-2-YL)THIO)METHYL)PYRIDIN- UND N2,N6-DIBENZYLPYRIDIN-2,6-DICARBOXAMID-DERIVATE UND ÄHNLICHE VERBINDUNGEN ALS PHOSPHOINOSITIDE 3-KINASE (PI3K) INHIBITOREN ZUR BEHANDLUNG VON KREBS
DÉRIVÉS DE 2,6-BIS(((1H-BÉNZO[D]IMIDAZOL-2-YL)THIO)MÉTHYL)PYRIDINE ET N2,N6-DIBÉNZYLPYRIDINE-2,6-DICARBOXAMIDE ET COMPOSÉS SIMILAIRES EN TANT QU'INHIBITEURS PHOSPHOINOSITIDE 3-KINASE (PI3K) POUR LE TRAITEMENT DU CANCER

(30) Priority: 24.08.2018 GR 20180102981
(43) Date of publication of application: 30.06.2021
(73) Proprietor: Biomedical Research Foundation of the Academy of Athens (BRFAA), 115 27 Athens (GR); Agricultural University of Athens (AUA), 11855 Athens (GR); Foundation for Research and Technology - Hellas (Forth), 70013 Heraklion, Crete (GR); Cournia, Zoe, 11141 Athens (GR); Efstratiadis, Argiris, 10676 Athens (GR); Kapella, Anna, 16346 Ilioupoli (GR); Couladouros, Elias, 15451 Neo Psichico (GR)
(72) Inventor: CHRISTOFORIDIS, Savvas, 70013 Heraklion, Crete (GR); COURNIA, Zoe, 14564 Kifissia (GR); EFSTRATIADIS, Argiris, 10676 Athens (GR); KAPELLA, Anna, 16346 Ilioupoli (GR); COULADOUROS, Elias, 15451 Neo Psichico (GR)
(74) Representative: Weickmann & Weickmann PartmbB
(86) International application number: PCT/EP2019/072648
(87) International publication number: WO 2020/039097

(56) References cited:
- WO-A1-2018/057810
- WO-A2-2004/072027
- WO-A2-2014/110476
- WO-A2-2015/048547
- KR-A- 20150 066 786
- KR-A- 20160 011 727
- US-A1- 2003 199 560
- US-A1- 2005 255 415
- GAINES THERESA ET AL: "Synthesis and evaluation of 2,5 and 2,6 pyridine-based CXCR4 inhibitors", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 24, no. 21, 20 August 2016 (2016-08-20), pages 5052 - 5060, XP029763079, ISSN: 0968-0896, DOI: 10.1016/J.BMC.2016.08.018
- AFSAR ALI ET AL: "Syntheses, characterization, and anti-cancer activities of pyridine-amide based compounds containing appended phenol or catechol groups", JOURNAL OF CHEMICAL SCIENCES, vol. 126, no. 4, July 2014 (2014-07-01), India, pages 1091 - 1105, XP055573184, ISSN: 0974-3626, DOI: 10.1007/s12039-014-0671-3
- PENNARUN G ET AL: "Apoptosis related to telomere instability and cell cycle alterations in human glioma cells treated by new highly selective G-quadruplex ligands", ONCOGENE, NATURE PUBLISHING GROUP UK, LONDON, vol. 24, no. 18, 21 April 2005 (2005-04-21), pages 2917 - 2928, XP002773925, ISSN: 0950-9232, DOI: 10.1038/SJ.ONC.1208468
- ANDREW N. DWYER ET AL: "Binding of Co 3+ and within a Pyridine-cored Molecular Cleft", SUPRAMOLECULAR CHEMISTRY., vol. 16, no. 6, September 2004 (2004-09-01), US, pages 405 - 410, XP055634383, ISSN: 1061-0278, DOI: 10.1080/10610270410001713312

## Description

### FIELD OF THE INVENTION

The invention relates to compounds for use in treating or preventing cancerous diseases, caused by or triggered by a phosphoinositide 3-kinase mutation, in a mammal carrying a mutation in the gene encoding PI3Kα, preferably in humans.

### BACKGROUND OF THE INVENTION

Carcinogenesis is a process by which normal cells are transformed into cancer cells. It is characterized by a progression of changes at the cellular, genetic, and epigenetic level that ultimately reprogram a cell to undergo uncontrolled cell division, thus mostly forming a malignant mass. Though it is well-established that carcinogenesis is largely the result of irregular activation of oncogenes and/or inactivation of tumor-suppressors, which lead to various pathological changes, the reasons of carcinogenesis are various or not yet known. Furthermore, carcinogenesis pathways involve many genes, factors and conditions, which themselves and their interactions remain very complicated or unclear. Consequently, the development of anti-cancer drugs is largely based on the stroke of luck by trial-and-error, with the society still suffering under the lack of effective anti-cancer medications.

Most drugs on the market are designed to bind directly to primary active sites (also known as orthosteric sites) of their biological targets. Allosteric modulators offer important advantages over orthosteric ones: they can be more selective because allosteric sites are less conserved (Bagal et al, J. Med. Chem., 62:247-265 (2019)), they can change protein levels or localization within the cell change (Generoso et al, Nat. Chem. Biol., 11:280-286 (2015)) subtle aspects of protein function (e.g. form or conformation of the quaternary structure (Zheng et al. J. Am. Chem. Soc., 140:5914-5924(2018); Shibayama et al., J. Biol. Chem., 292:18258-18269(2017)) and are unique in their ability to provide enzymatic activation (Milne et al. Nature, 450:712-716 (2007)). Furthermore, allosteric drugs can be used synergistically with orthosteric ligands, an approach that has been successfully used to prevent emergence of resistance in cancer therapy (Wylie et al. Nature, 543:733-737 (2017)). Allostery may also be the key to drug proteins that have been considered undruggable due to the absence of a known active site, such as KRAS. Allosteric targeting can also be reached via conformationally specific allosteric antibodies, which represents an important field of future research. There are already clear examples of monoclonal antibodies that allosterically target ion channels (Lee et al. Cell, 157:1393-1404 (2014)), GPCRs (Mukund et al. J. Biol. Chem., 288:36168-36178 (2013)), as well as cytokine and integrin receptors (Rizk et al. Cell Commun. Signal., 13:1-5 (2015); Schwarz et al. Circ. Residual., 99:25-33 (2006)). Importantly, allosteric modulators have the potential to combat drug-resistant mutations situated at orthosteric sites in patients. For example, the "gatekeeper mutation" T315I of BCR-ABL1 causes resistance to a set of clinically approved orthosteric drugs such as imatinib, bosutinib, nilotinib, and dasatinib. Treatment of the T315I mutant with an allosteric inhibitor ABL001 (asciminib) bound to the myristoyl pocket at the C-lobe of the kinase can overcome drug resistance (Schoepfer et al. J Med Chem 61:8120-8135 (2018);Wylie et al. Nature, 543:733-737 (2017)).

To date, the discovery of allosteric binding sites and lead compounds targeting those sites has been mostly serendipitous, achieved through high-throughput screening and subsequent follow up experimental work on the mechanism of action. The current lack of a suitable theoretical background to explain allostery in different protein classes, experimental data, and simulation technology capable to implement this drug design strategy, leads to high development costs of allosteric candidate drugs, even though over the past decade, a wealth of protein structural data has become readily available including many membrane protein classes (e.g., GPCRs and ion channels), which are common allosteric drug targets and, in parallel, improved simulation methods now provide better atomistic understanding of the protein dynamics and cooperative motions that are critical to allosteric mechanisms.

The PI3K-AKT-mTOR is the most frequently activated signaling pathway in breast cancer (Miller et al. Breast Cancer Res, 13:224 (2011)), and has been validated as a therapeutic target in Phase III trials (André et al., Lancet Oncol 15:267-274 (2014)). Furthermore, one of the signalling pathways which, when deregulated, becomes centrally involved in colon, mammary, and endometrial tumorigenesis is served by phosphoinositide-3'-kinase alpha (PI3Kα), which is one of the four PI3K isoforms (Engelman et al., Nat Rev Genet 7:606-619 (2006); Massacesi et al., Onco Targets Ther 9: 203-210 (2016)). PI3Kα is comprised of a catalytic subunit, p110α, encoded by the *PIK3CA* gene, and a regulatory subunit, p85α, encoded by the *PIK3R1* gene. Deregulation of the PI3Kα pathway has long been associated with cancer development, but only during the last 10 years was it discovered that mutations commonly occurring in *PIK3CA* are oncogenic (Liu P et al., Nat Rev Drug Disc, 8:627-644 (2009)).

PIK3CA is mutated in a breadth of cancers, with the most notable incidents in breast cancer, where PIK3CA is the most frequently mutated oncogene, harboring genetic alterations in ~27% of the cases on average, in endometrial cancer in 24% of the cases, and in colon cancer in ~15% of the cases (Liu P et al., Nat Rev Drug Disc, 8:627-644 (2009)). PIK3CA mutations are found in a variety of cancers and more specifically in: breast cancer, colon cancer, endometrial cancer, brain tumors, skin cancer, ovarian cancer, gastric cancer, lung cancer, thyroid cancer, head and neck cancer, cervical cancer, pancreatic cancer, liver/biliary tract cancer, pituitary tumors, urological tumors, leukemia/lymphoma, neuroblastoma (Samuels and Waldman, Curr Top Microbiol Immunol, 347:21-41 (2010)). 80% of these mutations result in amino acid replacements located in either one of *two hotspots:* (a) in the helical domain of PI3Kα the most common replacement is that of Glu by Lys in exon 9 (E545K), (b) in the kinase domain, a His is changed to Arg in exon 20 (H1047R) (Miled et al., Science 317:239-42 (2007)). Both types of these mutations increase the kinase activity of the enzyme, upregulate the downstream AKT pathway and VEGF signaling, and stimulate cell transformation, tumorigensesis and angiogenesis (Samuels et al., Cancer Cell 7:561-572 (2005); Parsons Clin Cancer Res 11:7965-7966 (2005), Hu et al., Clin Cancer Res 11:8208-8212 (2005). A list of human cancers bearing the H1047R mutations can be found in Thorpe et al., Nat Rev Cancer 15(1):7-24 (2015) and in Samuels and Waldmann, Curr Top Microbiol Immunol 347:21-41 (2010).

The PI3K pathway is implicated in resistance to chemotherapy, radiotherapy, hormone therapy, and targeted agents (Massacesi et al., Onco Targets Ther 9: 203-210 (2016)). PI3K inhibitors may thus restore sensitivity to cancer treatments when administered as part of combination therapies (Burris HA III, Cancer Chemother Pharmacol. 71:829-842 (2013)).

International patent application WO 2018/057810 A1 discloses 1H-benzo[d]imidazole derivatives as PI3K inhibitors for treating cancer.

A number of PI3K inhibitors are currently under clinical investigation, including pan-PI3K inhibitors targeting all four isoforms of class I PI3K, as well as the PI3Kα-selective inhibitor alpelisib (BYL719), which is in Phase III clinical trials for postmenopausal women with advanced breast cancer, which progressed (Dienstmann et al., Mol Cancer Ther 13(5):1021-1031 (2014); https://clinicaltrials.gov/ct2/show/NCT02437318). In early clinical studies, alpelisib exhibited favorable tolerability profiles, with adverse events consistent with "on-target" inhibition of PI3K (Massacesi et al., Onco Targets Ther 9:203-210 (2016)).

In May 24 2019 alpelisib received FDA approval as the first PI3K inhibitor indicated for the treatment of hormone receptor (HR)-positive, human epidermal growth factor receptor 2 (HER2)-negative, PIK3CA-mutated, advanced or metastatic breast cancer in combination with fulvestrant for postmenopausal women and male patients. To initiate alpelisib therapy, it is required that the presence of a PIK3CA mutation in the tissue and/or liquid biopsy sample collection should be confirmed via FDA-approved diagnostic tests. Alpelisib is an inhibitor of the active site of PI3Kα, which exploits differences between the active sites of PI3Kβ, γ, and δ isoforms to achieve its specificity. However, alpelisib is an inhibitor of the active site of PI3Kα, where Gerspacher et al., Bioorg Med Chem Lett.; 25:3582-4 (2015) converted the 5-(pyridin-4-yl)thiazol-2-amino bicyclic core scaffold of BYL719 (341) into novel 4H-thiazolo[5',4':4,5 ]pyrano [2,3-c]pyridine tricyclic scaffold ( 342), via an oxygen as the linker. Activity result showed that 343 (PI3Kα/β/δ/γ IC₅₀ = 5/670/220/200 nM) exhibited similar or better biochemical potency, selectivity against PI3Ka and cellular activity, as well as favorable pharmacokinetic properties due to enhanced solubility, compared to noncyclized analogs of BYL719 (341, PI3Kα/β/δ/γ IC₅₀ = 5/1200/290/250 nM). The crystal structure of p110a bound to the potent and selective inhibitor alpelisib (BYL719) has been published in (Furet et al., Bioorg Med Chem Lett.; 23(13):3741-8 (2013)). The amide hydrogen bond between the hinge residue and the inhibitor is colored in red for all panels. This hydrogen bond mimics the hydrogen bond made between the adenine ring of ATP with the hinge region of the kinase domain. Specificity for p110a over other PI kinases is primarily driven through the unique hydrogen bond interaction BYL719 can make with the non-conserved p110a residue Q859.

A key challenge in targeting the PI3K pathway is the identification of isoform-selective inhibitors because the four PI3K isoforms are involved in distinct cellular processes, including glucose homeostasis and the immune response (Knight et al, Cell 125:733-747 (2006). The high similarity of the four PI3K isoforms in the adenosine triphosphate (ATP) binding pocket often leads to the development of non-selective PI3K drug candidates, which may yield undesirable side effects. Targeting a single PI3K isoform in patients may allow administration at therapeutic doses without being limited by toxicities associated with inhibiting multiple isoforms. Although alpelisib has achieved some potent selectivity, active sites are prone to mutations which leads eventually to drug resistance. Allosteric modulators offer a novel approach for selective kinase inhibition because they target less conserved binding sites compared to the active site; thus, higher selectivity may be obtained (Changeux, Drug Disc Today 10:e223-228 (2013)).

Hence, there is still a need in the art for efficient and selective therapeutics that can prevent or inhibit tumor growth in mammals, in particular in humans.

The 3D structure of the human (Huang et al., Science 318:1744-1748 (2007)) and mouse (Hon et al., Oncogene 31:3655-3666 (2012)) catalytic subunit p110α as well as the structure of the human H1047R PI3Kα mutant (Mandelker et al., Proc Natl Acad Sci U S A 106:16996-17001 (2009)) have been solved by X-ray crystallography. Recent experimental data demonstrate that the H1047R PI3Kα mutation overactivates the enzyme by inducing dynamic changes in the kinase domain, which increase basal ATPase activity as well as expose the membrane binding regions, thereby augmenting basal membrane binding (Huang et al., Science 318:1744-1748 (2007); Mandelker et al., Proc Natl Acad Sci U S A 106:16996-17001 (2009)).

The inventors have developed an advanced targeted therapeutic approach of great specificity, exploiting distinct structural features of the mutant and wild-type PI3Kα proteins for the design of novel inhibitors. The inventors' recent studies have shown that it would be possible to target the H1047R mutated PI3Kα selectively by taking into account the dynamical and structural differences between the wild-type (WT) and mutant proteins (Gkeka et al., PLoS Comput Biol 10:e1003895 (2014); Lionta et al., Curr Top Med Chem 14:1923-1938 (2014); Gkeka et al., J Phys Chem B 119:1002-1016 (2015)). These studies were utilized for the identification of new binding pockets and the design of mutant-specific PI3Kα inhibitors to exploit the altered mutant conformation.. A recent crystal structure (Hon et al, Oncogene 31:3655-66 (2012)) confirmed the binding of a known PI3K inhibitor, PIK-108, in one of the binding pockets identified. Using advanced computational and mathematical techniques in combination with in vitro experiments, the inventors showed that binding to this specific binding pocket does not induce any allosteric effects to PI3Kα (Gkeka et al, J Phys Chem B 119:1002-1016 (2015). They further investigated other binding pockets in the vicinity of the mutation site and examined if compounds binding there could possibly act as allosteric inhibitors, i.e. regulate the protein by binding at a site other than the protein active site.

This approach aims to treat cancer by using small-molecule inhibitors acting only against oncogenic mutant forms of PIK3CA and being selective against the wild-type protein. The invention employs an array of computational, rational drug design, organic synthesis, biochemical *in vitro* assays and *in vivo* studies in xenografts and genetically modified mouse models.

Initially, the full-length catalytic p110α subunit of the WT and H1047R mutant PI3Kα was modeled using Molecular Dynamics simulations. Using data from the simulations, representative conformations for the PI3Kα WT protein and for the mutant H1047R were calculated. These conformations were further exploited to identify allosteric pockets for selective inhibition of the mutant protein (Gkeka et al., PLoS Comput Biol 10:e1003895 (2014); Gkeka et al., J Phys Chem B 119:1002-1016 (2015)). The identified binding sites were confirmed for allosteric communication with the active (ATP) site of PI3Kα using positional covariance matrices, with which the inventors measured the correlation of motions between the residues of each identified binding site and the active PI3Kα site. One cavity in the vicinity of the active site was confirmed as allosteric and was further used in de novo computer-aided drug design. Subsequent post-processing of top-scoring compounds for toxicity, pose viability, chemical diversity, and physicochemical properties led to identifying an initial lead compound and synthesizing several of its analogs.

PI3Kα activity assays on the identified lead compound and its analogs were performed using PIP2 containing liposomes (Gkeka et al., PLoS Comput Biol 10:e1003895 (2014)). Complexes of PIK3CA (WT or H10147R)/PIK3R1 were obtained from Millipore. The assay was based on a protocol provided by Millipore, with modifications (Papafotika et al., *in preparation*) which will be further described below. In this assay, PIP3 molecules produced by PI3Kα compete with biotinylated PIP3 for binding with a recombinant GST-GRP1-PH domain (amino acids 263- 380), which has been produced in bacteria, bound to glutathione coated 96-well plates. Quantitation of the competed amount of biotin-PIP3 is estimated by the peroxidase activity of streptavidin-HRP that binds to the plate and provides a measure for the activity of the protein. The compounds were preincubated with all the components of the assay mixture, except PIP2, for 10 min, at 25 ° C. PIP2 is added in the end of the preincubation period, thereby initiating the kinase reaction. IC50 values were calculated from dose- response curves using logit-log plots. All assays were carried out in at least three independent experiments and each concentration in triplicate. PIK-108 and wortmannin were used as controls (Gkeka et al., J Phys Chem B 119:1002-1016 (2015)).

Xenografts are *in vivo* models of cancer, where cells from a cancer cell line are implanted into an immunodeficient mouse. Xenograft models are used to create an environment that allows for the natural growth of cancer, its monitoring, and corresponding treatment evaluations. Herein, the inventors used immunodeficient (SCID) mice with heterotopic xenografts after subcutaneous injection of cells from the breast cancer cell line MDA-MB-231, which is wild-type PIK3CA and of the breast cancer cell line HCC1954, which bears the exon 20 H1047R mutation on PIK3CA.

In order to test the *in vivo* activity of potential anticancer drugs, establishing a corresponding cancer animal model is required. To generate mouse tumors at chosen anatomical sites, a variant of a genetic scheme that is based on Cre / loxP recombination is often used. Depending on the tissue-specificity of the promoter driving Cre expression, corresponding tumors develop in progeny derived from matings between Cre-producer mice and Cre-responder partners carrying a dormant oncogenic transgene, which becomes functional after excision of an upstream-positioned floxed DNA segment that blocks its expression [see Politi et al. Oncogene 23:1558-1565 (2004); Klinakis et al. Proc Natl Acad Sci U S A 106:2359-2364 (2009), Stratikopoulos et al. Cancer Cell 27:837-851 (2015)). To develop mouse strains carrying Pik3ca gain of function mutations (hereafter referred to as Pik3ca*), a knock-in gene targeting strategy was followed (Stratikopoulos et al. Cancer Cell 27:837-851 (2015)). Thus, either of the two hotspot mutations of the Pik3ca locus (in exon 20 or in exon 9) was targeted so that the mutated Pik3ca would be expressed from the endogenous locus and not from a transgene, thus, mimicking the expression found in human tumors. Activation of the exon 20 Pik3ca oncogenic mutation was achieved after mating the animals with WAPCre producers (WAP is a milk protein, the promoter of which is used for driving Cre expression, specifically in the mammary gland). Mammary tumors appeared in such animals after a very long latency of 490 days (median). However, mating of the WAPCre/exon20 Pik3ca* with MMTV-Myc expressing mice (which by themselves develop mammary tumors at 5-7 months) an acceleration in the appearance of tumors was observed in the birth-giving female mice with genotype WAPCre/exon20 Pik3ca* / MMTV-Myc (1 week following parturition). These mice were used to test the efficacy of compounds discussed herein.

It was an object of the present invention to identify compounds for use in treating or preventing cancerous diseases in a mammal based on a relevant biological activity demonstrated in *in vitro* biochemical assays and a non-human transgenic animal model. It was a further object of the invention to provide compounds and compositions for use in treating or preventing cancerous diseases in a mammal.

### SUMMARY OF THE INVENTION

The present invention defined in the appended claims generally refers to compounds for use in treating a cancerous disease caused by or triggered by a PI3Kα in a mammal, wherein the mammal to be treated carries a mutation in the gene encoding PI3Kα which leads to the occurrence of a PI3Kα mutant, and wherein the mammal is preferably a human. Compounds for use according to the invention have a substituted benzyl or pyridine scaffold. They are compounds of formula (I): 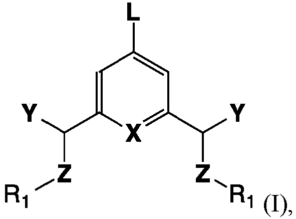
a tautomer, polymorph, hydrate, solvate, or a pharmaceutically acceptable salt thereof,
wherein
L is independently H or OH;
X is independently N or CH;
Y is independently H or =O;
Z is independently S or NH;
R1 is independently mono- or polycyclic aryl or heteroaryl selected from: 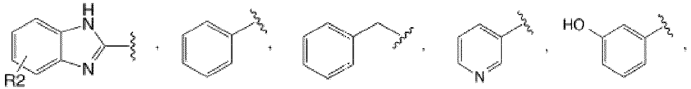 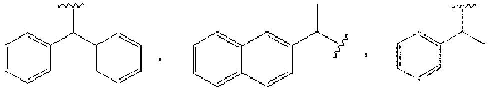 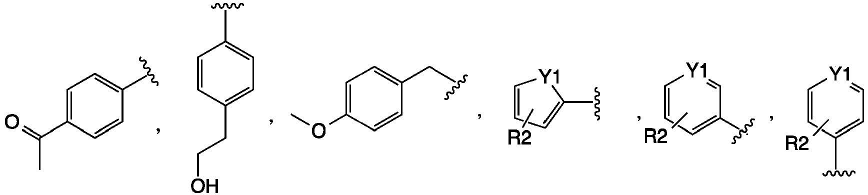
R₂ is independently at least one of hydroxyl, hydrogen, fluoro, chloro, or bromo; and In the five-membered rings, Y₁ is independently CH₂, O, S, or NH, whereas in the six-membered rings, Y₁ is independently CH or N.
In the context of the above definitions and definitions contained at other places of this specification, R₂ can also be absent as a substituent, i.e. its presence is optional. Furthermore, more than one of hydroxyl, hydrogen, fluoro, chloro or bromo substituents can be included as substituents in the formulas depending on the exact chemical structure.
Compounds falling under the definition of formula (I) or similar compounds were known in the art. For example, Korean patent applications KR 2016/0011727 A, and KR 2015/0066786, international applications WO 2015/048547 A2, and WO 2004/072027 A2, US patent application No. 2003/199560 A1, Gaines at al., (Medicinal Chemistry and Related Disciplines, 2016, vol.24, no.21, p.5052-5060), Afsar et al. (J. Chem. Soc. 2014, vol.126, no.4, p.1091-1105), and Pennarun et al. (Oncogene 2005, vol.24, no.18, p.2917-2928) disclose such compounds. However, to date the compounds have not been used for the specific treatment purpose of the invention and it was not recognized that they are specifically suitable for the treatment of cancerous diseases caused or triggered by a PI3Kα in a mammal, wherein the mammal to be treated carries a mutation in the gene encoding PI3Kα which leads to the occurrence of a PI3Kα mutant.
The invention provides the above substances for use in treating or preventing cancerous diseases caused by or triggered by PI3Kα mutation as defined in claim 1.

In a preferred embodiment, formula (I) refers to a compound having the following formula (II): 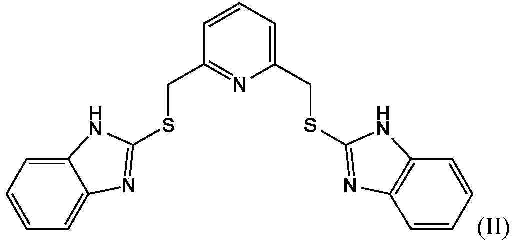 or a pharmaceutically acceptable salt thereof. This compound is also referred to herein with its internal name PI3K-010.

In another preferred embodiment, formula (I) refers to a compound having the following formula (III): 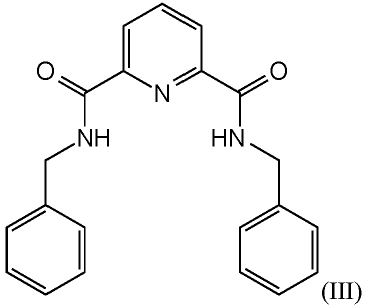 or a pharmaceutically acceptable salt thereof. This compound is also referred to herein with its internal name PI3K-021.

In another preferred embodiment, formula (I) refers to a compound having the following formula (IV): 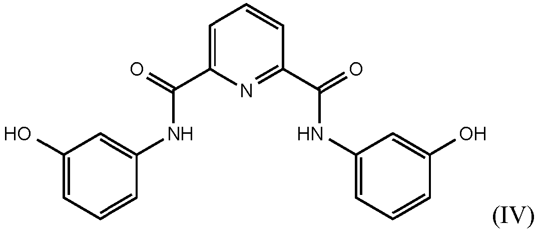 or a pharmaceutically acceptable salt thereof. This compound is also referred to herein with its internal name PI3K-024.

The present document further refers to a pharmaceutical composition comprising one or more compounds of formula (I) as defined above, and preferably compounds II and/or III. Optionally, the pharmaceutical composition may further comprise one or more additional therapeutic agents, for example additional, otherwise known, anti-cancer agents. This pharmaceutical composition can be provided for use in treating or preventing cancerous diseasesas described herein.

The above pharmaceutical compositions may further comprise one or more additional therapeutic agents, such as COX-2 oxygenase inhibitors.

Another aspect of the described in the present document but not claimed relates to a method for the manufacture of the above compounds of formula (I): 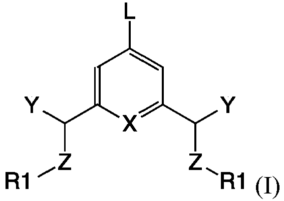 wherein the method comprises of first synthesizing chlorides with general formula (V): 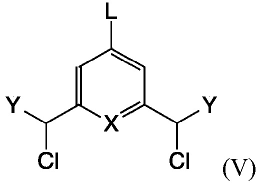 where in X, L, Y are defined as above, which then react with a moiety R₁-Z-H as defined for formula (I) above to yield a compound of formula (I) as depicted above. Methods for preparing compounds of formula (I) are disclosed for example in international application WO 2014/11076 A2, US patent application No. 2005/255415 A1, and Dwyer et al. (Supramolecular Chemistry 2004, vol.16, no.6, p.405-410).

A still further aspect described in the present document but not claimed relates to an assay for determining PI3Kα or PI3Kα mutant activity in a competitive immunoassay.

### BRIEF DESCRIPTION OF THE FIGURES

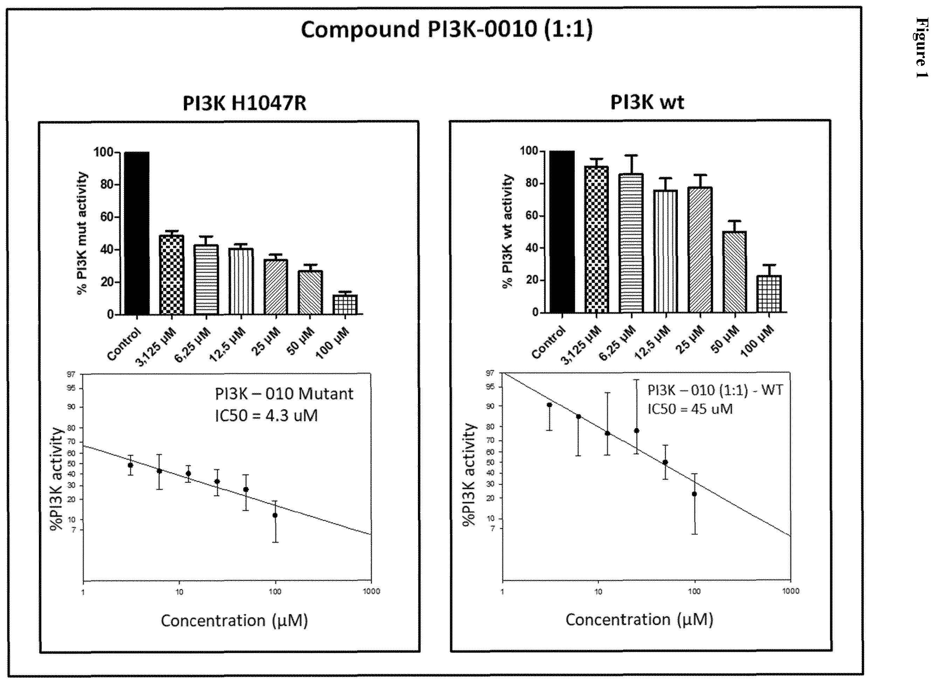

**Figure 1****.** *In vitro* PI3Kα activity assays demonstrating an 11-fold selectivity of PI3K-010 for H1047R PI3Kα. IC50s were estimated using the logit-log graph of SigmaPlot and linear regression analysis. Error bars show the standard error of the mean from at least three independent experiments, each one performed in triplicates.

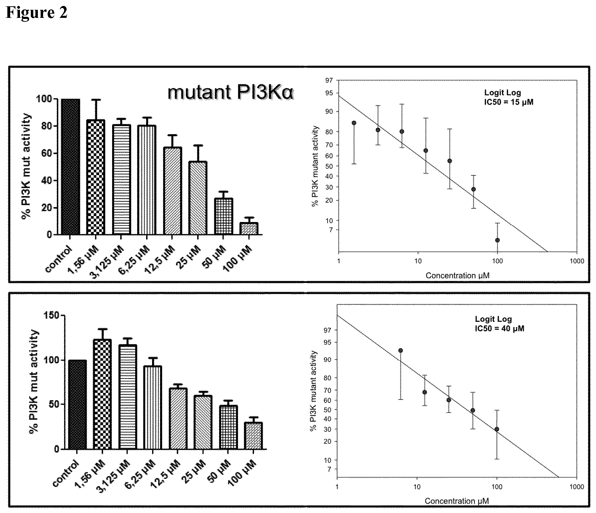

**Figure 2****.** Results of an experiment testing competition between ATP and PI3K-010 for the active site of PI3Kα. The activity of PI3Kα was assayed using a standard concentration of 100 µM ATP (upper panel) as well as a 20-fold higher concentration of ATP (lower panel), at increasing concentrations PI3K-010. IC50s were estimated using the logit-log graph of SigmaPlot and linear regression analysis. Error bars show the standard error of the mean from at least three independent experiments, each one performed in triplicates. The IC50 of PI3K-010 is not significantly altered in the presence of high ATP concentration, indicating that this compound is a non-competitive inhibitor of PI3Kα.

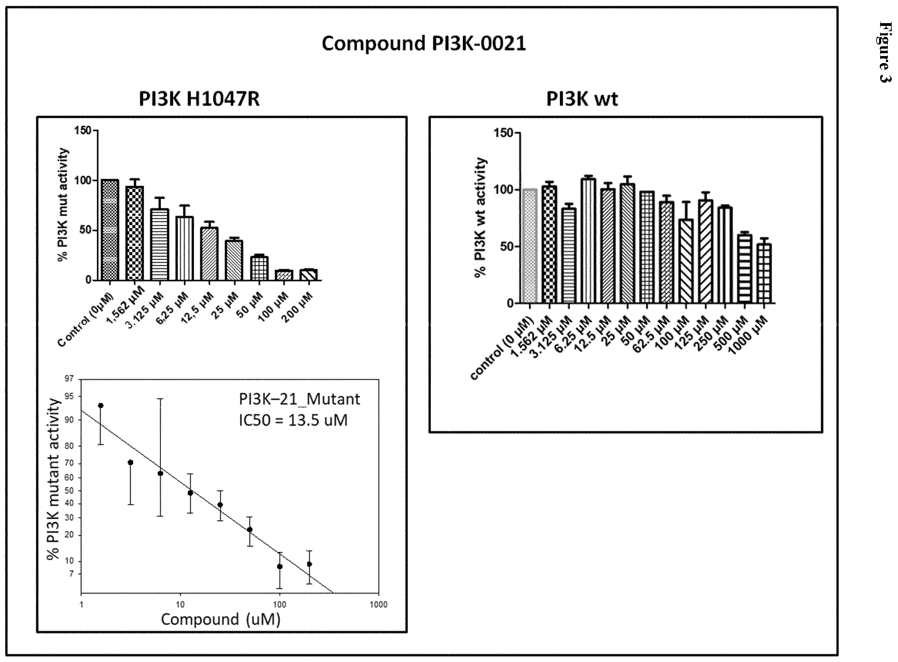

**Figure 3****.** *In vitro* PI3Kα activity assays illustrating the selectivity of PI3K-021 for H1047R PI3Kα. The IC50 of PI3K-021 against the mutant H1047R PI3Kα was estimated using the logit-log graph of SigmaPlot and linear regression analysis. Error bars show the standard error of the mean from at least three independent experiments, each one performed in triplicates. The IC50 against the WT PI3Kα was estimated by extrapolation.

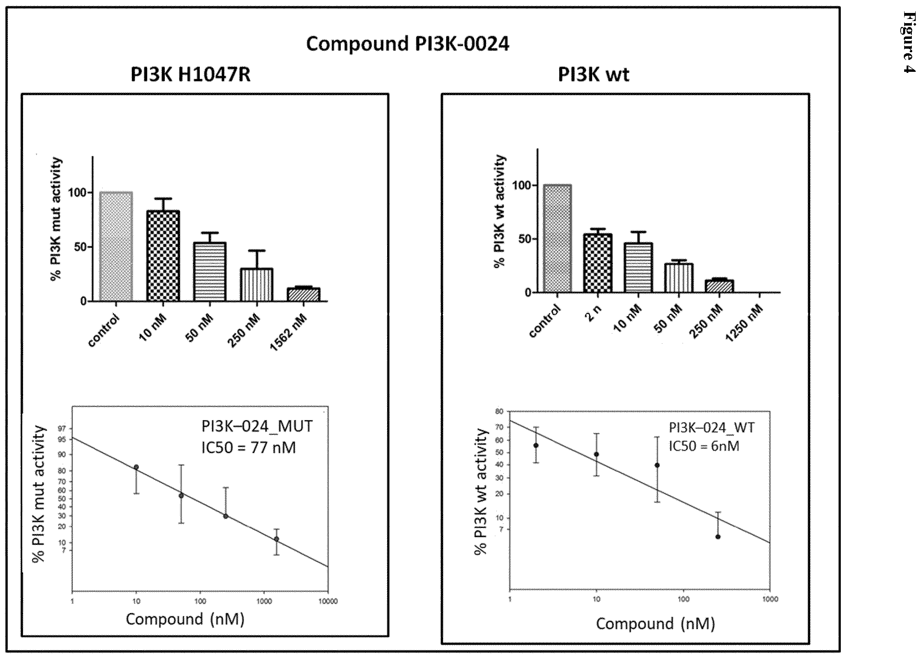

**Figure 4****.** *In vitro* PI3Kα activity assays for PI3K-024 against WT and H1047R PI3Kα. The IC50 of PI3K-024 against the mutant H1047R is 64 nM and 6 nM against the WT PI3Kα. These values were estimated using the logit-log graph of SigmaPlot and linear regression analysis. Error bars show the standard error of the mean from at least three independent experiments, each one performed in triplicates.

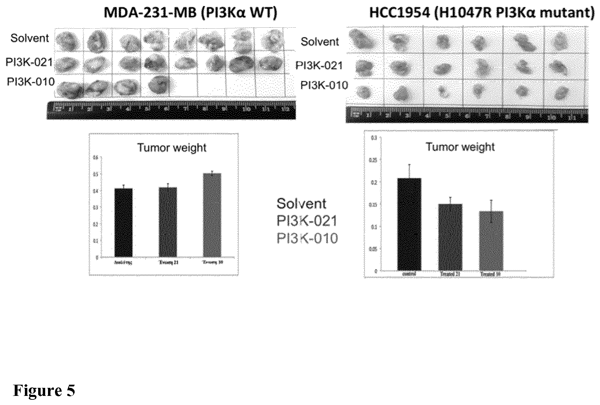

**Figure 5****.** Results of PI3K-010 or PI3K-021 treatment of xenografts generated by heterotopic transplantation of the human breast cancer cell line MDA-MB-231 cell line, which is wild-type PIK3CA and of the breast cancer line HCC1954, which bears the PIK3CA mutation H1047R.

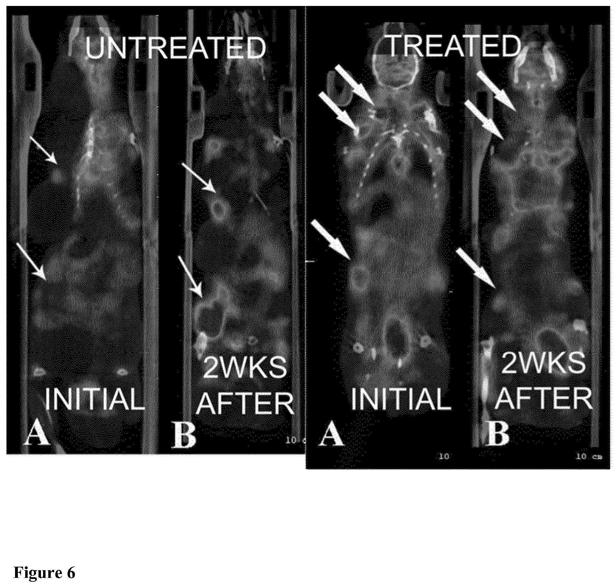

**Figure 6****.** Example of micro PET/CT imaging of WAPCre/exon20 Pik3ca*/MMTV-Myc mouse (compared with a negative wild-type control) at the beginning and end of treatment (one month) of breast cancer with the PI3K-010 compound.

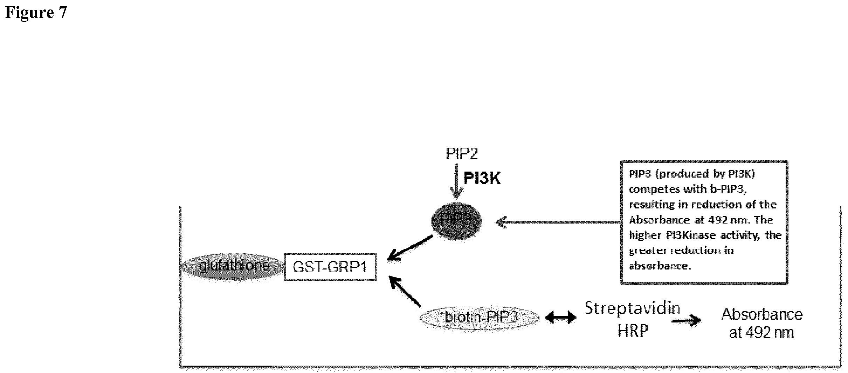

**Figure 7****.** Schematic representation of the principle of the PI3Kinase activity assay. In the assay, at first, GST-GRP1 is inclubated with glutathione-coated plates. Then, GST-GRP1 forms a stable complex with biotin-labeled-PIP3, which then is quantified using streptavidin-HRP conjugate and a read out with OPD at 492 nm. The unlabelel PIP3, that is generated when PI3Kα phosphorylates PIP2, competes with the labeled b-PIP3 for binding to the PH domain of GRP1, leading to a reduction of the signal at 492 nm.

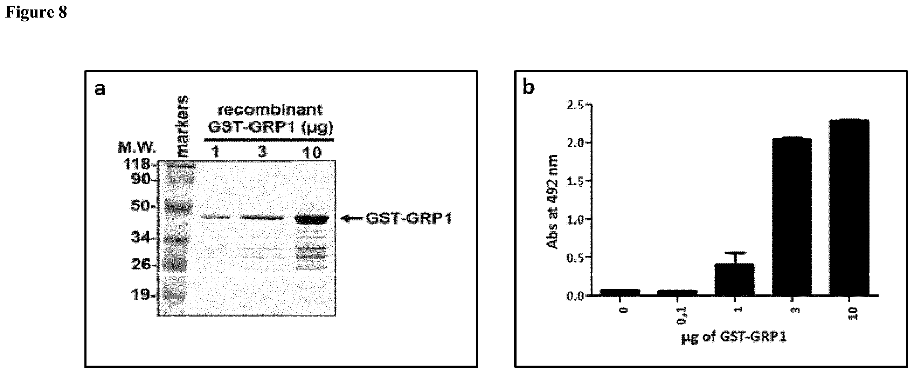

**Figure 8****:** Production and titration of the optimal amount of GST-GRP1 (PH domain)
   a. GST-GRP1 PH domain was expressed in Escherichia Coli strain (DE3) and affinity purified on glutathione-Sepharose 4B (Amersham Pharmacia), using the manufacturer's standard protocols. The purity of the protein was determined by SDS-PAGE.
   b. Increasing amounts of GST-GRP1PH domain were incubated with glutathione-coated plates followed by incubation at first with biotin-PIP3 (b-PIP3) and then with streptavidin-HRP. The amount of HRP (which reflects the quantity of bound biotin-PIP3) was measured by incubating the plate with Opposition Division (substrate of HRP), followed by measurement of the absorbance at 492 nm.

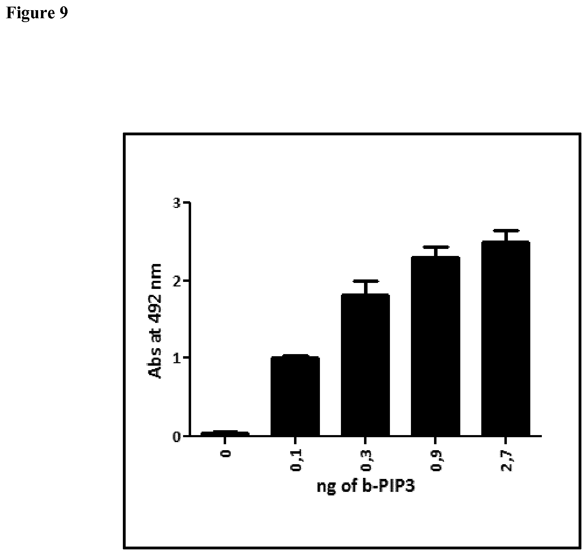

**Figure 9****:** Titration of b-PIP3. Different quantities of b-PIP3 were added to glutathione-coated plates, where GST-GRP1 was pre-bound, followed by the consecutive incubation with Streptavidin-HRP and Opposition Division, before measuring the absorbance at 492 nm. The amount of 0.3 ng bPIP3 was selected for subsequent assays, as the quantity of this reagent should be high enough to couple most of the available GST-GRP1 and to provide high signal, yet, it should not be large excess as it would require larger amounts of PI3K and PIP2 for the competition in the assay mixture, which would raise the cost for screening compound libraries.

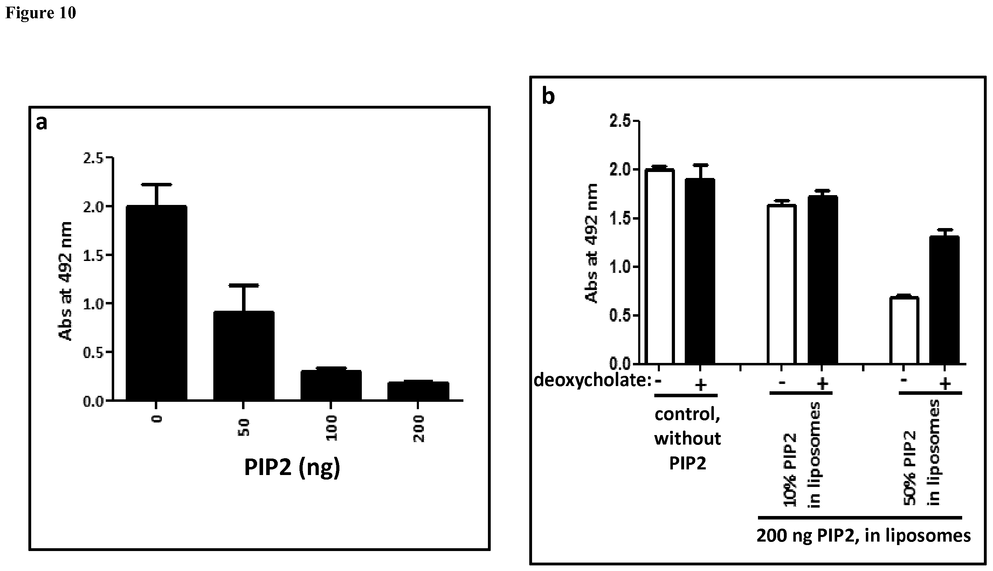

**Figure 10****:** The substrate PIP2 competes with b-PIP3 for binding to GRP1:
   a. When long side chain-PIP2 alone was incubated with the plates, there was a reduction of the signal, probably due to competition between PIP2 and bPIP3 for binding to GRP1.
   b. Incubation of the plate with PIP2 containing liposomes, that were prepared using lipids that have been isolated HCT116 cells, results in a much lower inhibition of the biotin-PIP3 signal, suggesting that the incorporation of PIP2 in liposomes reduces its binding to GRP1. In the sample containing 50% PIP2 in liposomes, where we observed inhibition of the signal despite the presence of liposomes, the signal was significantly recovered by the addition of sodium deoxycholate, at low concentration (below CMC), at the end of the enzymatic reaction, which led to a further recovery of the signal.
**Figure 11a****:** Identification of the preferred substrate preparation. The enzymatic reaction was tested using 2.5 or 5 ng PI3K. The substrates tested were either 200 ng PIP2 at a concentration of 10% or 50% PIP2 liposomes, or 2 µg soluble PIP2. Note that the most favorable preparation of the substrate is a mixture of 50% PIP2 in liposomes.
**Figure 11b****:** Titration of the substrate. Increasing concentrations of PIP2 were tested together with two different amounts of PI3K, 2 and 4 ng. The substrate (PIP2) was supplied at a concentration of 50% in liposomes.
**Figure 12****:** Standard curve: Absorbance versus PIP3 added exogenously. The standard curve follows the predicted typical hyperbolic profile of competitive inhibition, as expected to be the competition between the enzymatically produced PIP3 and b-PIP3 for binding to GST-GRP1.
**Figure 13****:** Validation of the specificity of the assay.
   a. The enzymatic reaction was assembled using 200 ng PIP2 (at a concentration of 50% in liposomes), the indicated amount of PI3K, ATP and buffer, and was incubated at 25°C for 16 min.
   b. Time-dependence is shown for incubation times of 4, 8, 16, 32 and 60 min.
**Figure 14****:** Validation of the specificity of the assay. IC₅₀ values of known PI3Kα inhibitors are determined using the inventive assay. The assessed IC₅₀ values for three known PI3Kα inhibitors are in agreement with previously reported values.

### DETAILED DESCRIPTION OF THE INVENTION

### Definitions

All terms used in the present application shall have the meaning usually employed by a relevant person skilled in the art, e.g. by a medicinal chemist, organic chemist, pharmacist, a molecular biologist, a physician, or a team thereof. By way of example, some definitions of specific terms are given below:

The articles "a" and "an" are used to refer to one or to more than one (i. e. to at least one) of the grammatical object of the article. For example, "a cell" means one cell or more than one cell.

"Adamantyl" refers to cyclic unsaturated aliphatic hydricarbyl groups arranged in three connected cyclohexane rings in the "armchair" configuration.

"Alkyl" includes monovalent saturated aliphatic hydrocarbyl groups. The hydrocarbon chain may be either straight-chained or branched.

"Aryl" refers to any functional group or substituent derived from an aromatic ring, such as a phenyl, naphthyl, thienyl, or indolyl. In preferred embodiments, the aryl is a substituted or unsubstituted phenyl, pyridinyl, or pyrimidinyl, more preferably a phenyl or a pyridinyl, and even more preferably a phenyl. The substitution may be an atom or a molecule substituted in place of a hydrogen atom on the parent chain of a hydrocarbon. Suitable substituents are known to a skilled person in the field of chemistry, more specifically, medicinal or organic chemistry.

"Cancer" or "tumor" refers to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within an animal, or may be a non-tumorigenic cancer cell, such as a leukemia cell. As used herein, the term "cancer" includes premalignant as well as malignant cancers. "Cancerous diseases" or "cancers", which are used interchangeably herein, include, but are not limited to, acute monocytic leukemia, acute myelogenous leukemia, acute myelomonocytic leukemia, acute promyelocytic leukemia, adult T-cell leukemia, adult T-cell lymphoma, astrocytoma, atypical carcinoid lung cancer, basal cell carcinoma, B-acute lymphocytic leukemia, B-cell acute lymphoblastic leukemia/lymphoma, Bladder cancer, brain cancer, breast cancer, bronchial cancer, Burkitt's lymphoma, cancer of the bile duct, cancer of unknown primary origin, cervix cancer, chronic myeloproliferative disorder, colon cancer, diffuse large cell lymphoma, endometrial cancer, ependymoma, esophageal cancer, gastric cancer, glioma, glioblastoma, head and neck cancer, hemagiopericytoma, hepatocellular carcinoma, Hodgkin's lymphoma, Kaposi's sarcoma, kidney cancer, large cell neuroendocrine carcinoma, large granular lymphocytic leukemia, leukemia, liver cancer, lung cancer, lymphoma, medulloblastoma, melanoma, Multiple myeloma, myelodysplastic syndrome, nasopharygeal cancer, neuroblastoma, NK cell tumor, non-Hodgkin's lymphoma, oesophageal squamous cell carcinoma, osteosarcoma, ovarian cancer, pancreatic cancer, peripheral T-cell leukemia, primary plasma cell leukemia, prostate cancer, renal clear cell carcinoma, retinoblastoma, rhabdomyosarcoma, sarcoma, small cell lung carcinoma, T-cell acute lymphoblastic leukemia, T-cell acute lymphoblastic lymphoma, testicular cancer, thymoma, thyroid cancer, urachal cancer, uterine cancer, vaginal cancer, and the like.

"Cycloalkyl" refers to cyclic saturated aliphatic hydrocarbyl groups. The numbers of C-atoms referenced in connection with a given cycloalkyl group corresponds to the number of ring forming carbon atoms, e.g. "C6 cycloalkyl" refers to a cyclohexyl. Preferably, cycloalkyls referenced herein are C6-C14 cycloalkyls.

"Cycloalkenyl" refers to cyclic unsaturated aliphatic hydrocarbyl groups. The numbers of C-atoms referenced in connection with a given cycloalkyl group correspond to the number of ring forming carbon atoms, e.g. "C6 cycloalkyl" refers to a cyclohexenyl. In some embodiments, the cycloalkenyl comprises one double-bond. In some embodiments, the cyclohexenyl comprises more than one double bond, preferably two double bonds.

"Deregulation" refers to an alteration or modification of the expression of a gene that encodes an enzyme/protein in a biosynthetic pathway, such that the level or activity of said enzyme/protein is altered or modified, which is found in, but is not limited to, cancer cells.

"Furanyl" comprises a furan ring that can be bound by any C-atom.

"Halo" or "halogen" refers to fluoro, chloro, bromo and iodo. Preferred halo groups are either fluoro or chloro.

"Haloalkyl" includes an "alkyl" group as defined further above which is substituted with one or more halogens which may be the same or different.

"Hydrate" refers to a type of crystalline form which retains a certain number of water molecules as part of the solid crystalline structure.

"Neoplasm" refers to an abnormal mass of tissue as a result of abnormal growth or division of cells. It usually causes a lump or tumor. Neoplasms may be benign, pre-malignant (carcinoma in situ) or malignant (cancer).

"Overexpression" refers to an excessive expression of a gene, thereby producing an excess of its effect or product. Most cancers arise through the overexpression of key cellular regulatory genes.

"Prodrug" refers to a medication that is initially administered to the body in an inactive (or less than fully active) form, and then becomes converted to its active form through the normal metabolic processes of the body.

"Pyrazolyl" comprises a pyrazole ring that can be bound by any C-atom as well as by its nitrogen atom.

"Pyrrolyl" comprises a pyrrole ring that can be bound by any C-atom as well as by its nitrogen atom.

"Solvate" refers to a type of crystalline form which retains a certain number of solvent molecule other than water as part of the solid crystalline structure.

"Tautomers" refer to constitutional isomers of an organic compound that interconvert readily by a chemical reaction called tautomerization, which commonly results in the formal migration of a hydrogen atom or proton, accompanied by a switch of a single bond and adjacent double bond.

"Thiophenyl" comprises a thiophen ring that can be bound by any C-atom.

Any "alkyl", "cycloalkyl", "furanyl", "pyrrolyl", "thiophenyl", "aryl or "phenyl" may be unsubstituted or substituted by one or more atoms or atom groups (molecules).

"Pharmaceutically acceptable" refers to being devoid of substantial toxic effects when used in doses usually employed in a medicinal dosage form, and thereby being approvable or preferably being approved by a regulatory agency of the Federal or a state government or being listed in the U. S. Pharmacopoeia or other generally recognized pharmacopoeia for use in animals, more preferably in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid,3- (4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4- chlorobenzenesulfonic acid, 2naphthalenesulfonic acid, 4- toluenesulfonic acid,camphorsulfonic acid, 4methylbicyclo [2.2.2]-oct-2-ene-1-carboxylic acid, glucoheptonic acid,3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced, forming an ion pair with a positively-charged base moiety.

"Pharmaceutically acceptable carrier" refers to a diluent, adjuvant, excipient or carrier with which a compound of the invention is administered.

"Polymorphs" refer to different crystalline forms of the same compound. Polymorphism denotes the ability of a material to exist in more than one form or crystal structure.

"Preventing" or "prevention" refers to a reduction in risk of acquiring a disease or disorder (i. e., causing at least one of the clinical symptoms of the disease not to develop in a subject that may be exposed to or predisposed to the disease but does not yet experience or display symptoms of the disease).

"Therapeutically effective amount" refers to the amount of a compound that, when administered to a subject for treating a disease, is sufficient to effect such treatment for the disease. The "therapeutically effective amount" can vary depending on the compound, the disease and its severity, and the age, weight, etc., of the subject to be treated. Preferably, the compound according to the present invention may be used at a dose of from 0.1 to 200 mg/kg, more preferably at a dose of from 0.5 to 50.0 mg/kg, preferably such doses being applied twice a day. Daily doses to be administered can also be from 50mg to 500 mg/day and preferably 150 to 300 mg/day. In any event, the suitable dose will be determined by those of skill in the art by appropriate routine dosing experiments carried out in the species intended to be treated or based on the response of the patient. Suitable doses can be applied in a single treatment or by application of appropriate partial doses at several different points in time.

"Treating" or "treatment" of any disease or disorder refers to, in one embodiment, to ameliorating the disease or disorder (i. e., arresting or reducing the development of the disease or at least one of the clinical symptoms thereof). In another embodiment, "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically (e. g., stabilization of a physical parameter), or both. In yet another embodiment, "treating" or "treatment" refers to delaying the onset of the disease or disorder. Generally, it is understood that methods of treatment are not encompassed by the scope of the invention.

### Compounds and Pharmaceutical Compositions

As a result of their intensive studies and inventive efforts, the inventors were able to create the first ever reported allosteric inhibitors of PI3Kα mutants.

Accordingly, the invention defined in claim 1 refers to a compound of formula (I) 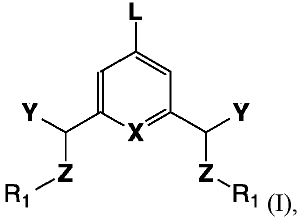
a tautomer, polymorph, hydrate, solvate, or a pharmaceutically acceptable salt thereof,
wherein
L is independently H or OH;
X is independently N or CH;
Y is independently H or =O;
Z is independently S or NH;
R₁ is independently mono- or polycyclic aryl or heteroaryl selected from: 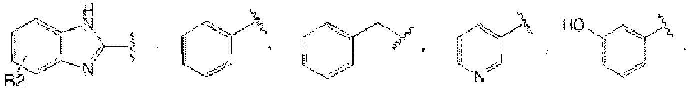 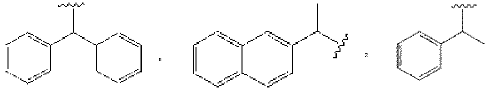 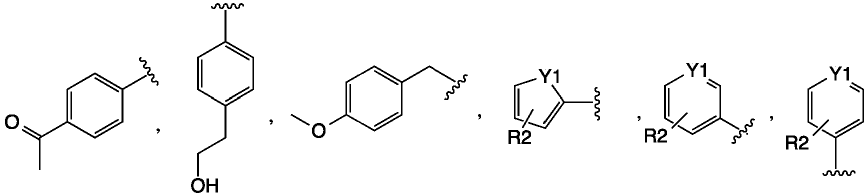
R₂ is independently at least one of hydroxyl, hydrogen, fluoro, chloro, or bromo;
in the five-membered rings, Y₁ is independently CH₂, O, S, or NH, whereas in the six-membered rings, Y₁ is independently CH, or N,
for use in treating a cancerous disease caused by or triggered by a PI3Kα in a mammal, wherein the mammal to be treated carries a mutation in the gene encoding PI3Kα which leads to the occurrence of a PI3Kα mutant, and wherein the mammal is preferably a human. It has been discovered by the present inventors that the compounds of formula (I) are isoform-specific heterotropic modulators of PI3Kα activity and at least decrease or even completely inhibit the activity of PI3Kα mutants which have been associated with certain cancer diseases by e.g. participating in their development and/or facilitating or promoting persistence and/or exacerbation thereof. Without wishing to be bound to any theory, the compounds have been discovered to allosterically inhibit the activity of mutant proteins, especially the hot spot mutant H1047R, by binding at a site which is distinct from the orthosteric binding pocket of ATP. The allosteric binding of a coumpound to the mutated PI3Kα causes conformational changes which affect the PI3Kα mutant catalytic activity irrespective of ATP binding and, consequently, the mutants' activities.

In preferred embodiments, formula (I) refers to a compound having the formula (II): 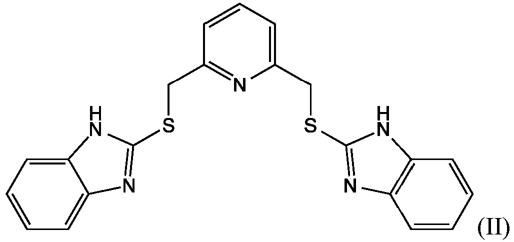 or a pharmaceutically acceptable salt thereof. This compound is also referred to herein with its internal name PI3K-010.

In preferred embodiments, formula (I) refers to a compound having the formula (III): 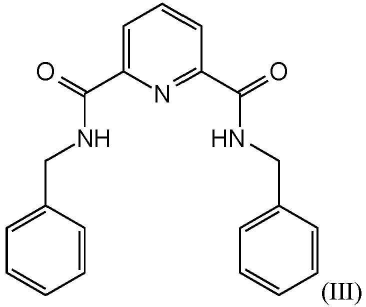 or a pharmaceutically acceptable salt thereof. This compound is also referred to herein with its internal name PI3K-021.

In preferred embodiments, formula (I) refers to a compound having the formula (IV): 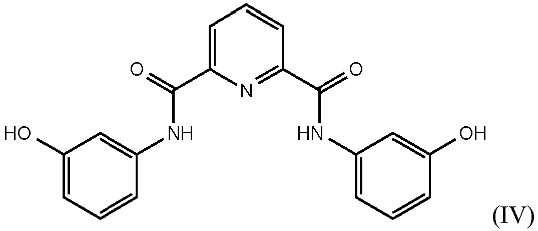 or a pharmaceutically acceptable salt thereof. This compound is also referred to herein with its internal name PI3K-024.

### Pharmaceutical Compositions

The present document further describes pharmaceutical compositions comprising the compounds of formula (I) or, preferably, of formula (II), or of formula (III), or of formula (IV) as depicted above.

Optionally, the pharmaceutical compositions may further comprise one or more additional therapeutic (i.e. pharmaceutically active) agents.

The one or more additional therapeutic agents of the pharmaceutical composition may be selected from the group (A) consisting of: 10-Hydroxycamptothecin, 17-Allylamino-geldanamycin, 2-Methoxyanti-mycin A3, 3,4-Dichloroisocoumarin, 4-Hydroxyphenylretinamide, 9-cis Retinoic acid, Abiraterone, Ado-Trastuzumab Emtansine, Adriamycin, Afatinib, N-(3-chlorophenyl)-6,7-dimethoxyquinazolin-4-amine, 2-Amino-4-(1H-indol-5-yl)-1,1,3-tricyanobuta-1,3-diene, Aldesleukin, Alemtuzumab, Amifostine, Anastrozole, Anisomycin, Aphidicolin, Arsenic Trioxide, Asparaginase Erwinia chrysanthemi, Avastin, Axitinib, N-[2(S)-(2(R)-2-Amino-3-mercaptopropylamino)-3-methylbutyl]-Lphenylalanyl-L-methionine trifluoroacetate salt, Bacillus Calmette-Guerin, bisphenol A diglycidyl ether, Bendamustine, Beta-lapachone , Betulinic acid, Bevacizumab, Bexarotene, Bicalutamide, BisBenzimide, Bleomycin, Bortezomib, Bosutinib, Buserelin, Busulfan, Cabazitaxel, Cabozantinib-S-Malate, Calpeptin, Camptothecin, Caffeic acid phenethyl ester, Capecitabine, CAprelsa (Vandetanib), Carboplatin, Carboplatin, Carfilzomib, Carmustine, Cetuximab, Chlorambucil, Ciglitazone, Cisplatin, Clodronate, Clofarabine, Cometriq, Crizotinib, Curcumin, Cyclo [Arg-Gly-Asp-D-Phe-Val], Cycloheximide, Cyclopamine, Cyclophosphamide, Cyclosporin A, Cyproterone, Cytarabine, D12-Prostaglandin J2, Dabrafenib, Dacarbazine, Dactinomycin, Dasatinib, Daunorubicin, Degarelix, Denosumab, Dexamethasone, Docetaxel, Doxorubicin, Ebselen, Ellipticine, Enzalutamide, Epirubicin, Erlotinib, Etoposide, Everolimus, Exemestane, Fludarabine, Fluorouracil, Flutamide, Folinic acid, Fulvestrant, Gefitinib, Geldanamycin, Gemcitabine, Genistein, Gingerol, Gliadel Wafer, Gliotoxin, Goserelin, 2-Chloro-5-nitrobenzanilide, 2-Amino-6-bromo-α-cyano-3-(ethoxycarbonyl)-4H-1-benzopyran-4-acetic acid ethyl ester, Hinokitiol, Hydroxyurea, Sobuzoxane, Idarubicin, ifosfamide, Imatinib, Indomethacin, Ipilimumab, Irinotecan, Ixabepilone, Lanreotide, Lapatinib, Lenalidomide, Letrozole, Lenvatinib Mesylate, Lenvima, Leucovorin, Leuprolide, Lomustin, Medroxyprogesterone, Megestrol, Melphalan, Mepesuccinate, Mercaptopurine, Mesna, Methotrexate, Methoxy verapamil, carbobenzoxy-L-leucyl-L-leucyl-L-leucinal, Mitomycin C, Mitoxantrone, N,N-Dimethylsphingosine, Nelarabine, Nilotinib, Nivolumab, Octreotide, Ofatumumab, Oligomycin A, Omacetaxine, Oxaliplatin, Paclitaxel, Pamidronate, Panitumumab, Pazopanib, Pegaspargase, Pemetrexed, Pembrolizumab, Pertuzumab, Pifithrin, plerixafor, Podophyllotoxin, Pomalidomide, Ponatinib, Prednisone, 2,2-Bis(hydroxymethyl)-1-azabicyclo[2.2.2]octan-3-one, Procarbazine, Radium 223 Dichloride, Raltitrexed, Rapamycin, Recombinant Human Papillomavirus (HPV) Bivalent Vaccine, Recombinant HPV Quadravalent Vaccine, Recombinant HPV Bivalent , Vaccine, Recombinant HPV Quadravalent Vaccine, Recombinant Interferon Alfa-2b, Regorafenib, Resveratrol, all trans Retinoic acid, ,Rheumatrex, Rituximab, Rolipram, Roscovitine, Rottlerin, Shikonin, Sipuleucel-T, Sirolimus, Sorafenib, Sphingosine, Splitomycin, Staurosporine, Stilboestrol, Streptozocin, 3-(4-Dimethylaminobenzylidenyl)-2-indolinone, 3-[[(4-Dimethyl-amino)phenyl] methylene]-1,3-dihydro-2H-indol-2-one, Sulindac sulphide, Sunitinib, Tamoxifen, Temozolomide, Temsirolimus, Thalidomide, Topotecan, Toremifene, Trametinib, Trastuzumab, Trichostatin-A, Trifluoperazine, 4-[(E)-2-(5,6,7,8-Tetrahydro-5,5,8,8-tetramethyl-2-naphthalenyl)-1-propenyl]benzoic acid, 3,4-Dihydroxy-α-cyanothiocinnam-amide, (3-Hydroxy-4-nitrobenzylidene)malononitrile, Vandertanib, Valproic acid, Vemurafenib, Verapamil, Vinblastine, Vincristine, Vinorelbine, Wortmannin, 4-Chloro-6-(2,3-xylidino)-2-pyrimidinylthioacetic acid, Ziv-Aflibercept, Zoledronic Acid, salts thereof, and combinations thereof.

Preferably, the one or more therapeutic agents are selected from, but not restricted to, the group (A'), i.e. Abiraterone, Ado-Trastuzumab Emtansine, Afatinib, Afinitor (Everolimus), Anastrozole, Avastin, Bevacizumab, Cabazitaxel, Capecitabine, Carboplatin, Carmustin, Cisplatin, Crizotinib, Cyclophosphamide, Degarelix, Denosumab, Docetaxel, Doxorubicin, Enzalutamide, Epirubicin, Erlotinib, Etoposide, Everolimus, Exemestane, Fluorouracil, Fulvestrant, Gefitinib, Gemcitabine, Ixabepilone, Lapatinib, Letrozole, Leuprolide, Lomustine, Megestrol, Methotrexate, Mitomycin C, Paclitaxel, Pamidronate, Pemetrexed, Pertuzumab, Prednisone, Radium 223 Dichloride, Sipuleucel-T, Sunitinib, Tamoxifen, Temodar, Temozolomide, Topotecan, Toremifene, Trastuzumab, Cabozantinib-S-Malate, Caprelsa (Vandetanib), Cometriq (Cabozantinib-S-Malate), Doxorubicin Hydrochloride, Ipilimumab, Lenvatinib Mesylate, Nexavar (Sorafenib Tosylate) Nivolumab, Vandetanib, Yervoy (Ipilimumab), salts thereof, and any combination thereof.

The pharmaceutical compositions may comprise, besides the compound of formula (I) or formula (II) described above, one or more additional therapeutic agent(s) selected from the group consisting of:
*for Acute Lymphoblastic Leukemia (ALL):*
   Abitrexate (Methotrexate), Adriamycin PFS (Doxorubicin Hydrochloride), Adriamycin RDF (Doxorubicin Hydrochloride), Arranon (Nelarabine), Asparaginase Erwinia chrysanthemi, Cerubidine (Daunorubicin Hydrochloride), Clafen (Cyclophosphamide), Clofarabine, Clofarex (Clofarabine), Clolar (Clofarabine), Cyclophosphamide, Cytarabine, Cytosar-U (Cytarabine), Cytoxan (Cyclophosphamide), Dasatinib, Faunorubicin Hydrochloride, Doxorubicin Hydrochloride, Erwinaze (Asparaginase Erwinia hrysanthemi), Folex (Methotrexate), Folex PFS (Methotrexate), Gleevec (Imatinib Mesylate), Iclusig (Ponatinib Hydrochloride), Imatinib Mesylate, Marqibo (Vincristine Sulfate Liposome), Mercaptopurine, Methotrexate, Methotrexate LPF (Methorexate), Mexate (Methotrexate), Mexate-AQ (Methotrexate), Nelarabine, Neosar (Cyclophosphamide), Oncaspar (Pegaspargase), Pegaspargase, Purinethol (Mercaptopurine), Rubidomycin (Daunorubicin Hydrochloride), Sprycel (Dasatinib), Tarabine PFS (Cytarabine), Vincasar PFS (Vincristine Sulfate), Vincristine Sulfate, or Vincristine Sulfate Liposome;
*for Acute Myeloid Leukemia (AML):*
   Adriamycin PFS (Doxorubicin Hydrochloride). Adriamycin RDF (Doxorubicin Hydrochloride), Arsenic Trioxide, Cerubidine (Daunorubicin Hydrochloride), Clafen (Cyclophosphamide), Cyclophosphamide, Cytarabine, Cytosar-U (Cytarabine), Cytoxan (Cyclophosphamide), Daunorubicin Hydrochloride, Doxorubicin Hydrochloride, Neosar (Cyclophosphamide), Rubidomycin (Daunorubicin Hydrochloride), Tarabine PFS (Cytarabine), Trisenox (Arsenic Trioxide), Vincasar PFS (Vincristine Sulfate), or Vincristine Sulfate;
*for Chronic Lymphocytic Leukemia (CLL):*
   Alemtuzumab, Ambochlorin (Chlorambucil), Amboclorin (Chlorambucil), Arzerra (Ofatumumab), Bendamustine Hydrochloride, Campath (Alemtuzumab), ChlorambucilClafen (Cyclophosphamide), Cyclophosphamide, Cytoxan (Cyclophosphamide), Fludara (Fludarabine Phosphate), Fludarabine Phosphate, Leukeran (Chlorambucil), Linfolizin (Chlorambucil), Neosar (Cyclophosphamide), Ofatumumab, Treanda (Bendamustine Hydrochloride), Chlorambucil-Prednisone, or CVP (a combination of Cyclophosphamide, Vincristine and Prednisone);
*for Chronic Myelogenous Leukemia (CML):*
   Bosulif (Bosutinib), Bosutinib, Clafen (Cyclophosphamide), Cyclophosphamide, Cytarabine, Cytosar-U (Cytarabine), Cytoxan (Cyclophosphamide), Dasatinib, Gleevec (Imatinib Mesylate), Iclusig (Ponatinib Hydrochloride), Imatinib Mesylate, Neosar (Cyclophosphamide), Nilotinib, Omacetaxine Mepesuccinate, Ponatinib Hydrochloride, Sprycel (Dasatinib), Synribo (Omacetaxine Mepesuccinate), Tarabine PFS (Cytarabine), or Tasigna (Nilotinib);
*for Meningeal Leukemia:*
   Cytarabine, Cytosar-U (Cytarabine), or Tarabine PFS (Cytarabine);
*for Burkitt's lymphoma:*
   Methotrexate (Anti-Rheumatic) oral, Rituxan iv, methotrexate sodium oral, dexamethasone oral, DexPak, rituximab iv, methotrexate sodium inj, Adriamycin iv, cyclophosphamide oral, Trexall oral, vincristine iv, cyclophosphamide iv, dexamethasone sodium phosphate inj, Dexamethasone Intensol oral, methotrexate sodium (PF) inj, Rheumatrex oral, cytarabine inj, doxorubicin iv, dexamethasone sodium phos (PF) inj, etoposide iv, ifosfamide iv, Ifex iv, cytarabine (PF) inj, Adriamycin PFS iv, DexPak 10 day oral, DexPak 6 Day oral, ifosfamidemesna iv, Toposar iv, etoposide phosphate iv, Etopophos iv, Vincasar PFS iv, or Baycadron oral;
*for diffuse large cell lymphoma:*
   prednisone oral, Rituxan iv, dexamethasone oral, DexPak 13 Day oral, rituximab iv, Adriamycin iv, cyclophosphamide oral, Prednisone Intensol oral, vincristine iv, cyclophosphamide iv, bleomycin inj, dexamethasone sodium phosphate inj, Dexamethasone Intensol oral, cytarabine inj, melphalan oral, doxorubicin iv, Alkeran oral, etoposide oral, dexamethasone sodium phos (PF) inj, melphalan iv, procarbazine oral, etoposide iv, cytarabine (PF) inj, Adriamycin PFS iv, DexPak 10 day oral, DexPak 6 Day oral, Rayos oral, Alkeran iv, Matulane oral, epirubicin iv, Toposar iv, etoposide phosphate iv, Etopophos iv, Ellence iv, Vincasar PFS iv, or Baycadron oral;
*For Brain Cancer:*
   Afinitor (Everolimus), Avastin (Bevacizumab), BiCNU (Carmustine), CArmustine wafer, Lomustine, Temodar (Temozolomide), Temozolomide, Temodar oral, vincristine iv, Camptosar iv, irinotecan iv, bevacizumab iv, temozolomide oral, Gliadel Wafer impl, procarbazine oral, BiCNU iv, Temodar iv, Matulane oral, Vincasar PFS iv, carmustine iv, carmustine in polifeprosan impl, or temozolomide iv;
*for Multiple Myeloma and Other Plasma Cell Neoplasms:*
   Aredia (Pamidronate Disodium), Bortezomib, Carfilzomib, Clafen (Cyclophosphamide), Cyclophosphamide, Cytoxan (Cyclophosphamide), Doxil (Doxorubicin Hydrochloride Liposome), Doxorubicin Hydrochloride Liposome, Dox-SL (Doxorubicin Hydrochloride Liposome), Evacet (Doxorubicin Hydrochloride Liposome), Kyprolis (Carfilzomib), Lenalidomide, LipoDox (Doxorubicin Hydrochloride Liposome), Mozobil (Plerixafor), Neosar (Cyclophosphamide), Pamidronate Disodium, Plerixafor, Pomalidomide (Pomalyst), Pomalyst, Revlimid (Lenalidomide), Synovir (Thalidomide), Thalidomide, Thalomid (Thalidomide), Velcade (Bortezomib), Zoledronic Acid, or Zometa (Zoledronic Acid);
*for Non-Small Cell Lung Cancer:*
   Abitrexate (Methotrexate), Abraxane (Paclitaxel Albumin-stabilized Nanoparticle Formulation), Afatinib Dimaleate, Alimta (Pemetrexed Disodium), Avastin (Bevacizumab), Bevacizumab, Carboplatin, Cisplatin, Crizotinib, Erlotinib Hydrochloride, Folex (Methotrexate), Folex PFS (Methotrexate), Gefitinib, Gilotrif (Afatinib Dimaleate), Gemcitabine Hydrochloride, Gemzar (Gemcitabine Hydrochloride), Iressa (Gefitinib), Methotrexate, Methotrexate LPF (Methotrexate), Mexate (Methotrexate), Mexate-AQ (Methotrexate), Paclitaxel, Paclitaxel Albumin-stabilized Nanoparticle Formulation, Paraplat (Carboplatin), Paraplatin (Carboplatin), Pemetrexed Disodium, Platinol (Cisplatin), Platinol-AQ (Cisplatin), Tarceva (Erlotinib Hydrochloride), Taxol (Paclitaxel), Xalkori (Crizotinib), a combination of Carboplatin-Taxol, or a combination of Gemcitabine-Cisplatin;
*for Small Cell Lung Cancer:*
   Abitrexate (Methotrexate), Etopophos (Etoposide Phosphate), Etoposide, Etoposide 5 Phosphate, Folex (Methotrexate), Folex PFS (Methotrexate), Hycamtin (Topotecan Hydrochloride), Methotrexate, Methotrexate LPF (Methotrexate), Mexate (Methotrexate), Mexate-AQ (Methotrexate), Toposar (Etoposide), Topotecan Hydrochloride, or VePesid (Etoposide);
*for Bladder Cancer:*
   Adriamycin PFS (Doxorubicin Hydrochloride), Adriamycin RDF (Doxorubicin Hydrochloride), Cisplatin, Doxorubicin Hydrochloride, Platinol (Cisplatin), or Platinol-AQ (Cisplatin);
*for Breast Cancer:*
   Perjeta (pertuzumab), Abitrexate (Methotrexate), Abraxane(Paclitaxel Albumin-stabilized Nanoparticle Formulation), Ado-Trastuzumab Emtansine, Adriamycin PFS (Doxorubicin Hydrochloride), Adriamycin RDF (Doxorubicin Hydrochloride), Adrucil (Fluorouracil), Afinitor (Everolimus), Anastrozole, Aredia (Pamidronate Disodium), Arimidex (Anastrozole), Aromasin (Exemestane), Capecitabine, Clafen (Cyclophosphamide), Cyclophosphamide, Cytoxan (Cyclophosphamide), Docetaxel, Doxorubicin Hydrochloride, Efudex (Fluorouracil), Ellence (Epirubicin Hydrochloride), Epirubicin Hydrochloride, Everolimus, Exemestane, Fareston (Toremifene), Faslodex (Fulvestrant), Femara (Letrozole), Fluoroplex (Fluorouracil), Fluorouracil, Folex (Methotrexate), Folex PFS (Methotrexate), Fulvestrant, Gemcitabine Hydrochloride, Gemzar (Gemcitabine Hydrochloride), Herceptin (Trastuzumab), Ixabepilone,
   Ixempra (Ixabepilone), Kadcyla (Ado-Trastuzumab Emtansine), Lapatinib Ditosylate, Letrozole, Megace (Megestrol Acetate), Megestrol Acetate, Methotrexate, Methotrexate LPF (Methotrexate), Mexate (Methotrexate), Mexate-AQ (Methotrexate), Neosar (Cyclophosphamide), Nolvadex (Tamoxifen Citrate), Novaldex (Tamoxifen Citrate), Paclitaxel, Paclitaxel Albumin-stabilized Nanoparticle Formulation, Pamidronate Disodium, Perjeta (Pertuzumab), Pertuzumab, Tamoxifen Citrate, Taxol (Paclitaxel), Taxotere (Docetaxel), Trastuzumab, Toremifene, Tykerb (Lapatinib Ditosylate), or Xeloda (Capecitabine);
*for Cervical Cancer:*
   Blenoxane (Bleomycin), Bleomycin, Cisplatin, Hycamtin (Topotecan Hydrochloride), Platinol (Cisplatin), Platinol-AQ (Cisplatin), Topotecan Hydrochloride, or a combination of Gemcitabine-Cisplatin;
*for Endometrial Cancer:*
   paclitaxel, decataxel, doxorubicin, cisplatin, carboplatin, capecitabine, gemcitabine, tamoxifen, hydroxyprogesterone caproate, letrozole, megestrol acetate, medroxyprogesterone;
*for Esophageal Cancer:*
   Cyramza (Ramucirumab), Docetaxel, Herceptin (Trastuzumab), Ramucirumab, Trastuzumab;
*for Colon Cancer:*
   Adrucil (Fluorouracil), Avastin (Bevacizumab), Bevacizumab, Camptosar (Irinotecan Hydrochloride), Capecitabine, Cetuximab, Efudex (Fluorouracil), Eloxatin, Oxaliplatin), Erbitux (Cetuximab), Fluoroplex (Fluorouracil), Fluorouracil, Irinotecan Hydrochloride, Leucovorin Calcium, Oxaliplatin, Panitumumab, Regorafenib, Stivarga (Regorafenib), Vectibix (Panitumumab), Wellcovorin (Leucovorin Calcium), Xeloda (Capecitabine), Zaltrap (Ziv-Aflibercept), Ziv-Aflibercept, CAPOX (a combination of capecitabine and oxaliplatin), FOLFIRI (a combination of Folinic acid, Fluorouracil, and Irinotecan), FOLFIRI-Bevacizumab (a combination of Folinic acid, Fluorouracil, Irinotecan, and Bevacizumab), FOLFIRICetuximab (a combination of Folinic acid, Fluorouracil, Irinotecan, and Cetuximab), FOLFOX (a combination of Folinic acid, Fluorouracil, and Oxaliplatin), or XELOX (a combination of Capecitabine and Oxaliplatin);
*for Rectal Cancer:*
   Adrucil (Fluorouracil), Avastin (Bevacizumab), Bevacizumab, Camptosar (Irinotecan Hydrochloride), Cetuximab, Efudex (Fluorouracil), Erbitux (Cetuximab), Fluoroplex (Fluorouracil), Fluorouracil, Irinotecan Hydrochloride, Panitumumab, Regorafenib, Stivarga (Regorafenib), Vectibix (Panitumumab), Zaltrap (Ziv-Aflibercept), Ziv-Aflibercept, CAPOX, FOLFIRI, FOLFIRI-Bevacizumab, FOLFIRI-Cetuximab, FOLFOX, or XELOX;
*for Gastric Cancer:*
   Adriamycin PFS (Doxorubicin Hydrochloride), Adriamycin RDF (Doxorubicin Hydrochloride), Adrucil (Fluorouracil), Docetaxel, Doxorubicin Hydrochloride, Efudex (Fluorouracil), Fluoroplex (Fluorouracil), Fluorouracil, Herceptin (Trastuzumab), Mitomycin C, Mitozytrex (Mitomycin C), Mutamycin (Mitomycin C), Taxotere (Docetaxel), or Trastuzumab;
*for Glioblastoma:*
   Avastin (Bevacizumab), Temodar oral, vincristine iv, Camptosar iv, irinotecan iv, bevacizumab iv, temozolomide oral, Gliadel Wafer impl, procarbazine oral, BiCNU iv,
   Temodar iv, Matulane oral, Vincasar PFS iv, carmustine iv, carmustine in polifeprosan impl, or temozolomide iv;
*For Hepatocellular Carcinoma:*
   Sorafenib, Sunitinib, Erlotinib, Bevacizumab, or Sirolimus;
*for large cell neuroendocrine carcinoma:*
   sunitinib (Sutent), everolimus (Afinitor), or the combination of fluorouracil (Adrucil, 5-FU), doxorubicin (Adriamycin), and streptozocin (Zanosar);
*for Melanoma:*
   Aldesleukin, Dabrafenib, Dacarbazine, DTIC-Dome (Dacarbazine), Intron A (Recombinant Interferon Alfa-2b), Ipilimumab, Mekinist (Trametinib), Peginterferon Alfa-2b, PEG-Intron (Peginterferon Alfa-2b), Proleukin (Aldesleukin), Recombinant Interferon Alfa-2b, Sylatron (Peginterferon Alfa-2b), Tafinlar (Dabrafenib), Trametinib, Vemurafenib, Yervoy (Ipilimumab), or Zelboraf (Vemurafenib);
*for Neuroblastoma:*
   Adriamycin PFS (Doxorubicin Hydrochloride), Adriamycin RDF (Doxorubicin Hydrochloride), Clafen (Cyclophosphamide), Cyclophosphamide, Cytoxan (Cyclophosphamide), Doxorubicin Hydrochloride, Neosar (Cyclophosphamide), Vincasar PFS (Vincristine Sulfate), or Vincristine Sulfate;
*for Ovarian Cancer:*
   Adriamycin PFS (Doxorubicin Hydrochloride), Adriamycin RDF (Doxorubicin Hydrochloride), Carboplatin, Clafen (Cyclophosphamide), Cisplatin, Cyclophosphamide, Cytoxan (Cyclophosphamide), Doxorubicin Hydrochloride, Dox-SL (Doxorubicin Hydrochloride Liposome), DOXIL (Doxorubicin Hydrochloride Liposome), Doxorubicin Hydrochloride Liposome, Evacet (Doxorubicin Hydrochloride Liposome), Gemcitabine Hydrochloride, Gemzar (Gemcitabine Hydrochloride), Hycamtin (Topotecan Hydrochloride), LipoDox (Doxorubicin Hydrochloride Liposome), Neosar (Cyclophosphamide), Paclitaxel, Paraplat (Carboplatin), Paraplatin (Carboplatin), Platinol (Cisplatin), Platinol-AQ (Cisplatin), Taxol
   (Paclitaxel), Topotecan Hydrochloride, BEP (a combination of Bleomycin, Etoposide, and Cisplatin), or a combination of Carboplatin-Taxol, or a combination of Gemcitabine-Cisplatin;
*for Head and Neck Cancer:*
   Bleomycin, Cetuximab, Docetaxel, Erbitux (Cetuximab), Hydrea (Hydroxyurea), Keytruda (Pembrolizumab), Methotrexate, Nivolumab, Taxotere (Docetaxel) Trexall (Methotrexate);
*for Liver Cancer:*
   Nexavar (Sorafenib Tosylate), Regorafenib, Sorafenib Tosylate, Stivarga (Regorafenib);
*for Thyroid Cancer:*
   Cabozantinib-S-Malate, Caprelsa (Vandetanib), Cometriq (Cabozantinib-S-Malate), Doxorubicin Hydrochloride, Ipilimumab, Lenvatinib Mesylate, Lenvima (Lenvatinib Mesylate), Nexavar (Sorafenib Tosylate), Nivolumab, Vandetanib, Yervoy (Ipilimumab);
*for Pancreatic Cancer:*
   Abraxane, Nab-Paclitaxel, Sunitinib, Everolimus, Adrucil (Fluorouracil), Efudex (Fluorouracil), Erlotinib Hydrochloride, Fluoroplex (Fluorouracil), Fluorouracil, Gemcitabine Hydrochloride, Gemzar (Gemcitabine Hydrochloride), Mitomycin C, Mitozytrex (Mitomycin C), Mutamycin (Mitomycin C), or Tarceva (Erlotinib Hydrochloride);
*for Penile Cancer:*
   Blenoxane (Bleomycin), or Bleomycin;
*for Prostate Cancer:*
   Abiraterone Acetate, Cabazitaxel, Degarelix, Denosumab, Docetaxel, Enzalutamide, Jevtana (Cabazitaxel), Leuprolide Acetate, Lupron (Leuprolide Acetate), Lupron Depot (Leuprolide Acetate), Lupron Depot-3 Month (Leuprolide Acetate), Lupron Depot-4 Month (Leuprolide Acetate), Lupron Depot-Ped (Leuprolide Acetate), Prednisone, Prolia (Denosumab), Provenge (Sipuleucel-T), Radium 223 Dichloride, Sipuleucel-T, Taxotere (Docetaxel), Viadur (Leuprolide Acetate), Xgeva (Denosumab), Xofigo (Radium 223 Dichloride), Xtandi (Enzalutamide), or Zytiga (Abiraterone Acetate);
*for renal clear cell carcinoma:*
   Afinitor (Everolimus), Aldesleukin, Avastin (Bevacizumab), Axitinib, Bevacizumab, Everolimus, Inlyta (Axitinib), Nexavar (Sorafenib Tosylate), Pazopanib Hydrochloride,
   Proleukin (Aldesleukin), Sorafenib Tosylate, Sunitinib Malate, Sutent (Sunitinib Malate), Temsirolimus, Torisel (Temsirolimus), or Votrient (Pazopanibv Hydrochloride);
*for retinoblastoma:*
   Clafen (Cyclophosphamide), Cyclophosphamide, Cytoxan (Cyclophosphamide), or Neosar (Cyclophosphamide); for rhabdomyosarcoma: Cosmegen (Dactinomycin), Dactinomycin, Vincasar PFS (Vincristine Sulfate), or Vincristine Sulfate, and
*for the prevention of Cervical Cancer:*
   Cervarix (Recombinant Human Papillomavirus (HPV) Bivalent Vaccine), Gardasil (Recombinant HPV Quadrivalent Vaccine), Recombinant HPV Bivalent Vaccine, Recombinant HPV Quadrivalent Vaccine.

The pharmaceutical composition may comprise a compound of formula (I), or preferably the compound having the formula (II), and, alternatively or in addition to the additional therapeutic agents of groups (A) or (A') as set out above, one or more additional therapeutic agent(s) selected from the group (B) consisting of:
Acetylsalicylic acid, Diflunisal, Salsalate, Ibuprofen, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Nabumetone, Piroxicam, Meloxicam, Tenoxicam, Droxicam, Lornoxicam, Isoxicam, Mefenamic acid, Meclofenamic acid, Flufenamic acid, Tolfenamic acid, Celecoxib, Rofecoxib, Valdecosib, Parecoxib, Lumiracoxib, Etoricoxib, Firocoxib, Nimesulide, Licofelone, H-harpagide , Lysine clonixinate, salts thereof, and combinations thereof. Preferably, the one or more additional therapeutic agent(s) in this aspect is a selective cyclooxygenase-2 (COX-2) inhibitor. Accordingly, the additional therapeutic agent can be selected from the group (B') consisting of
Celecoxib, Rofecoxib, Valdecosib, Parecoxib, Lumiracoxib, Etoricoxib, Firocoxib, pharmaceutically acceptable salts thereof, and combinations thereof.

### Pharmaceutical formulations

Another aspect of the present disclosure relates to a pharmaceutical formulation comprising a compound of formula (I) or, preferably, formula (II), or of formula (III), or of formula (IV), or a pharmaceutical composition as described above (which may additionally comprise the above-referenced additional therapeutic agents, triple helix forming oligonucleotides (TFOs) and/or antimetabolites), and at least one pharmaceutically acceptable carrier.

The at least one pharmaceutically acceptable carrier is selected from the group consisting of peanut oil, sesame oil, cottonseed oil, corn oil, olive oil and mixtures of oils; ethyl oleate, isopropyl myristate, fatty acid glycerides, acetylated fatty acid glycerides; ethanol, isopropyl alcohol, hexadecyl alcohol, glycerol and propylene glycol, polyethyleneglycol, mineral oil, petrolatum; water, sterilized water, Ringer's solution, an isotonic aqueous saline solution, a dextrose solution, a glucose solution; β -cyclodextrin, hydroxypropyl-β-cyclodextrin; microcrystalline cellulose, crystalline cellulose, hydroxypropyl cellulose, hydroxypropyl methyl cellulose, methyl cellulose, carboxymethyl cellulose, sodium carboxymethyl cellulose, calcium carboxymethyl cellulose, cellulose acetate phthalate, cellulose acetate butyrate, hydroxyethyl cellulose, ethyl cellulose; and combinations thereof.

The pharmaceutically acceptable carrier can be selected from the group consisting of β-cyclodextrin, hydroxypropyl-β-cyclodextrin, hydroxypropyl cellulose, hydroxypropyl methyl cellulose, methyl cellulose, carboxymethyl cellulose, hydroxyethyl cellulose, ethyl cellulose, and combinations thereof. In certain preferred aspects, the pharmaceutically acceptable carrier can be selected from the group consisting of hydroxypropyl-β-cyclodextrin, carboxymethyl cellulose and combinations thereof.

The above referenced compounds, preferably the compound having the formula (II), or pharmaceutical formulations comprising a compound of any compound referred to above, preferably compound having the formula (II), comprise at least one pharmaceutically acceptable carrier as described above, whereby the said formulations are typically in the form of a capsule, cachet, tablet, lozenge, powder, granule, oral suspension or solution.

### Methods of treatment involving the compounds of formula (I)

The present document also describes a method of treating or preventing a cancerous disease in a mammal, comprising the administration of a therapeutically effective amount of the compound of the present invention, of the pharmaceutical composition of the present invention, or of the pharmaceutical formulation of the present invention to the mammal in need of such treatment. Such methods are not encompassed by the scope of the invention but only described to illustrate the field of the invention which relates to compounds for use in such methods.

### Compounds and pharmaceutical compositions for use in medicine

The invention relates to the compounds of formula (I) for use in treating a cancerous disease as defined in claim 1.

In some embodiments, said compounds are for use in treating or preventing a cancerous disease in a mammal. The mammal is preferably human. The cancerous disease is a cancer caused by or triggered by a PI3Kα mutation as defined in claim 1. Thus, the subjects to be treated by administering the compounds of the present invention exhibit a PI3Kα mutation which is susceptible to allosteric inhibition by the compounds of formula (I), namely a mutation in the gene encoding PI3Kα which leads to occurance of a PI3Kα mutant as described above, especially the H1047R mutant. The subjects are treated by administering the compounds for use of the present invention in order to prevent an outbreak of a cancerous disease or to treat an already existing cancerous disease.

In preferred embodiments, said compounds comprise a therapeutically effective amount of the above referenced compounds of formula (I) for administration to a subject in need thereof.

Any of the compounds of formula (I) described herein, preferably the compound of formula (II) or (III) or (IV), is preferably administered by a route selected from the group consisting of oral, buccal, sublingual, transdermal, transmucosal, intranasal, intravenous, intraperitoneal, intramuscular, subcutaneous and intrathecal administration, preferably by a route of oral, buccal, or sublingual administration.

In other embodiments of this aspect of the present invention, the additional therapeutic agent(s) is/are additionally or alternatively selected from the group (B) as described above in the compounds and pharmaceutical compositions section. Preferably, the additional therapeutic agent is one of the selective COX-2 inhibitors selected from the group (B') as described.

Preferably, the cancerous disease to be treated or prevented in the above embodiments of the present invention is selected from the group (C) consisting of:
acute monocytic leukemia, acute myelogenous leukemia, acute myelomonocytic leukemia, acute promyelocytic leukemia, adult T-cell leukemia, adult T-cell lymphoma, astrocytoma, atypical carcinoid lung cancer, basal cell carcinoma, B-acute lymphocytic leukemia, B-cell acute lymphoblastic leukemia/lymphoma, Bladder cancer, brain cancer, breast cancer, bronchial cancer, Burkitt's lymphoma, cancer of the bile duct, cancer of unknown primary origin, cervix cancer, chronic myeloproliferative disorder, colon cancer, diffuse large cell lymphoma, endometrial cancer, ependymoma, esophageal cancer, gastric cancer, glioma, glioblastoma, head and neck cancer, hemagiopericytoma, hepatocellular carcinoma, Hodgkin's lymphoma, Kaposi's sarcoma, kidney cancer, large cell neuroendocrine carcinoma, large granular lymphocytic leukemia, leukemia, liver cancer, lung cancer, lymphoma, medulloblastoma, melanoma, multiple myeloma, myelodysplastic syndrome, nasopharygeal cancer, neuroblastoma, NK cell tumor, non-Hodgkin's lymphoma, oesophageal squamous cell carcinoma, osteosarcoma, ovarian cancer, pancreatic cancer, peripheral T-cell leukemia, primary plasma cell leukemia, prostate cancer, renal clear cell carcinoma, retinoblastoma, rhabdomyosarcoma, sarcoma, small cell lung carcinoma, skin cancer, T-cell acute lymphoblastic leukemia, T-cell acute lymphoblastic lymphoma, testicular cancer, thymoma, thyroid cancer, urachal cancer, uterine cancer, vaginal cancer, and combinations thereof.

More preferably, the cancerous disease is selected from the group (C') consisting of B-acute lymphocytic leukemia, Burkitt's lymphoma, diffuse large cell lymphoma, multiple myeloma, primary plasma cell leukemia, atypical carcinoid lung cancer, bladder cancer, brain cancer, breast cancer, cervix cancer, colon cancer, gastric cancer, glioblastoma, hepatocellular carcinoma, large cell neuroendocrine carcinoma, liver cancer, endometrial cancer, medulloblastoma, melanoma, neuroblastoma, oesophageal squamous cell carcinoma, osteosarcoma, ovarian cancer, endometrial cancer, prostate cancer, skin cancer, renal clear cell carcinoma, retinoblastoma, rhabdomyosarcoma, small cell lung carcinoma, thyroid cancer, and combinations thereof.

The compounds as described herein above have been found to be particularly suitable for use in treating a cancerous disease selected from the group consisting of breast cancer, endometrial cancer, colon cancer, brain cancer, skin cancer, ovarian cancer, gastric cancer, lung cancer, thyroid cancer, head and neck cancer, cervical cancer, pancreatic cancer, liver/biliary tract cancer, pituitary tumors, urological tumors, leukemia/lymphoma, neuroblastoma, preferably particularly colon cancer, brain cancer, endometrial cancer, or breast cancer.

In some embodiments of the above aspects of the present invention, the cancerous disease is an adenocarcinoma. Preferably, the cancerous disease is in these embodiments selected from the group consisting of: pancreatic ductal adenocarcinoma, mammary adenocarcinoma, prostatic adenocarcinoma, endometrial adenocarcinoma, ovarian adenocarcinoma, gastric adenocarcinoma, liver adenocarcinoma, thyroid adenocarcinoma, head and neck adenocarcinoma, bladder adenocarcinoma, and gastrointestinal adenocarcinoma or lung adenocarcinoma and most preferably selected from pancreatic ductal adenocarcinoma, mammary adenocarcinoma, endometrial adenocarcinoma, ovarian adenocarcinoma, gastric adenocarcinoma, liver adenocarcinoma, thyroid adenocarcinoma, head and neck adenocarcinoma, bladder adenocarcinoma, and gastrointestinal adenocarcinoma, and lung adenocarcinoma.

### Method for the manufacture of compounds of formula (I)

Another aspect of the disclosure refers to a method for the manufacture of any compound of formula (I) described herein. In general, the compounds of formula (I) are prepared by alkylating dichloro analogs with formula (V) 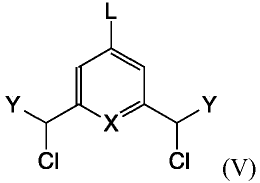
wherein L is independently H or OH; X is independently N or CH; Y is independently H or =O,
by reaction with a compound of formula R₁-Z-H, in which Z is independently S or NH, and R1 is independently a mono- or polycyclic aryl or heteroaryl selected from: 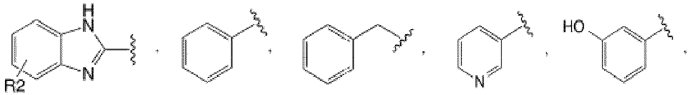 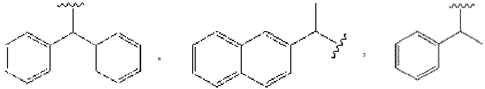 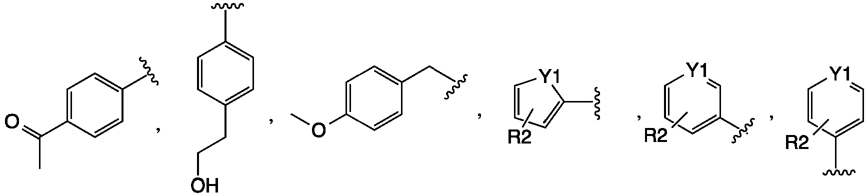
   wherein R₂ is independently at least one of hydroxyl, hydrogen, fluoro, chloro, or bromo;
   in the five-membered rings, Y₁ is independently CH₂, O, S, or NH,
whereas in the six-membered rings, Y₁ is independently CH, or N, to give the corresponding substituted benzyl or pyridine of formula (I). Optionally, the compound of formula (I) is subsequently converted to the corresponding, pharmaceutically acceptable salt form.

In a preferred aspect of the disclosure, the method relates to the preparation of the compound of formula (II), or its pharmaceutically acceptable salt form, by reacting a compound of formula R₁-Z-H, where R₁ and Z are defined as above, and a dichloride of formula (V) 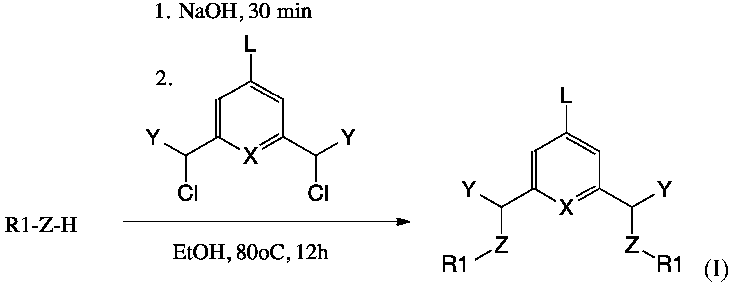 where R₁, L, Z, X are defined as above, and Y = H, to yield the compound of formula (II), and, optionally, converting it to its corresponding salt form.

In other preferred embodiments, the method relates to the preparation of the compounds of formula (III) or (IV), or their pharmaceutically acceptable salt form, by reacting a compound of formula R₁-Z-H, where R₁ and Z are defined as above, and a dichloride of formula (V) 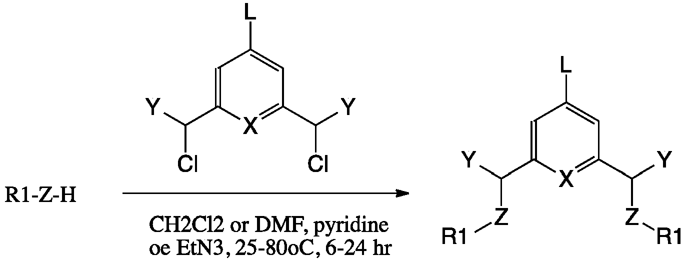 where R₁, L, Z, X are defined as above, and Y is =O, to yield the compound of formula (III), or (IV), and, optionally, converting it to its corresponding salt form.

Corresponding chlorides of formula (V) may be commercially available or may be synthesized with the following example scheme

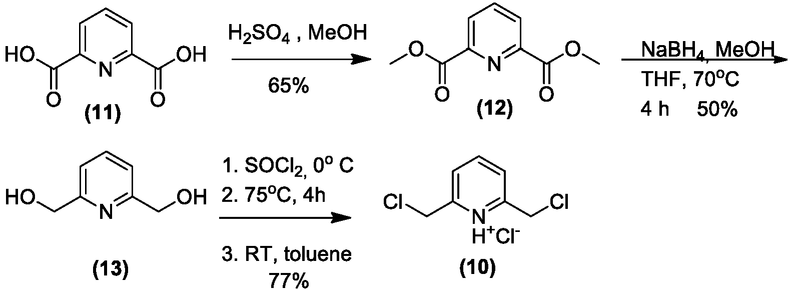

Preferably, the compounds of formula (I), and in particular of formula (II), may be synthesized according to the route depicted in Example 1 (cf. Example 1 for details on the reaction conditions, solvents etc.). It is an especially preferred embodiment of the invention to crystallize the product from H₂O and MeOH, resulting in a high yield and/or purity of the product. In this regard, it will be readily apparent to the person of skill in the art how to synthesize compounds of formula (I) other than the compound of formula (II) by carrying out similar reaction steps with the respective intermediates, analogously to the reaction scheme shown in Example 1.

Preferably, the compounds of formula (I), and in particular of formula (III), may be synthesized according to the route depicted in Example 2 (cf. Example 2 for details on the reaction conditions, solvents etc.). In this regard, it will be readily apparent to the person of skill in the art how to synthesize compounds of formula (I) other than the compound of formula (II) by carrying out similar reaction steps with the respective intermediates, analogously to the reaction scheme shown in Example 2.

Preferably, the compounds of formula (I), and in particular of formula (IV), may be synthesized according to the route depicted in Example 3 (cf. Example 3 for details on the reaction conditions, solvents etc.). In this regard, it will be readily apparent to the person of skill in the art how to synthesize compounds of formula (I) other than the compound of formula (II) by carrying out similar reaction steps with the respective intermediates, analogously to the reaction scheme shown in Example 3.

### Assay for determining the activity of PI3Kα or PI3Kα mutants.

Another aspect of the present disclosure is an activity assay for phosphatidylinositol 3-kinase α as already mentioned earlier in this specification. The assays described to date are either time-consuming, or require specialized equipment. The assay described here is fast and uses common equipment. By faithfully recapitulating the properties of the membrane bound enzyme, the assay assesses quantitatively the activity of PI3Kα.
The assay includes
a)-a solid phase where GST-GRP1-molecules become immobilized,
b) incubation of PI3Kα or PI3Kα mutant with buffer, ATP and PIP2, for the catalysis of the conversion of PIP2 to PIP3,
c) addition of competitor PIP3, carrying a detectable label or reporter molecule, and
d) determining enzyme activity based on the amount of PIP3 obtained in step b) which competes with competitor PIP3 for binding to the functionalized solid phase.
The assay is based on the high affinity and specificity by which PIP3, the product of the reaction catalyzed by PI3Kα, binds to proteins with pleckstrin homology (PH) domain, such as the General Receptor for Phosphoinositides Isoform 1 (GRP1 PH), whereas the substrate of the PI3Kα-catalyzed reaction, PIP2, binds only minimally to GRP1 (Lindsay et al. J Cell Sci. 119:5160-5168 (2006), Ferguson et al. Mol Cell 6:373-84 (2000), Fleming et al. Biochem J 351:173-182 (2000)). The inventive activity assay accordingly measures the activity of PI3Kα or PI3Kα mutants by determining the amount of enzymatically produced PIP3 in an indirect, competitive test format. Furthermore, the effect of (potential) inhibitor molecules on the activity of PI3Kα or PI3Kα mutants can be determined by means of the assay.

Preferably, the test is performed by providing a functionalized solid phase, preferably a well plate, onto which GRP1 molecules are fixated, preferably as a GST (glutathione-S transferase)-GRP1 complex bound to a glutathione-coated solid phase. To this purpose, GST-GRP1 can be produced by expressing a cDNA encoding the fusion protein GST-GRP1 in an appropriate expression system. Such expression system preferably is a bacterial expression system for recombinant protein expression, preferably an E.coli based expression system, especially an E.coli strain BL21(DE3) into which the cDNA is transformed after insertion into an appropriate expression vector. The expressed protein can e.g. be purified by affinity chromatography, preferably using a glutathione-Sepharose 4B chromatography material.

The assay includes addition of PIP3 competitor molecules, which preferably carry a detectable label or a reporter molecule which upon further reaction enables detection of the presence of the competitor-PIP3. In an especially preferred aspect, the competitor-PIP3 is biotin-PIP3 and in a further reaction a label which is present on the biotin binding partner streptavidin is used for detection. An especially preferred label is horseradish peroxidase (HRP) which when contacted with an appropriate substrate provides for a readily detectable colour or fluorescence signal. Quantification may be performed by adding a binding partner of biotin which carries a detectable label, e.g. a streptavidin-HRP -conjugate, followed by incubation with a substrate of HRP that produces a detectable product, e.g. OBD which is converted into a product that absorbs light at 492 nm. Quantification of the amount of captured competitor-PIP3 can be performed directly or at a later stage of the test procedure.

In one preferred manner of performing the assay, the competitor-PIP3 is added directly to the functionalized solid phase and forms a stable complex with the GST-GRP1-complex on the solid phase whereupon captured competitor-PIP3 is quantified after removal of excess reactants.

In another preferred aspect of the disclosure, the competitor-PIP3 is added to the solid phase together with or after addition of the PIP3 product of the PI3K*α* or PI3K *α* mutant catalyzed reaction. Quantification in all these cases allows to determine the amount of enzymatically produced PIP3 due to the competitive binding of PIP3 and competitor-PIP3 to the solid phase and the proportionally reduced signal provided via the detectable label included with the competitor-PIP3. The higher the activity of PI3K*α* or PI3K*α* mutant, the greater is the amount of PIP3 which is produced and the lower amount of competitor-PIP3 will be bound to the solid phase. Comparison with a standard curve of the signal provided by the label included with the competitor-PIP3 allows for an assessment of the relative activity of PI3K*α* or PI3K*α* mutant.

A PI3K*α* or PI3K*α* mutant catalyzed reaction is preferably set up by providing the enzyme(s) in a reaction mixture together with ATP and with PIP2 as the enzyme reaction substrate. In a preferred aspect, ATP is added to the enzyme(s) and a preincubation step is performed. Such preincubation is preferably effected at a temperature of 0°C to 35°C, more preferably at 15°C to 30°C and most preferably at room temperature of about 20°C to about 28°C, especially 25°C. The preincubation step usually is performed for a time period of about 5 to 20 minutes, depending on the temperature and overall conditions.

The enzymatic reaction is then initiated by adding a substrate solution containing PIP2. As the substrate for the enzymatic reaction, a water-soluble PIP2 can be used carrying short side chains (Gray et al., Anal Biochem 313(2):234-245 (2003), Carson et al., Biochem J 409(2):519-524 (2008)). In such case, all reagents are present in the water phase. Furthermore, "natural" PIP2 contained in liposomal membranes and carrying long, natural fatty chains can be employed (Lingaraj et al., J Biomol Screen 13(9):906-911 (2008), Mandelker et al., Proc Natl Acad Sci U S A 106(40):16996-17001 (2009)). In such case, the reaction mixture contains two phases and the enzymatic activity is exerted at the interphase between membranes and water, which even better simulates the physiological microenvironment of the enzymatic reaction at cellular membranes.

The reaction mixture including the enzyme or enzyme mutant and the substrate in an appropriate solution, preferably a buffered solution, and ATP is incubated for a further appropriate time period, preferably 5 to 20 minutes and especially 8 to 12 minutes, at the same temperature as indicated above for the optional preincubation step. After termination of the reaction, the mixture which now includes enzymatically produced PIP3 is transferred to contact the GST-GRP1 functionalized solid phase. As mentioned above and as the skilled person will be well aware of, the competitor PIP3 can be contacted with the solid phase already earlier or added simultaneously with the reaction mixture subsequently. Determining the signal provided by the label or the reporter molecule of the competitor PIP3 and the reduction thereof as compared to an earlier signal obtained for the functionalized solid phase without the reaction mixture or compared to standard curve provided for the determination of PIP3 amounts allows for quantification of the enzymatically produced PIP3.

The assay allows to measure the activity of PI3Kα or the PI3Kα mutant with and without the presence of a potential inhibitor or modulator molecule included in the reaction mixture. In the context of the present disclosure, the assay is a valuable asset to determine the effect of such potential inhibitor or modulator on the proteins and thus their potential use in medicine, especially cancer treatment as explained above in detail.

### EXAMPLES

### Example 1. Synthesis scheme for the generation of the compound of formula (II) (Reference Example)

### Preparation of 2,6-bis(((1H-benzo[d]imidazol-2-yl)thio)methyl)pyridine

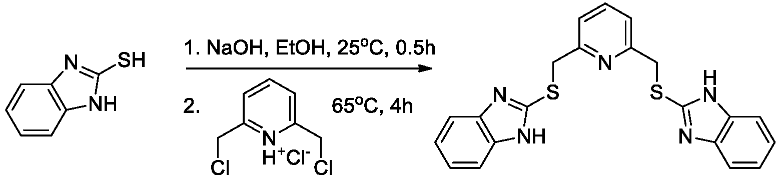

**Preparation of 2,6-bis(((1*H*-benzo[*d*]imidazol-2-yl)thio)methyl)pyridine.** In a solution of 1*H*-benzo[*d*]imidazole-2-thiol (100 mg, 0.67 mmol) in EtOH under argon atmosphere, NaOH was added (27 mg, 0.67 mmol). The reaction mixture was stirred in 25⁰C for 30 min. Next, a solution of 2,6-bis(chloromethyl)pyridine, in its hydrochloric acid salt form, was added (72 mg, 0.34 mmol) in EtOH (1.7 mL) and the reaction was refluxed at 65°C. After 4 hours the reaction was cooled down to 25⁰C and a solution of NaOH 1N (0.5 mL) was added until pH = 9. The reaction was concentrated in vacuo and the product was crystallized from H₂O and MeOH (114 mg, white solid in 85% yield).
**¹H NMR (500 MHz) 25 °C, DMSO:** *δ* 12.63 (s, 2H, -NH-), 7.71 (t, *J =* 7.7 Hz, 1H, Ar*H*), 7.44 (s, 6H, ArH), 7.11 (dd, *J =* 5.4, 2.8 Hz, 4H, ArH), 4.65 (s, 4H, - C*H*₂-)*.*
**MS:** [M-H]⁻_{calc.}: 402.1; [M-H]⁻_{exper.}: 401.7

### Example 2. Synthesis scheme for the generation of the compound of formula (III) (Reference Example)

### Preparation of N²,N⁶-dibenzylpyridine-2,6-dicarboxamide

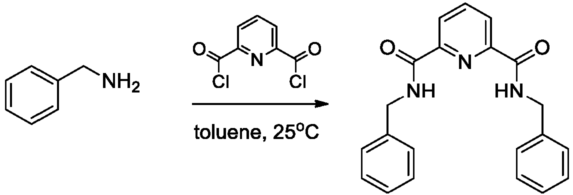

**Preparation of *N*²,*N*⁶-dibenzylpyridine-2,6-dicarboxamide.** In a solution of phenylmethanamine (0.2 mL, 0.49 mmol) in toluene (3 mL), pyridine-2,6-dicarbonyl dichloride (50 mg, 0.25 mmol) was added. The reaction mixture was stirred for 12 hours at 25°C under argon atmosphere. Next, aqueous solution of NaOH 1M (3 mL) is added in the reaction mixture and the reaction is extracted with EtOAc. The organic layer was washed with saturated aqueous NaCl solution, dried over Na₂SO₄, and condensed under reduced pressure. The emerging solid was crystallized from toluene to produce *N*²,*N*⁶-dibenzylpyridine-2,6-dicarboxamide (60 mg, 70% yield).
**¹H NMR (500 MHz) 25 °C, DMSO:** *δ* 8.39 (d, *J =* 7.8 Hz, 2H, ArH), 8.04 (t, *J =* 7.5 Hz, 1H, Ar*H*), 7.33 - 7.28 (m, *J =* 3.9 Hz, 10H, Ar*H*), 4.65 (s, 4H, -C*H*₂-) ppm
**MS:** [M+H]⁺_{calc.}: 346.2; [M+H]⁺_{exper.}: 345.8

### Example 3. Synthesis scheme for the generation of the compound of formula (IV) (Reference Example)

### Preparation of N²,N⁶-bis(3-hydroxyphenyl)pyridine-2,6-dicarboxamide

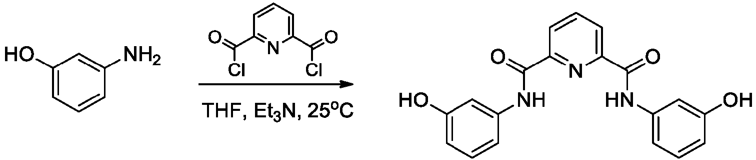

**Preparation of *N*²,*N*⁶-bis(3-hydroxyphenyl)pyridine-2,6-dicarboxamide.** In a solution of 3-aminophenol (54 mg, 0.49 mmol) together with Et₃N (70 µL, 0.49 mmol) in THF (1 mL), pyridine-2,6-dicarbonyl dichloride (50 mg, 0.245 mmol) in THF (2.5 mL) at -20 °C, was added. The reaction mixture was stirred for 2 hours at 25°C under argon atmosphere. The reaction mixture was condensed under reduced pressure and the emerging solid was crystallized from MeOH to produce *N*²,*N*⁶-bis(3-hydroxyphenyl)pyridine-2,6-dicarboxamide (25 mg in 29% yield).
**¹H NMR (500 MHz) 25 °C, DMSO:** *δ* 10.90 (s, 2H, -NH- or -OH), 9.53 (s, 2H, -N*H*- or -O*H*), 8.38 (d, *J =* 7.6 Hz, 2H, Ar*H*), 8.29 (t, *J =* 7.6 Hz, 1H, Ar*H*), 7.47 (s, 2H, Ar*H*), 7.32 (d, *J* = 7.9 Hz, 2H, Ar*H*), 7.21 (t, *J =* 8.0 Hz, 2H, Ar*H*), 6.59 (d, *J* = 7.8 Hz, 2H, Ar*H*) ppm
**MS:** [M+H]⁺_{calc.}: 350.1; [M+H]⁺_{exper.}: 349.7

### Example 4. In vitro cell-free PI3Kα activity assays using PIP2 containing liposomes for PI3K-010.

To evaluate the *in vitro* activity of the compound having the formula (II) (PI3K-010), PI3Kα activity assays were performed using PIP2 containing liposomes (Gkeka et al PLOS Comp Biol 2014). In this assay, PIP3 molecules produced by PI3Kα compete with biotinylated PIP3 for binding to recombinant GST-GRP1-PH domain (amino acids 263- 380), which has been produced in bacteria, bound to glutathione coated 96-well plates. Quantitation of the competed amount of biotin-PIP3 is estimated by the peroxidase activity of streptavidin-HRP that binds to the plate and provides a measure for the activity of the protein. The compounds were preincubated with all the components of the assay mixture, except PIP2, for 10 min, at 25°C. PIP2 is added in the end of the preincubation period, thereby initiating the kinase reaction.

IC50 values for compound with formula (II) 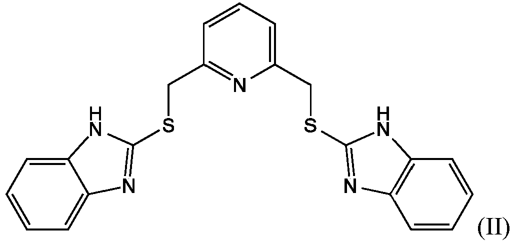 were calculated from dose- response curves using logit-log plots for the wild type PI3Kα protein as well as the mutated PI3Kα protein containing the H1047R mutation.

All assays were carried out in at least two independent experiments and each concentration in triplicate. PIK-108 and wortmannin were used as controls (Gkeka et al, 2014 J Phys Chem B). The compound with formula (II), PI3K-010, showed an 11-fold inhibition selectivity for the mutant PI3Kα H1047R protein compared to the wild-type PI3Kα (cf. Figure 1).

Next, experiments in low (100µM) and high ATP (2mM) concentrations were performed in order to identify whether PI3K-010 is an ATP-competitive inhibitor. We found that ATP concentration is only slightly affecting the IC50 of PI3K-010, and thus this compound can be considered a non-competitive inhibitor (cf. Figure 2).

### Example 5. In vitro cell-free PI3Kα activity assays using PIP2 containing liposomes for PI3K-021.

To evaluate the *in vitro* activity of the compound having the formula (III) (PI3K-010), PI3Kα activity assays were performed using PIP2 containing liposomes (Gkeka et al PLOS Comp Biol 2014). In this assay, PIP3 molecules produced by PI3Kα compete with biotinylated PIP3 for binding with recombinant GST-GRP1-PH domain (amino acids 263- 380), which has been produced in bacteria, bound to glutathione coated 96-well plates. Quantitation of the competed amount of biotin-PIP3 is estimated by the peroxidase activity of streptavidin-HRP that binds to the plate and provides a measure for the activity of the protein. The compounds were preincubated with all the components of the assay mixture, except PIP2, for 10 min, at 25 °C. PIP2 is added in the end of the preincubation period, thereby initiating the kinase reaction.

IC50 values for compound with formula (III) 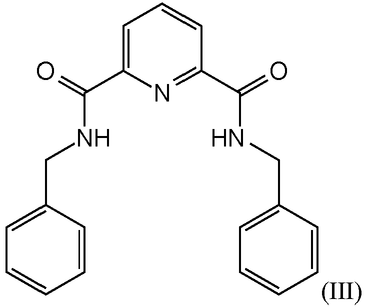 were calculated from dose-response curves using logit-log plots for the wild type PI3Kα protein as well as the mutated PI3Kα protein containing the H1047R mutation.

All assays were carried out in at least two independent experiments and each concentration in triplicate. The compound with formula (III), PI3K-021, showed a selectivity of >100 fold for the mutant PI3Kα H1047R protein compared to the wild-type PI3Kα (Figure 3). The IC50 of PI3K-021 was found to be 13.5 µM for the mutant H1047R PI3Kα, while for the WT the exact IC50 value could not be estimated due to the very low inhibition of PIK-021 against the WT protein even in the highest tested concentrations of PI3K-21. By extrapolation we estimated the IC50 of PI3K-021 to be > 1000 µM for the WT PI3Kα (cf. Figure 3).

### Example 6. In vitro cell-free PI3Kα activity assays using PIP2 containing liposomes for PI3K-024.

To evaluate the in vitro activity of the compound having the formula (IV) (PI3K-024), PI3Kα activity assays were performed using PIP2 containing liposomes (Gkeka et al PLOS Comp Biol 2014). In this assay, PIP3 molecules produced by PI3Kα compete with biotinylated PIP3 for binding with recombinant GST-GRP1-PH domain (amino acids 263- 380), which has been produced in bacteria, bound to glutathione coated 96-well plates. Quantitation of the competed amount of biotin-PIP3 is estimated by the peroxidase activity of streptavidin-HRP that binds to the plate and provides a measure for the activity of the protein. The compounds were preincubated with all the components of the assay mixture, except PIP2, for 10 min, at 25 ° C. PIP2 is added in the end of the preincubation period, thereby initiating the kinase reaction.

IC50 values for compound with formula (IV) 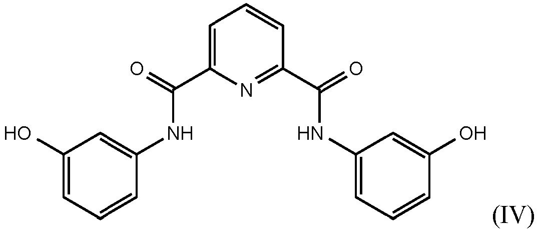 were calculated from dose- response curves using logit-log plots for the wild type PI3Kα protein as well as the mutated PI3Kα protein containing the H1047R mutation.

All assays were carried out in at least two independent experiments and each concentration in triplicate. The compound with formula (IV), PI3K-024, showed showed nanomolar inhibition for both WT and mutant proteins (IC50 = 6 nM for the WT and 64 nM for the mutant H1047R PI3Kα proteins) (cf. Figure 4).

### Example 7. PI3K-010 or PI3K-021 treatment of xenografts generated by heterotopic transplantation of the breast cancer MDA-MB-231 cell line or the breast cancer HCC1954 cell line.

The inventors evaluated the *in vivo* activity of the compound having the formula (I) (PI3K-010) and of the compound having the formula (II) (PI3K-021), on mouse bearing heterotopic xenografts after subcutaneous injection of cells from the breast cancer MDA-MB-231 cell line, which is wild-type PIK3CA and of the breast cancer HCC1954 cell line, which bears exon 20 H1047R mutation on PIK3CA. For transplantation, eight-week old NOD/SCID mice were anesthetized using a steady flow of a mixture of isofluorane gas (4%) and oxygen and inoculated subcutaneously into their lateral flanks with 10⁶ cancer cells and monitored daily until the tumors became palpable (tumor volume ~100 mm³). The mice were then divided randomly into two groups for PI3K-010 or vehicle administration for a period of two weeks. Administration was achieved by mixing PI3K-010 or PI3K-021 with corn oil and subsequent oral dosing in 100mg/Kg doses twice a day. Average weights + S.E.M (n=7 for MDA-MB-231 and n-5 for HCC1954) are indicated. Figure 5 illustrates the gross appearance of surgically recovered PI3K-010-treated or PI3K-021 or untreated (control) xenografts.

### Example 8. Micro PET/CT imaging of WAPCre/exon20 Pik3ca*/MMTV-Myc mouse

PET/CT images of a WAPCre/exon20 Pik3ca*/MMTV-Myc mouse were acquired at the beginning and end of treatment (two weeks) of breast cancer with the PI3K-010 compound (cf. Figure 6). The compound was administered per os (by gavage) at 100mg/Kg doses. The control mouse received only solvent. The diminished uptake of 18F-FDG by remnants of breast cancer is evident. The white arrows indicate the anatomical sites of breast. High-level concentration of 18F-FDG in the urinary bladder (excretion of the tracer) and also in the heart is noted. To further study the effect of PI3K-010, we surgically removed breast tumors. Immunohistochemical analysis of the xenografts using an antibody against the cell proliferation marker Ki67 showed tumor necrosis and reduced proliferation after treatment in comparison with the controls (proliferation index 15.0+3.3% and 69.9+5.1%, respectively)

### Example 8. Establishment of a new membrane-based activity assay for phosphatidylinositol 3-kinase α.

This example describes the development of a method for the measurement of lipid PI3Kα activity which is quite robust, easy to carry out, quick, inexpensive, does not need specialized equipment and can be used in a high throughput mode.

As described above, the central idea of the assay is based on the high affinity and specificity by which PIP3, the product of the catalyzed reaction, binds to proteins with pleckstrin homology (PH) domain, such as the General Receptor for Phosphoinositides Isoform 1 (GRP1 PH). This assay was used to determine the activity of PI3Kα wild type and oncogenic mutants H1047R and E545K in the basal state but also when physiologically activated by phosphorylated receptors tyrosine kinases. In the process of validating the assay three different types of PIP2 substrates were tested, a water soluble diC8PtdIns(4,5)P2 and two "liposomal" forms where different quantities of long acyl side chains PIP2 were incorporated to natural lipids that were extracted from HCT116 cells, so as to simulate as best as possible the physiological membrane status. The assay was also used to test and evaluate several "denovo" synthesized compounds in order to find mutant specific inhibitors.

### Materials and Methods

### Preparation of liposomes

For the preparation of PIP₂-containing liposomes lipids were prepared from HCT116 cells. HCT116 is a colorectal cancer cell line in which the *PIK3CA* gene carries the H1047R mutation in exon 20. Extracted lipids were mixed with PIP₂ (containing long side chains, from brain, Avanti) and the mixture was dried under N₂ stream. Two liposomal preparations were made, containing 10% or 50% PIP₂. After the sample was completely dried, it was left for another 30 minutes under the N₂ stream, and it was further dried in a speed vacuum for 1 h. Then, an aqueous solution (20 mM Tris pH=7.4, 100 mM KCl, 1 mM EDTA pH=8 and 1 mM EGTA) was added and the lipid mixture was incubated at room temperature for 1 h, with occasional vortexing (every 10 min). Subsequently, the sample was subjected to 5 freeze/thaw cycles, followed by sonication in a waterbath for 30 minutes. Finally, the preparation was extruded using the Avanti mini-Extruder (according to manufacturer's instructions), with a 100 nm membrane, which generates a homogeneous preparation of small unilamellar vesicles with diameter below 100 nm.

### Expression and purification of GST-GRP1 domain

The cDNA of GST-GRP1 was transformed into the Escherichia Coli strain BL21 (DE3). GST-GRP1 was affinity purified on glutathione-Sepharose 4B (Amersham Pharmacia), using the manufacturers standard protocols. The purity of the protein was determined by SDS-PAGE.

### PI3Kinase activity assay

96-well plates, coated with glutathione, were incubated with blocking buffer (50 mM Tris pH=7.4, 150 mM NaCl, 0.05% Tween and 0.2% gelatin) for 1 h at room temperature (RT), under shaking. Then, 1 µg purified GST-GRP1 was added to each well and incubated for 1 h at RT, under shaking. Finally, the wells were washed 5 times with wash buffer (50 mM Tris pH=7.4, 150 mM NaCl and 0.2% gelatin), for 2 min per washing step. In parallel, PI3K reactions were set up in micro-tubes by the addition of 5 µl reaction buffer (250 mM Tris pH=7.4, 10 mM MgCl₂), 2.5 µl ATP (100 µM), 2 µl BSA (10 µg/µl), 1-2 µl PI3K (2 ng/µl), 1 µl of the compound (inhibitor) under investigation, and H₂O to a final volume of 20 µl, and the mixture was incubated for 10 min at 25°C (pre-incubation step). The reaction was initiated by the addition of 5 µl substrate solution (containing 40 ng/µl PIP₂) (200 ng total PIP₂) and allowed to proceed for 8 to 12 min at 25°C. The reaction was terminated by the addition of 75 µl stop solution, which contains 5 mM EDTA, 1% BSA, 0.07% sodium deoxycholate and 0.3 ng biotinylated-diC8PtdIns(3,4,5)P3 (biotin-PIP₃), pH=8, in wash buffer, and the whole mixture was transferred to the wells of the 96-well plate (which contains GST-GRP1, prepared as described above) and was incubated for 1 h at RT, under shaking (during this step, the PIP₃ product of the PI3K reaction competes with biotin-PIP₃ for binding to GST-GRP1). The wells were washed 5 times with wash buffer, 2 min each, and streptavidin-HRP conjugate was added and incubated for 40 min at RT under shaking. The wells were washed 5 times, 2 min each, and the substrate of HRP (OPD, o-phenylenediamine) was added and incubated for 1-20 min (until the development of a brown colour). The absorbance of the solution in the wells was read at 492 nm using an ELISA reader. For the evaluation of inhibitors, or the phosphorylated PDGFR peptide, or the screening of the new compounds, 1 or 2 µl of the tested molecule (in the appropriate buffer) was added to the PI3K reaction mixture before the pre-incubation step, before the addition of the PIP₂ substrate. As a control, the effect of an equal volume of the solvent was tested under the same conditions.

To calculate the amount of PIP3 produced by the enzymatic reaction (in AU), which allows evaluation of the levels of PI3K activity, a standard curve of the A₄₉₂ₙₘ against PIP3 was prepared. To generate the standard curve, i.e. absorbance - versus - arbitrary units of PIP3 molecules, a PIP3 stock solution was used that was prepared by incubating PIP2 with PI3K under the regular PI3K reaction conditions. This final preparation served as the standard of PIP3 molecules (in AU) for the generation of the standard curve. The standard curve follows the anticipated hyperbolic profile of antagonistic inhibition between PI3K-produced PIP3 and b-PIP3 for binding to GST-GRP1. Although the use of the above standard curve allows only assessment of the amount of PIP3 molecules in arbitrary units (AU), this measurement is sufficient for comparative in vitro studies, e.g. experiments that address the molecular mechanisms regulating the activity of PI3K, or in small molecule screening studies aiming to identify inhibitors of PI3K.

### Results

### Principle of the new PI3Kinase α activity assay

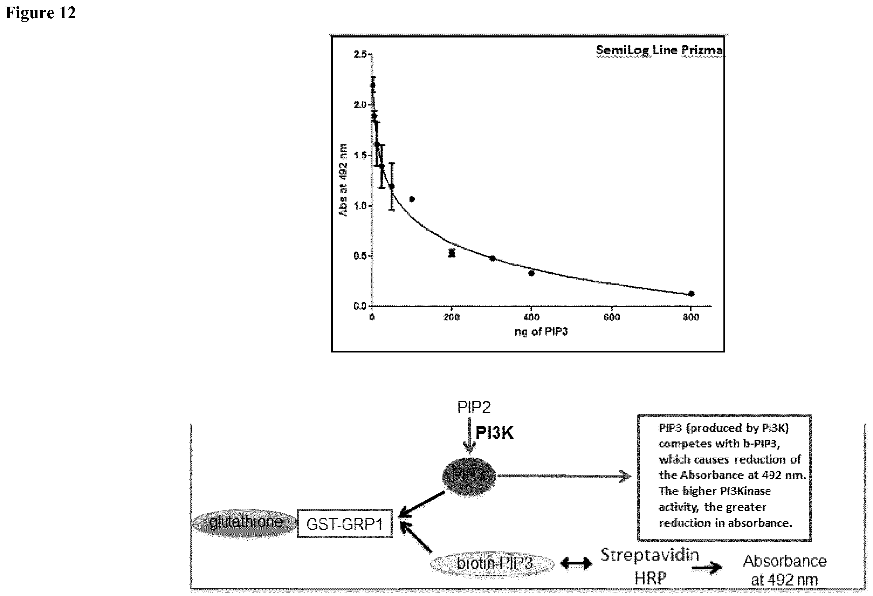

To establish a simple, membrane-based PI3Kα activity assay, we took advantage of the fact that the product of the catalytic activity of PI3Kα, the membrane lipid PIP₃, binds with high affinity and selectivity to the PH domain of GRP1, while the substrate of the reaction, PIP₂, binds only minimally to GRP1 (Lindsay et al. J Cell Sci 119:5160-5168 (2006): Ferguson et al. Mol Cell 6:373-384 (2000); Fleming et al. Biochem J 51:173-182 (2000)). Thus, to assay the activity of PI3Kα, we used the PH domain of GRP1 fused to GST (for simplicity, from here on called GST-GRP1), bound to glutathione-coated 96-well plates, to capture the PIP₃ molecules produced by PI3K. To quantify the amount of produced PIP₃, we employed an indirect, competition-based assay. To this end, following the termination of the enzymatic reaction, biotin-PIP₃ is added exogenously to the mixture containing the PI3Kα reaction products (to function as a competitor of the binding between GRP1 and PIP3), and the whole mixture is incubated with the wells of the 96-well plate (Figure 7); during this incubation, both PIP₃ (the product of the PI3K reaction, shown in red) and biotin-PIP₃ (added exogenously) form independent complexes with GST-GRP1 in a competitive manner. Finally, streptavidin-HRP is added to the wells, to assess the amount of bound biotin-PIP₃. When PI3K is present, the higher the PI3Kα activity, the greater amount of PIP₃ is produced, the lower amount of biotin-PIP₃ & streptavidin-HRP will be bound to the plate, which leads to a lower value of the absorbance at 492 nm (Figure 7). On the contrary, in the presence of a PI3Kα inhibitor, the levels of PIP₃ are reduced proportionally to the effectiveness of the inhibitor, which results in a higher binding of biotin-PIP₃ & streptavidin-HRP to the wells of the plate, thereby leading to a higher absorbance at 492 nm. Thus, the stronger the inhibitor, the higher the absorbance. A standard curve of the absorbance at 492nm against increasing levels of PIP₃ allows assessment of the relative activity of PI3Kα in arbitrary units (see further below, Fig. 12).

### Establishment of the conditions of the PI3K activity assay

All reagents and conditions of the assay have been optimized with the aim to achieve low noise, maximum possible dynamic range and specificity. At first the amount of GST-GRP1 that should be bound to plates to capture sufficient amount of biotin-PIP₃, was optimized to produce a high absorbance at 492 nm. GST-GRP1 was expressed in bacteria and was purified using glutathione-coated beads (Fig. 8a). To determine the optimum amount of GST-GRP1 that results in sufficiently high signal, increasing amounts of GST-GRP1 were added to glutathione coated plates, followed by incubation with biotin-PIP3, streptavidin-HRP and measurement of the absorbance at 492 nm. As shown in Fig. 8a, the optimum quantity of GST-GRP1 was 3 µg, as a higher amount of the protein (10 µg) did not significantly improve the absorbance at 492 nm.

Then, the amount of b-PIP3 that should be used in the assay was optimized. As expected, we observed that the higher the amount of b-PIP3 added to the plate, the higher the absorbance (Fig. 9). Among the amounts tested, the quantity of 0.3 µg of b-PIP3 resulted in sufficiently high absorbance, while higher amount of b-PIP3 did not drastically increase the signal (Fig. 9). Since the assay is based on the competition between exogenous b-PIP3 and PIP3 produced by PI3K, the higher b-PIP3 added in the assay (beyond the amount required to produce an acceptable signal), the larger amounts of PI3K and PIP2 would be required for the competition in the assay mixture, which would raise the cost for screening libraries of compounds. Therefore, the amount of 0.3 µg was selected for subsequent assays.

Next the optimum concentration of the reagents used in the PI3K reaction was determined, starting from the substrate PIP₂. Although a soluble version of PIP₂ with short side chains (diC8) can be used in the assay, here the inventors sought to employ as a substrate the natural PIP₂ (which contains long, natural fatty chains), in liposomal membranes. In this case, in the reaction mixture containing two phases (water and membranes), the enzymatic activity is exerted at the interphase between membranes and water, which simulates better the physiological microenvironment of the enzymatic reaction at cellular membranes. At first, given that PIP₂ binds to some extent to GST-GRP1 (despite the high specificity of GST-GRP1 for PIP₃ (Lietzke et al., Mol Cell 6(2):385-394 (2000), He et al., J Lipid Res 49(8):1807-1815 (2008)), we tested whether PIP₂ interferes with binding of biotin-PIP₃ to GST-GRP1. When PIP₂ was used as a sole component of the liposomal membranes (in the absence of other lipids), there was substantial inhibition of the signal even at the lowest concentration of PIP₂ (50 ng/100 µl reaction) (Fig. 10a), probably due to competitive inhibition between PIP₂ and biotin-PIP₃ for binding to GST-GRP1 (Corbin et al, Biochemistry 43(51):16161-16173 (2004), He et al., J Lipid Res 49(8):1807-1815 (2008), Lai et al., J Mol Biol 425(17):3073-3090 (2013)). On the other hand, the interference of PIP₂ with the signal was significantly reduced when PIP₂ was incorporated in liposomes together with natural lipids extracted from HCT116 cells (compare the sample of Fig. 10b containing 200 ng PIP2 at a concentration of 10% in liposomes, with the corresponding sample in Fig. 10a), probably due to the different conformation, availability, and/or micro-environment of PIP₂ when it is incorporated in the membranes together with other lipids. When a high concentration of PIP2 was used in liposomes (50%), there was inhibition of binding of biotin-PIP3 to GRP1 despite the presence of liposomes, which was substantially rescued by the addition of sodium deoxycholate, at a concentration below the CMC. Thus, in all subsequent experiments the substrate PIP₂ was integrated in liposomal membranes, which have been prepared using lipids extracted from cellular membranes, while deoxycholate was added to reduce non-specific binding of lipids to GRP1.

Finally, the inventors moved to the last step of the establishment of the assay, where they determined the optimal combination of PI3K levels relatively to different amounts of the substrate PIP₂ (prepared in liposomes). Testing a stable amount of PIP2 (200 ng) in two different lipid ratios, 10% PIP2 versus 50% PIP2 in liposomes, revealed that the high concentration of PIP2 (50% in liposomes) is critical for PI3K to achieve high levels of activity. Additionally, when the soluble form of PIP2 was used as a substrate (diC8PtdIns(4,5)P2), in which the hydrocarbon chains of the ester are short, as much as 2 µg of PIP2 were required to achieve less than half of the activity reached with 200 ng of PIP2 at 50% concentration in liposomes. Thus, the watersoluble form of PIP2 is a much worse substrate in comparison to lipid PIP2 (50%) in liposomes. Therefore, the lipid form of PIP2, at 50% concentration in liposomes, is the form chosen for subsequent experiments.

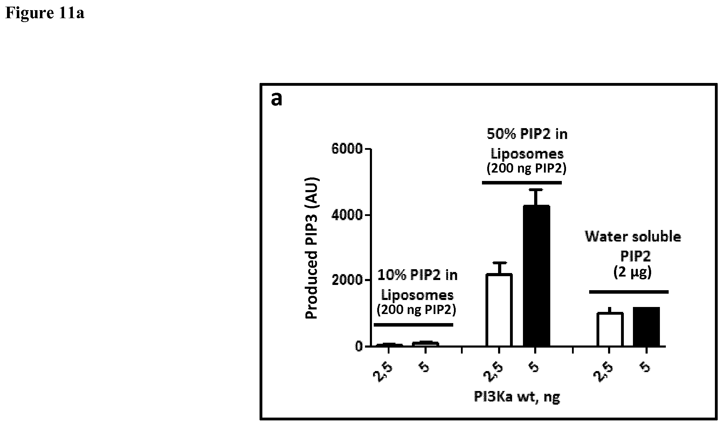

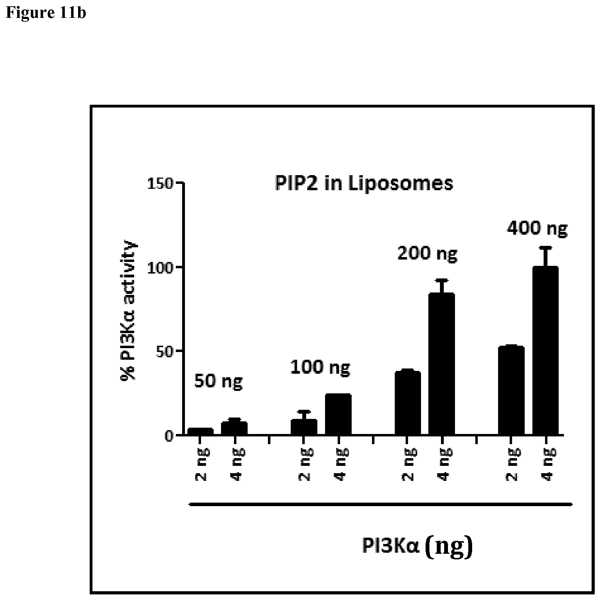

To identify the optimal amount of PIP2 for the assay (in the form of 50% in liposomes), we tested the combination of increasing quantities of PIP₂ with two different amounts of PI3K, 2 and 4 ng. As shown in Fig. 11b, at 200 ng of PIP₂ the reaction reaches a plateau, i.e. the levels of produced PIP3 are high enough to almost completely block the binding of biotin-PIP3 to GST-GRP1. Since the substrate needs to be in excess in order to exclude the possibility of being a limiting factor for the reaction, the amount of 200 ng of substrate was used in all subsequent experiments.

Given that the assay is based on the competition between the product (PIP₃) and biotin-PIP₃ for binding to GST-GRP1, the drop of the absorbance when PIP3 is present is not expected to be directly proportional to the amount of PIP₃ produced by PI3K. Therefore, a standard curve is necessary to convert the absorbance values, determined by the assay, to the amount of PIP3 produced by PI3K. As expected, the experimental standard curve (absorbance at 492 nm versus the amount of PIP3, added exogenously) follows a hyperbolic profile, typical for an assay where an antagonist (PIP3, produced by PI3K), competitively removes the agonist (biotin-PIP3) which is responsible for the measured signal (absorbance at 492 nm).

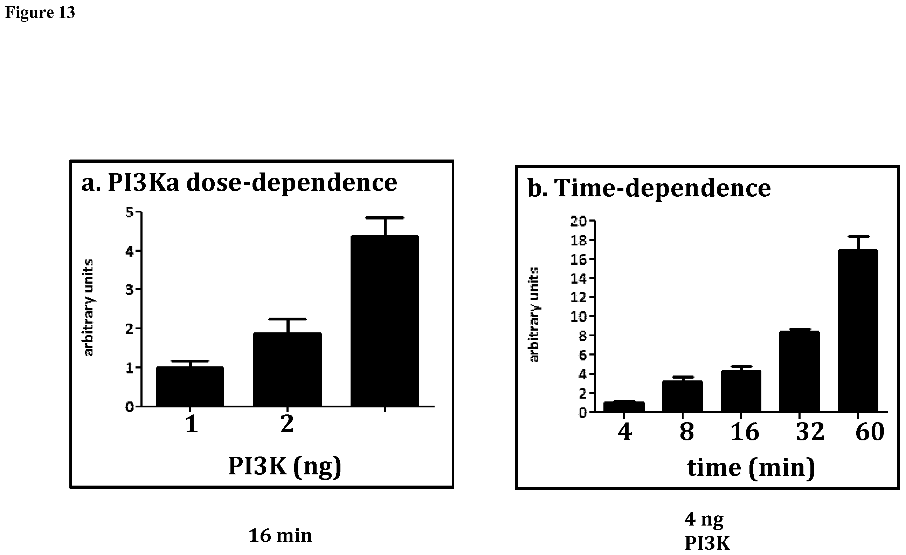

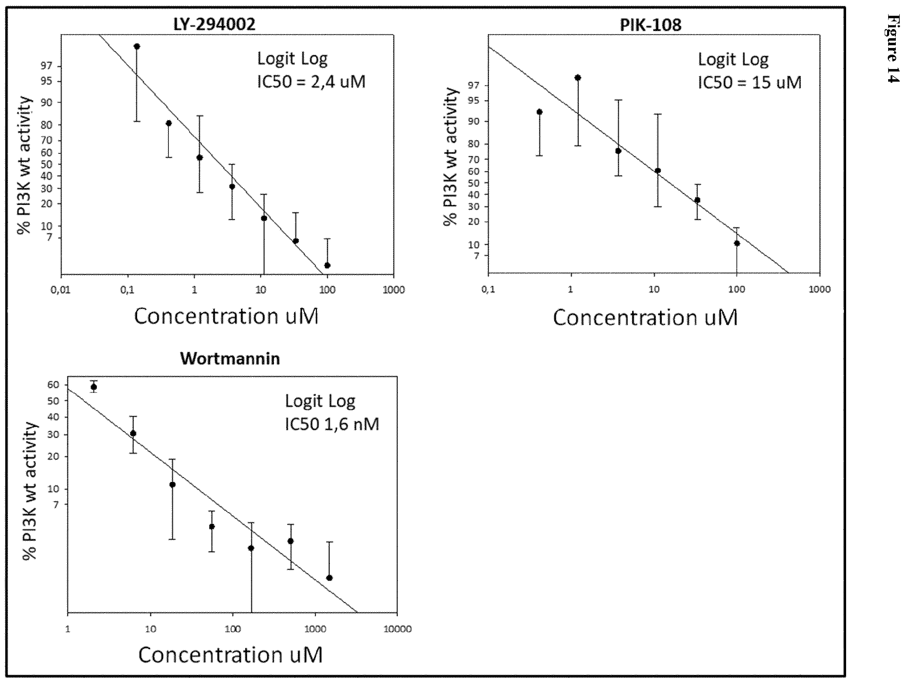

Having established the conditions of the PI3K activity assay, allowing assessment of the activity of PI3K in arbitrary units, we proceeded to the validation of the specificity of the method. At first, we tested whether the assay fulfils the hallmarks of the enzymatic reactions, i.e. dependence on the dose of the enzyme and on the duration of the reaction. Indeed, the quantified enzymatic activity was PI3K dose-dependent (Fig. 13a) as well as time dependent (Fig. 13b). As a further validation test of the assay, we employed well-known PI3Kinase inhibitors to test their effect on the activity of the enzyme. Data in figure 14 show that the IC50 values of known inhibitors, such as Wortmannin, LY-294002 and PIK-108, are in accordance with what is reported in the literature (figure 14).

### References

André F et al. Lancet Oncol 15:267-274 (2014).
Bagal et al. J Med Chem 62:247-265 (2019)
Burris HA III. Cancer Chemother Pharmacol 71(4):829-842 (2013).
Carson JD et al. Biochem J 409(2):519-524 (2008)
Changeux JP. Drug Discov Today Technol. 10:e223-228 (2013).
Corbin et al. Biochemistry 43(51):16161-16173 (2004).
Dienstmann R et al. Mol Cancer Ther 13(5):1021-1031 (2014).
Engelman JA et al. Nat Rev Genet 7:606-619 (2006).
Ferguson et al. Mol Cell 6(2):373-384 (2000).
Fleming et al. Biochem J 351:173-182 (2000).
Furet et al. Bioorg Med Chem Lett 23(13):3741-3748 (2013)
Generoso et al. Nat. Chem. Biol 11:280-286 (2015)
Gerspacher et al., Bioorg Med Chem Lett 25: 3582-3584 (2015)
Gkeka P et al., PLoS Comput Biol., 10:e1003895 (2014).
Gkeka P et al., J Phys Chem B. 119:1002-1016 (2015).
Gray A et al. Anal Biochem 313(2):234-245 (2003)
He J et al. J Lipid Res 49(8):1807-1815 (2008).
Hon et al. Oncogene. 31:3655-3666 (2012).
Hu et al., Clin Cancer Res 11:8208-8212 (2005)
Huang CH et al. Science 318:1744-1748 (2007).
Klinakis A et al. Proc Natl Acad Sci U S A 106:2359-2364 (2009)
Knight et al. Cell 125:733-747 (2006).
Lai CL et al. J Mol Biol 425(17):3073-3090 (2013)
Lee et al., Cell, 157:1393-1404 (2014)
Lietzke SE et al Mol Cell 6(2):385-394 (2000).
Limin H et al. Clin Cancer Res 11:8208-8212 (2005).
Lindsay Y et al. J Cell Sci 119:5160-5168 (2006).
Lingaraj T et al. J Biomol Screen 13(9):906-911 (2008).
Lionta E et al. Curr Top Med Chem 14:1923-1938 (2014).
Liu P et al. Nat Rev Drug Discov, 8:627-644 (2009)
Mandelker D et al. Proc Natl Acad Sci U S A 106:16996-7001 (2009).
Massacesi C et al. Onco Targets Ther 9:203-210 (2016).
Miled N et al. Science 317:239-242 (2007).
Miller TW et al. Breast Cancer Res 13:224 (2011)
Milne et al. Nature, 450:712-716 (2007)
Mukund et al. J Biol Chem 288:36168-36178 (2013)
Parsons R. Clin Cancer Res 11:7965-7966 (2005).
Politi K et al. Oncogene 23:1558-1565 (2004)
Rizk et al, Cell Commun Signal 13:1-5 (2015)
Samuels Y and Waldmann T Curr Top Microbiol Immunol. 347:21-41 (2010).
Samuels Y et al. Cancer Cell 7:561-573 (2005)
Schoepfer et al. J Med Chem 61:8120-8135 (2018)
Stratikopoulos et al. Cancer Cell 27:837-851 (2015)
Thorpe et al., Nat Rev Cancer 15(1):7-24 (2015)
Wylie et al. Nature, 543:733-737 (2017)
Zheng et al. J Am Chem Soc 140:5914-5924 (2018)

## Claims

1. A compound of formula (I) 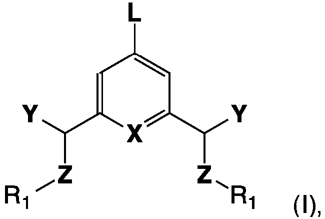
a tautomer, polymorph, hydrate, solvate, or a pharmaceutically acceptable salt thereof, wherein
L is independently H or OH;
X is independently N or CH;
Y is independently H or =O;
Z is independently S or NH;
R1 is independently mono- or polycyclic aryl or heteroaryl selected from: 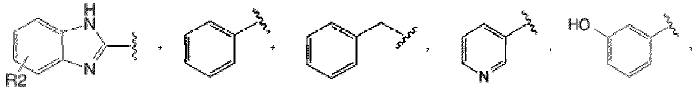 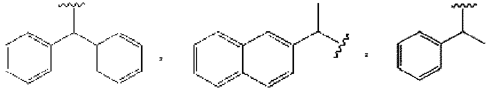 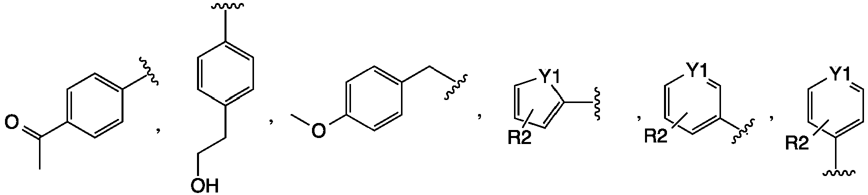
R₂ is independently at least one of hydroxyl, hydrogen, fluoro, chloro, or bromo; and
in the five-membered rings, Y₁ is independently CH₂, O, S, or NH, whereas in the six-membered rings, Y₁ is independently CH, or N,
for use in treating a cancerous disease caused by or triggered by a PI3Kα in a mammal, wherein the mammal to be treated carries a mutation in the gene encoding PI3Kα which leads to the occurrence of a PI3Kα mutant, and wherein the mammal is preferably a human.

2. The compound for use according to claim 1, (I), wherein
L is H;
X is N;
Y is H;
Z is S;
R₁ is selected from any of the following substituted or unsubstituted phenyl, pyridine, or pyrimidine, or one of the fragments below: 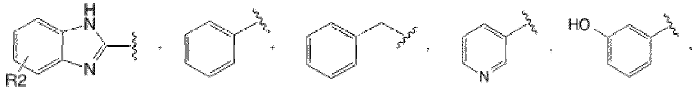 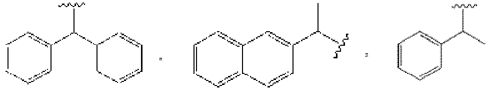 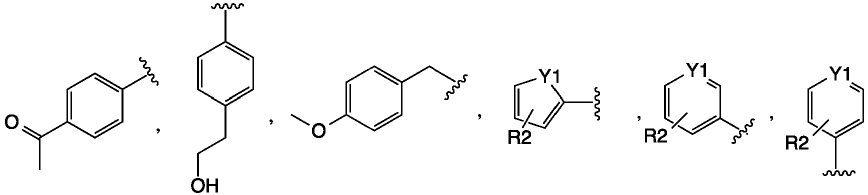
R2 is independently at least one of hydroxyl, hydrogen, fluoro, chloro, or bromo; and
in the five-membered rings, Y1 is independently CH₂, O, S, or NH, whereas in the six-membered rings, Y1 is independently CH, or N.

3. The compound for use according to claim 1, wherein
L is H;
X is N;
Y is carbonyl;
Z is NH;
R₁ is selected from any of the following substituted or unsubstituted phenyl, pyridine, or pyrimidine, or one of the fragments below: 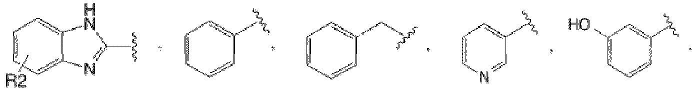 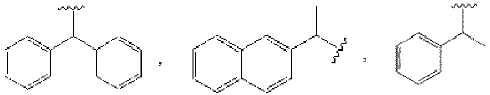 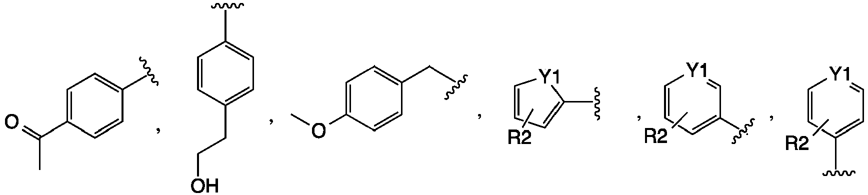
R₂ is independently at least one of hydroxyl, hydrogen, fluoro, chloro, or bromo;
in the five-membered rings, Y₁ is independently CH₂, O, S, or NH, whereas in the six-membered rings, Y₁ is independently CH, or N.

4. The compound for use according to claim 1, wherein
L is H;
X is N;
Y is independently H or carbonyl;
Z is independently S or NH;
R₁ is selected from following fragments: 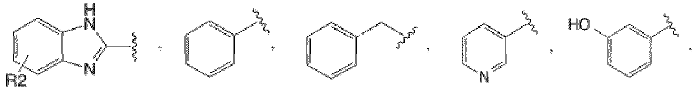 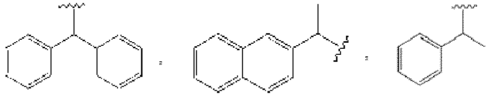 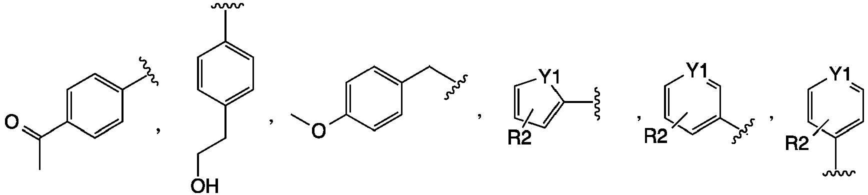
R₂ is independently at least one of hydroxyl, hydrogen, fluoro, chloro, or bromo; and
in the five-membered rings, Y₁ is independently CH₂, O, S, or NH, whereas in the six-membered rings, Y₁ is independently CH, or N.

5. The compound for use according to any one of claims 1-4, having the formula of either: 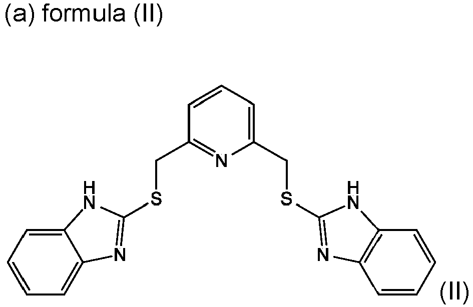
or a pharmaceutically acceptable salt thereof; 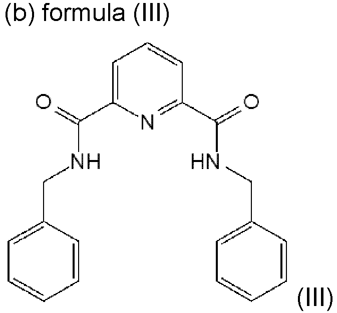
or a pharmaceutically acceptable salt thereof; or 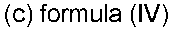 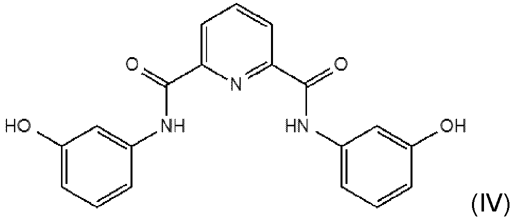
or a pharmaceutically acceptable salt thereof.

6. The compound of Formula II 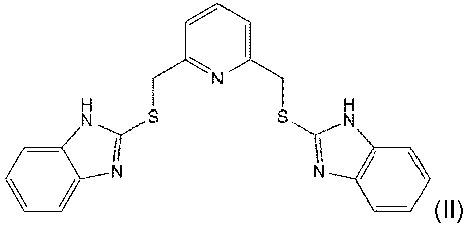
or the compound of Formula III 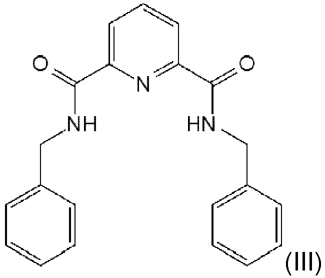
for use in the treatment of cancer.

7. The compound for use according to anyone of claims 1 to 6, for use in combination with one or more additional therapeutic agents, preferably wherein the one or more additional therapeutic agents are selected from the group consisting of:
10-Hydroxycamptothecin, 17-Allylamino-geldanamycin, 2-Methoxyanti-mycin A3, 3,4-Dichloroisocoumarin, 4-Hydroxyphenylretinamide, 9-cis Retinoic acid, Abiraterone, Ado-Trastuzumab Emtansine, Adriamycin, Afatinib, N-(3-chlorophenyl)-6,7-dimethoxyquinazolin-4-amine, 2-Amino-4-(1H-indol-5-yl)-1,1,3-tricyanobuta-1,3-diene, Aldesleukin, Alemtuzumab, Amifostine, Anastrozole, Anisomycin, Aphidicolin, Arsenic Trioxide, Asparaginase Erwinia chrysanthemi, Avastin, Axitinib, N-[2(S)-(2(R)-2-Amino-3-mercaptopropylamino)-3-methylbutyl]-Lphenylalanyl-L-methionine trifluoroacetate salt, Bacillus Calmette-Guerin, bisphenol A diglycidyl ether, Bendamustine, Beta-lapachone , Betulinic acid, Bevacizumab, Bexarotene, Bicalutamide, BisBenzimide, Bleomycin, Bortezomib, Bosutinib, Buserelin, Busulfan, Cabazitaxel, Cabozantinib-S-Malate, Calpeptin, Camptothecin, Caffeic acid phenethyl ester, Capecitabine, CAprelsa (Vandetanib), Carboplatin, Carboplatin, Carfilzomib, Carmustine, Cetuximab, Chlorambucil, Ciglitazone, Cisplatin, Clodronate, Clofarabine, Cometriq, Crizotinib, Curcumin, Cyclo [Arg-Gly-Asp-D-Phe-Val], Cycloheximide, Cyclopamine, Cyclophosphamide, Cyclosporin A, Cyproterone, Cytarabine, D12-Prostaglandin J2, Dabrafenib, Dacarbazine, Dactinomycin, Dasatinib, Daunorubicin, Degarelix, Denosumab, Dexamethasone, Docetaxel, Doxorubicin, Ebselen, Ellipticine, Enzalutamide, Epirubicin, Erlotinib, Etoposide, Everolimus, Exemestane, Fludarabine, Fluorouracil, Flutamide, Folinic acid, Fulvestrant, Gefitinib, Geldanamycin, Gemcitabine, Genistein, Gingerol, Gliadel Wafer, Gliotoxin, Goserelin, 2-Chloro-5-nitrobenzanilide, 2-Amino-6-bromo-a-cyano-3-(ethoxycarbonyl)-4H-1-benzopyran-4-acetic acid ethyl ester, Hinokitiol, Hydroxyurea, Sobuzoxane, Idarubicin, ifosfamide, Imatinib, Indomethacin, Ipilimumab, Irinotecan, Ixabepilone, Lanreotide, Lapatinib, Lenalidomide, Letrozole, Lenvatinib Mesylate, Lenvima, Leucovorin, Leuprolide, Lomustin, Medroxyprogesterone, Megestrol, Melphalan, Mepesuccinate, Mercaptopurine, Mesna, Methotrexate, Methoxy verapamil, carbobenzoxy-L-leucyl-L-leucyl-L-leucinal, Mitomycin C, Mitoxantrone, N,N-Dimethylsphingosine, Nelarabine, Nilotinib, Nivolumab, Octreotide, Ofatumumab, Oligomycin A, Omacetaxine, Oxaliplatin, Paclitaxel, Pamidronate, Panitumumab, Pazopanib, Pegaspargase, Pemetrexed, Pembrolizumab, Pertuzumab, Pifithrin, plerixafor, Podophyllotoxin, Pomalidomide, Ponatinib, Prednisone, 2,2-Bis(hydroxymethyl)-1-azabicyclo[2.2.2]octan-3-one, Procarbazine, Radium 223 Dichloride, Raltitrexed, Rapamycin, Recombinant Human Papillomavirus (HPV) Bivalent Vaccine, Recombinant HPV Quadravalent Vaccine, Recombinant HPV Bivalent , Vaccine, Recombinant HPV Quadravalent Vaccine, Recombinant Interferon Alfa-2b, Regorafenib, Resveratrol, all trans Retinoic acid, ,Rheumatrex, Rituximab, Rolipram, Roscovitine, Rottlerin, Shikonin, Sipuleucel-T, Sirolimus, Sorafenib, Sphingosine, Splitomycin, Staurosporine, Stilboestrol, Streptozocin, 3-(4-Dimethylaminobenzylidenyl)-2-indolinone, 3-[[(4-Dimethyl-amino)phenyl] methylene]-1,3-dihydro-2H-indol-2-one, Sulindac sulphide, Sunitinib, Tamoxifen, Temozolomide, Temsirolimus, Thalidomide, Topotecan, Toremifene, Trametinib, Trastuzumab, Trichostatin-A, Trifluoperazine, 4-[(E)-2-(5,6,7,8-Tetrahydro-5,5,8,8-tetramethyl-2-naphthalenyl)-1-propenyl]benzoic acid, 3,4-Dihydroxy-a-cyanothiocinnam-amide, (3-Hydroxy-4-nitrobenzylidene)malononitrile, Vandertanib, Valproic acid, Vemurafenib, Verapamil, Vinblastine, Vincristine, Vinorelbine, Wortmannin, 4-Chloro-6-(2,3-xylidino)-2-pyrimidinylthioacetic acid, Ziv-Aflibercept, Zoledronic Acid, salts thereof, and combinations thereof,
preferably selected from the group consisting of: Abiraterone, Ado-Trastuzumab Emtansine, Afatinib, Afinitor (Everolimus), Anastrozole, Avastin, Bevacizumab, Cabazitaxel, Capecitabine, Carboplatin, Carmustin, Cisplatin, Crizotinib, Cyclophosphamide, Degarelix, Denosumab, Docetaxel, Doxorubicin, Enzalutamide, Epirubicin, Erlotinib, Etoposide, Everolimus, Exemestane, Fluorouracil, Fulvestrant, Gefitinib, Gemcitabine, Ixabepilone, Lapatinib, Letrozole, Leuprolide, Lomustine, Megestrol, Methotrexate, Mitomycin C, Paclitaxel, Pamidronate, Pemetrexed, Pertuzumab, Prednisone, Radium 223 Dichloride, Sipuleucel-T, Sunitinib, Tamoxifen, Temodar, Temozolomide, Topotecan, Toremifene, Trastuzumab, Cabozantinib-S-Malate, Caprelsa (Vandetanib), Cometriq (Cabozantinib-S-Malate), Doxorubicin Hydrochloride, Ipilimumab, Lenvatinib Mesylate, Nexavar (Sorafenib Tosylate), Nivolumab, Vandetanib, Yervoy (Ipilimumab), salts thereof, and any combination thereof.

8. The compounds for use according to claim 7, further comprising one or more additional therapeutic agents, which are selected from the group consisting of:
Acetylsalicylic acid, Diflunisal, Salsalate, Ibuprofen, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Nabumetone, Piroxicam, Meloxicam, Tenoxicam, Droxicam, Lornoxicam, Isoxicam, Mefenamic acid, Meclofenamic acid, Flufenamic acid, Tolfenamic acid, Celecoxib, Rofecoxib, Valdecosib, Parecoxib, Lumiracoxib, Etoricoxib, Firocoxib, Nimesulide, Licofelone, Hharpagide, Lysine clonixinate, pharmaceutically acceptable salts thereof, and combinations thereof, preferably selected from the group consisting of Celecoxib, Rofecoxib, Valdecosib, Parecoxib, Lumiracoxib, Etoricoxib, Firocoxib, pharmaceutically acceptable salts thereof, and combinations thereof.

9. The compound for the use of any one of claims 1-8, wherein the cancerous disease is selected from the group consisting of: acute monocytic leukemia, acute myelogenous leukemia, acute myelomonocytic leukemia, acute promyelocytic leukemia, adult T-cell leukemia, adult T-cell lymphoma, astrocytoma, atypical carcinoid lung cancer, basal cell carcinoma, B-acute lymphocytic leukemia, B-cell acute lymphoblastic leukemia/lymphoma, Bladder cancer, brain cancer, breast cancer, bronchial cancer, Burkitt's lymphoma, cancer of the bile duct, cancer of unknown primary origin, cervix cancer, chronic myeloproliferative disorder, colon cancer, diffuse large cell lymphoma, endometrial cancer, ependymoma, esophageal cancer, gastric cancer, glioma, glioblastoma, head and neck cancer, hemagiopericytoma, hepatocellular carcinoma, Hodgkin's lymphoma, Kaposi's sarcoma, kidney cancer, large cell neuroendocrine carcinoma, large granular lymphocytic leukemia, leukemia, liver cancer, lung cancer, lymphoma, medulloblastoma, melanoma, Multiple myeloma, myelodysplastic syndrome, nasopharygeal cancer, neuroblastoma, NK cell tumor, non-Hodgkin's lymphoma, oesophageal squamous cell carcinoma, osteosarcoma, ovarian cancer, pancreatic cancer, peripheral T-cell leukemia, primary plasma cell leukemia, prostate cancer, renal clear cell carcinoma, retinoblastoma, rhabdomyosarcoma, sarcoma, small cell lung carcinoma, T-cell acute lymphoblastic leukemia, T-cell acute lymphoblastic lymphoma, testicular cancer, thymoma, thyroid cancer, urachal cancer, uterine cancer, vaginal cancer, and combinations thereof,
preferably selected from the group consisting of B-acute lymphocytic leukemia, Burkitt's lymphoma, diffuse large cell lymphoma, multiple myeloma, primary plasma cell leukemia, atypical carcinoid lung cancer, bladder cancer, brain cancer, breast cancer, cervix cancer, colon cancer, endometrial cancer, gastric cancer, glioblastoma, head and neck cancer, hepatocellular carcinoma, large cell neuroendocrine carcinoma, liver cancer, medulloblastoma, melanoma, neuroblastoma, oesophageal squamous cell carcinoma, osteosarcoma, ovarian cancer, pituitary cancer, prostate cancer, renal clear cell carcinoma, retinoblastoma, rhabdomyosarcoma, small cell lung carcinoma, colon adenocarcinoma, lung adenocarcinoma, prostatic adenocarcinoma, urachal adenocarcinoma, vaginal adenocarcinoma, mammary adenocarcinoma, esophageal adenocarcinoma, bronchial adenocarcinoma, pancreatic adenocarcinoma, gastrointestinal adenocarcinoma, endometrial adenocarcinoma, ovarian adenocarcinoma, gastric adenocarcinoma, liver adenocarcinoma, thyroid adenocarcinoma, head and neck adenocarcinoma, bladder adenocarcinoma, and gastrointestinal adenocarcinoma; and combinations thereof, preferably wherein the cancerous disease is pancreatic ductal adenocarcinoma, mammary adenocarcinoma, prostatic adenocarcinoma, or lung adenocarcinoma, endometrial adenocarcinoma, ovarian adenocarcinoma, gastric adenocarcinoma, liver adenocarcinoma, thyroid adenocarcinoma, head and neck adenocarcinoma, bladder adenocarcinoma, or gastrointestinal adenocarcinoma and combinations thereof.

## Patentansprüche

1. Verbindung der Formel (I) 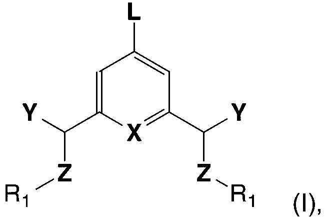
Tautomer, Polymorph, Hydrat, Solvat oder pharmazeutisch annehmbares Salz davon, wobei
L unabhängig H oder OH ist;
X unabhängig N oder CH ist;
Y unabhängig H oder =O ist;
Z unabhängig S oder NH ist;
R1 unabhängig mono- oder polyzyklisches Aryl oder Heteroaryl ist, ausgewählt aus: 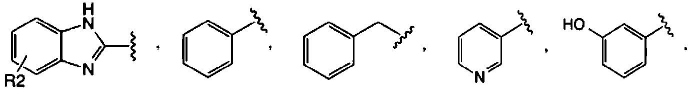 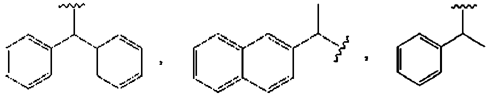 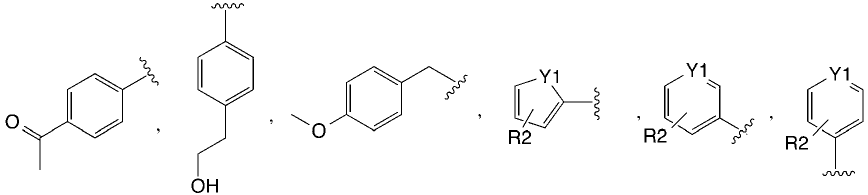
R₂ unabhängig mindestens eines von Hydroxyl, Wasserstoff, Fluor, Chlor oder Brom ist, und
in den fünf-gliedrigen Ringen Y₁ unabhängig CH₂, O, S oder NH ist, wohingegen in den sechs-gliedrigen Ringen Y₁ unabhängig CH oder N ist,
zur Verwendung bei der Behandlung einer durch PI3Kα verursachten oder ausgelösten Krebserkrankung bei einem Säuger, wobei der zu behandelnde Säuger eine Mutation in dem für PI3Kα kodierenden Gen aufweist, die zum Auftreten einer PI3Kα-Mutante führt, und wobei der Säuger vorzugsweise ein Mensch ist.

2. Verbindung zur Verwendung nach Anspruch 1, (I), wobei
L H ist;
X N ist;
Y H ist;
Z S ist;
R₁ aus beliebigen der folgenden substituierten oder unsubstituierten Phenyl-, Pyridin- oder Pyrimidin-Gruppen oder einem der folgenden Fragmente ausgewählt ist: 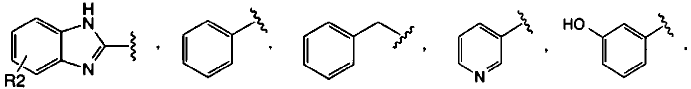 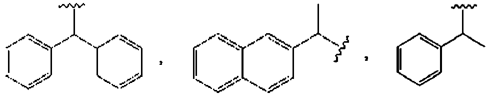 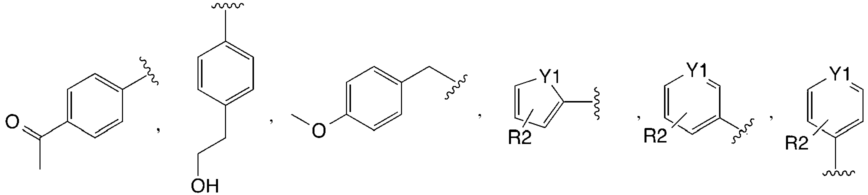
R2 unabhängig mindestens eines von Hydroxyl, Wasserstoff, Fluor, Chlor oder Brom ist; und
in den fünf-gliedrigen Ringen Y₁ unabhängig CH₂, O, S oder NH ist, wohingegen in den sechs-gliedrigen Ringen Y₁ unabhängig CH oder N ist.

3. Verbindung zur Verwendung nach Anspruch 1, wobei
L H ist;
X N ist;
Y Carbonyl ist;
Z NH ist;
R₁ aus beliebigen der folgenden substituierten oder unsubstituierten Phenyl-, Pyridin- oder Pyrimidin-Gruppen oder einem der folgenden Fragmente ausgewählt ist: 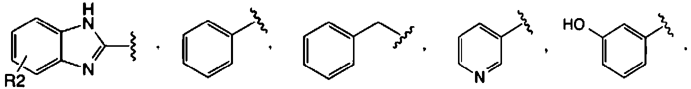 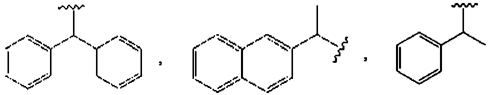 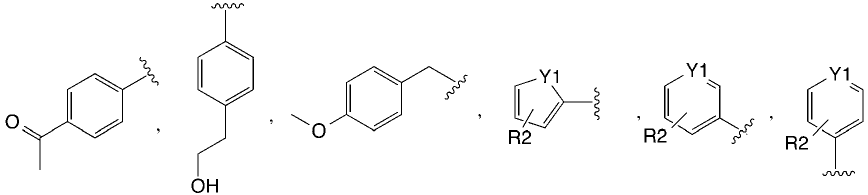
R₂ unabhängig mindestens eines von Hydroxyl, Wasserstoff, Fluor, Chlor oder Brom ist;
in den fünf-gliedrigen Ringen Y₁ unabhängig CH₂, O, S oder NH ist, wohingegen in den sechs-gliedrigen Ringen Y₁ unabhängig CH oder N ist.

4. Verbindung zur Verwendung nach Anspruch 1, wobei
L H ist;
X N ist;
Y unabhängig H oder Carbonyl ist;
Z unabhängig S oder NH ist;
R₁ aus den folgenden Fragmenten ausgewählt ist: 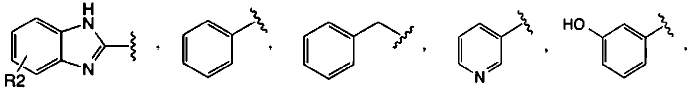 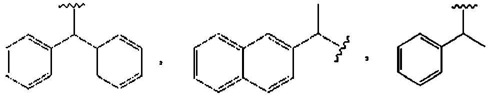 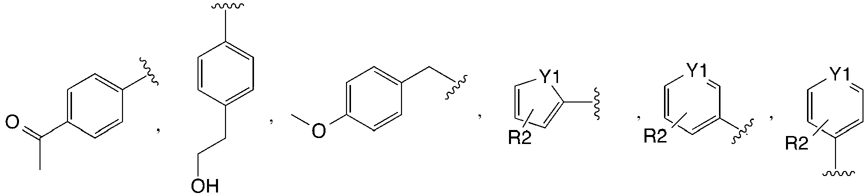
R₂ unabhängig mindestens eines von Hydroxyl, Wasserstoff, Fluor, Chlor oder Brom ist;
in den fünf-gliedrigen Ringen Y₁ unabhängig CH₂, O, S oder NH ist, wohingegen in den sechs-gliedrigen Ringen Y₁ unabhängig CH oder N ist.

5. Verbindung zur Verwendung nach einem der Ansprüche 1-4, welche eine der folgenden Formeln aufweist: 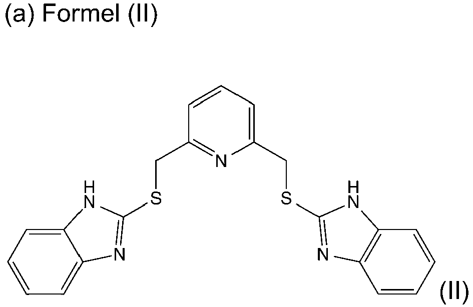
oder ein pharmazeutisch annehmbares Salz davon; 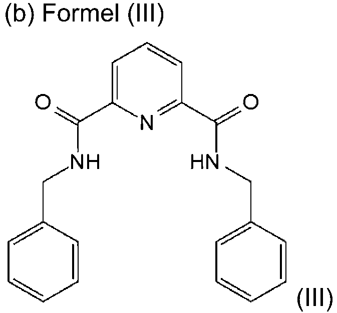
oder ein pharmazeutisch annehmbares Salz davon, oder 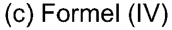 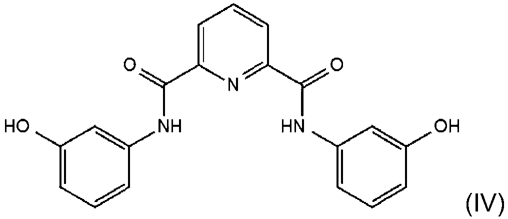
oder ein pharmazeutisch annehmbares Salz davon.

6. Verbindung der Formel II 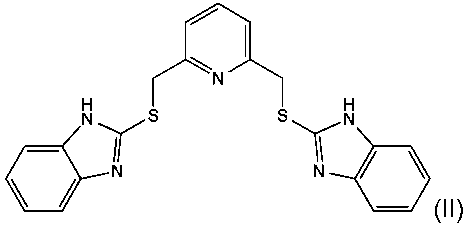
oder Verbindung der Formel III 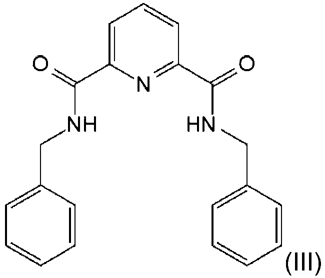
zur Verwendung bei der Behandlung von Krebs.

7. Verbindung zur Verwendung nach einem der Ansprüche 1 bis 6, zur Verwendung in Kombination mit einem oder mehreren zusätzlichen therapeutischen Wirkstoffen, wobei die ein oder mehreren zusätzlichen therapeutischen Wirkstoffe vorzugsweise aus der Gruppe ausgewählt sind, bestehend aus:
10-Hydroxycamptothecin, 17-Allylamino-geldanamycin, 2-Methoxyantimycin A3, 3,4-Dichlorisocoumarin, 4-Hydroxyphenylretinamid, 9-cis Retinsäure, Abirateron, Ado-Trastuzumab Emtansin, Adriamycin, Afatinib, N-(3-Chlorphenyl)-6,7-dimethoxyquinazolin-4-amin, 2-Amino-4-(1H-indol-5-yl)-1,1,3-tricyanobuta-1,3-dien, Aldesleukin, Alemtuzumab, Amifostin, Anastrozol, Anisomycin, Aphidicolin, Arsentrioxid, Asparaginase Erwinia chrysanthemi, Avastin, Axitinib, N-[2(S)-(2(R)-2-Amino-3-mercaptopropylamino)-3-methylbutyl]-Lphenylalanyl-L-methionin-Trifluoracetat-Salz, Bacillus Calmette-Guerin, Bisphenol A diglycidylether, Bendamustin, Beta-Lapachon , Betulinsäure, Bevacizumab, Bexaroten, Bicalutamid, Bis-Benzimid, Bleomycin, Bortezomib, Bosutinib, Buserelin, Busulfan, Cabazitaxel, Cabozantinib-S-Malat, Calpeptin, Camptothecin, Kaffeesäurephenethylester, Capecitabin, CAprelsa (Vandetanib), Carboplatin, Carboplatin, Carfilzomib, Carmustin, Cetuximab, Chlorambucil, Ciglitazon, Cisplatin, Clodronat, Clofarabin, Cometriq, Crizotinib, Curcumin, Cyclo [Arg-Gly-Asp-D-Phe-Val], Cycloheximid, Cyclopamin, Cyclophosphamid, Cyclosporin A, Cyproteron, Cytarabin, D12-Prostaglandin J2, Dabrafenib, Dacarbazin, Dactinomycin, Dasatinib, Daunorubicin, Degarelix, Denosumab, Dexamethason, Docetaxel, Doxorubicin, Ebselen, Ellipticin, Enzalutamid, Epirubicin, Erlotinib, Etoposid, Everolimus, Exemestan, Fludarabin, Fluoruracil, Flutamid, Folinsäure, Fulvestrant, Gefitinib, Geldanamycin, Gemcitabin, Genistein, Gingerol, Gliadel-Wafer, Gliotoxin, Goserelin, 2-Chlor-5-nitrobenzanilid, 2-Amino-6-brom-α-cyano-3-(ethoxycarbonyl)-4H-1-benzopyran-4-essigsäureethylester, Hinokitiol, Hydroxyharnstoff, Sobuzoxan, Idarubicin, Ifosfamid, Imatinib, Indomethacin, Ipilimumab, Irinotecan, Ixabepilon, Lanreotid, Lapatinib, Lenalidomid, Letrozol, Lenvatinib Mesylat, Lenvima, Leucovorin, Leuprolid, Lomustin, Medroxyprogesteron, Megestrol, Melphalan, Mepesuccinat, Mercaptopurin, Mesna, Methotrexat, Methoxy verapamil, Carbobenzoxy-L-leucyl-L-leucyl-L-leucinal, Mitomycin C,
Mitoxantron, N,N-Dimethylsphingosin, Nelarabin, Nilotinib, Nivolumab, Octreotid, Ofatumumab, Oligomycin A, Omacetaxin, Oxaliplatin, Paclitaxel, Pamidronate, Panitumumab, Pazopanib, Pegaspargase, Pemetrexed, Pembrolizumab, Pertuzumab, Pifithrin, Plerixafor, Podophyllotoxin, Pomalidomid, Ponatinib, Prednison, 2,2-Bis(hydroxymethyl)-1-azabicyclo[2.2.2]octan-3-on, Procarbazin, Radium 223 Dichlorid, Raltitrexed, Rapamycin, rekombinantes bivalentes Vakzin gegen Humanes Papillomavirus (HPV), rekombinantes quadrivalentes Vakzin gegen HPV, rekombinantes bivalentes Vakzin gegen HPV, rekombinantes quadrivalentes Vakzin gegen HPV, rekombinantes Interferon Alfa-2b, Regorafenib, Resveratrol, all trans Retinsäure, Rheumatrex, Rituximab, Rolipram, Roscovitin, Rottlerin, Shikonin, Sipuleucel-T, Sirolimus, Sorafenib, Sphingosin, Splitomycin, Staurosporin, Stilboestrol, Streptozocin, 3-(4-Dimethylaminobenzylidenyl)-2-indolinon, 3-[[(4-Dimethyl-amino)phenyl] methylen]-1,3-dihydro-2H-indol-2-on, Sulindac-Sulfid, Sunitinib, Tamoxifen, Temozolomid, Temsirolimus, Thalidomid, Topotecan, Toremifen, Trametinib, Trastuzumab, Trichostatin-A, Trifluoperazin, 4-[(E)-2-(5,6,7,8-Tetrahydro-5,5,8,8-tetramethyl-2-naphthalenyl)-1-propenyl]benzoesäure, 3,4-Dihydroxy-α-cyanothiocinnamamid, (3-Hydroxy-4-nitrobenzyliden)malononitril, Vandertanib, Valproinsäure, Vemurafenib, Verapamil, Vinblastin, Vincristin, Vinorelbin, Wortmannin, 4-Chlor-6-(2,3-xylidin)-2-pyrimidinylthioessigsäure, Ziv-Aflibercept, Zoledronsäure, deren Salzen und deren Kombinationen,
vorzugsweise ausgewählt aus der Gruppe bestehend aus:
Abirateron, Ado-Trastuzumab Emtansin, Afatinib, Afinitor (Everolimus), Anastrozol, Avastin, Bevacizumab, Cabazitaxel, Capecitabin, Carboplatin, Carmustin, Cisplatin, Crizotinib, Cyclophosphamid, Degarelix, Denosumab, Docetaxel, Doxorubicin, Enzalutamid, Epirubicin, Erlotinib, Etoposid, Everolimus, Exemestan, Fluoruracil, Fulvestrant, Gefitinib, Gemcitabin, Ixabepilon, Lapatinib, Letrozol, Leuprolid, Lomustin, Megestrol, Methotrexat, Mitomycin C, Paclitaxel, Pamidronat, Pemetrexed, Pertuzumab, Prednison, Radium-223-Dichlorid, Sipuleucel-**T,** Sunitinib, Tamoxifen, Temodar, Temozolomid, Topotecan, Toremifen, Trastuzumab, Cabozantinib-S-Malat, Caprelsa (Vandetanib), Cometriq (Cabozantinib-S-Malat), Doxorubicin-Hydrochlorid, Ipilimumab, Lenvatinib-Mesylat, Nexavar (Sorafenib-Tosylate), Nivolumab, Vandetanib, Yervoy (Ipilimumab deren Salzen und beliebigen Kombinationen davon.

8. Verbindungen zur Verwendung nach 7, ferner umfassend ein oder mehrere zusätzliche therapeutische Wirkstoffe, die aus der Gruppe ausgewählt sind, bestehend aus:
Acetylsalicylsäure, Diflunisal, Salsalat, Ibuprofen, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Nabumeton, Piroxicam, Meloxicam, Tenoxicam, Droxicam, Lornoxicam, Isoxicam, Mefenaminsäure, Meclofenaminsäure, Flufenaminsäure, Tolfenaminsäure, Celecoxib, Rofecoxib, Valdecosib, Parecoxib, Lumiracoxib, Etoricoxib, Firocoxib, Nimesulid, Licofelon, Hharpagid, Lysin-Clonixinat, pharmazeutisch annehmbaren Salzen davon und Kombinationen davon,
vorzugsweise ausgewählt aus der Gruppe bestehend aus Celecoxib, Rofecoxib, Valdecosib, Parecoxib, Lumiracoxib, Etoricoxib, Firocoxib, pharmazeutisch annehmbaren Salzen davon und Kombinationen davon.

9. Verbindung zur Verwendung nach einem der Ansprüche 1-8, wobei die Krebserkrankung aus der Gruppe ausgewählt ist, bestehend aus: akuter monozytärer Leukämie, akuter myeloischer Leukämie, akuter myelomonozytärer Leukämie, akuter promyelozytärer Leukämie, T-Zell-Leukämie bei Erwachsenen, T-Zell-Lymphom bei Erwachsenen, Astrozytom, atypischem Karzinoid der Lunge, Basalzellkarzinom, akuter B-Zell-Lymphoblastenleukämie, akuter B-Zell-Lymphoblastenleukämie/- Lymphom, Blasenkrebs, Hirntumor, Brustkrebs, Bronchialkarzinom, Burkitt-Lymphom, Gallengangskarzinom, Karzinom unbekannten Primärursprungs, Gebärmutterhalskrebs, chronischer myeloproliferativer Erkrankung, Dickdarmkrebs, diffusem großzelligem Lymphom, Gebärmutterschleimhautkrebs, Ependymom, Speiseröhrenkrebs, Magenkrebs, Gliom, Glioblastom, Kopf-Hals-Krebs, Hämangioperizytom, Leberzellkarzinom, Hodgkin-Lymphom, Kaposi-Sarkom, Nierenkrebs, großzelligem neuroendokrinem Karzinom, großgranulärer lymphozytärer Leukämie, Leukämie, Leberkrebs, Lungenkrebs, Lymphom, Medulloblastom, Melanom, Multiplem Myelom, myelodysplastischem Syndrom, Nasopharynxkarzinom, Neuroblastom, NK-Zelltumor, Non-Hodgkin-Lymphom, Plattenepithelkarzinom der Speiseröhre, Osteosarkom, Eierstockkrebs, Bauchspeicheldrüsenkrebs, peripherer T-Zell-Leukämie, primärer Plasmazell-Leukämie, Prostatakrebs, klarzelligem Nierenzellkarzinom, Retinoblastom, Rhabdomyosarkom, Sarkom, kleinzelligem Lungenkarzinom, akuter T-Zell-Lymphoblastenleukämie, akutem T-Zell-Lymphoblastenlymphom, Hodenkrebs, Thymom, Schilddrüsenkrebs, Urachuskarzinom, Gebärmutterkrebs, Vaginalkrebs und Kombinationen davon,
vorzugsweise ausgewählt aus der Gruppe bestehend aus akuter B-Lymphoblastenleukämie, Burkitt-Lymphom, diffusem großzelligem Lymphom, Multiplem Myelom, primärer Plasmazell-Leukämie, atypischem Karzinoid-Lungenkarzinom, Blasenkrebs, Hirntumor, Brustkrebs, Gebärmutterhalskrebs, Dickdarmkrebs, Gebärmutterschleimhautkrebs, Magenkrebs, Glioblastom, Kopf-Hals-Krebs, Leberzellkarzinom, großzelliges neuroendokrinem Karzinom, Leberkrebs, Medulloblastom, Melanom, Neuroblastom, Ösophagus-Plattenepithelkarzinom, Osteosarkom, Eierstockkrebs, Hypophysentumor, Prostatakrebs, klarzelligem Nierenzellkarzinom, Retinoblastom, Rhabdomyosarkom, kleinzelligem Lungenkarzinom, Kolon-Adenokarzinom, Lungen-Adenokarzinom, Prostata-Adenokarzinom, Urachus-Adenokarzinom, Vaginal-Adenokarzinom, Brust-Adenokarzinom, Ösophagus-Adenokarzinom, Bronchial-Adenokarzinom, Pankreas-Adenokarzinom, Magen-Darm-Adenokarzinom, Endometrium-Adenokarzinom, Ovarial-Adenokarzinom, Magen-Adenokarzinom, Leber-Adenokarzinom, Schilddrüsen-Adenokarzinom, Kopf-Hals-Adenokarzinom, Blasen-Adenokarzinom und Magen-Darm-Adenokarzinom; sowie Kombinationen davon,
wobei es sich bei der Krebserkrankung vorzugsweise um duktales Pankreas-Adenokarzinom, Brust-Adenokarzinom, Prostata-Adenokarzinom oder Lungen-Adenokarzinom, Endometrium-Adenokarzinom, Ovarial-Adenokarzinom, Magen-Adenokarzinom, Leber-Adenokarzinom, Schilddrüsen-Adenokarzinom, Kopf-Hals-Adenokarzinom, Blasen-Adenokarzinom oder Magen-Darm-Adenokarzinom sowie Kombinationen davon handelt.

## Revendications

1. Composé de formule (I) 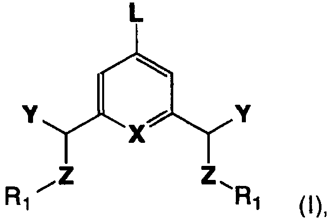
tautomère, polymorphe, hydrate, solvate ou sel pharmaceutiquement acceptable de celui-ci, dans lequel
L est indépendamment H ou OH;
X est indépendamment N ou CH;
Y est indépendamment H ou =O;
Z est indépendamment S ou NH;
R1 est indépendamment un groupe aryle ou hétéroaryle mono- ou polycyclique choisi parmi: 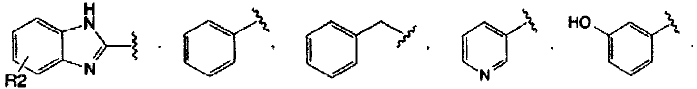 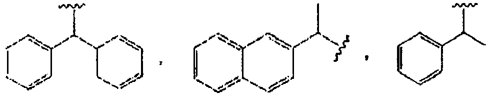 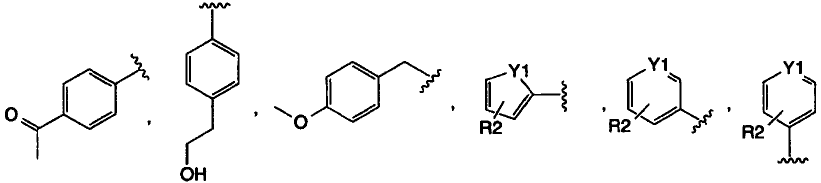
R₂ est indépendamment au moins un groupe parmi hydroxyle, hydrogène, fluoro, chloro ou bromo; et
dans les cycles à cinq chaînons, Y₁ est indépendamment CH₂, O, S ou NH,
tandis que dans les cycles à six chaînons, Y₁ est indépendamment CH ou N, destiné à être utilisé dans le traitement d'une maladie cancéreuse causée ou déclenchée par une PI3Kα chez un mammifère, dans lequel le mammifère destiné à être traité porte une mutation du gène codant la PI3Kα qui conduit à l'apparition d'un mutant de la PI3Kα, et dans lequel le mammifère est de préférence un humain.

2. Composé destiné à être utilisé selon la revendication 1, (I), dans lequel
L est H;
X est N;
Y est H;
Z est S;
R₁ est choisi parmi l'un quelconque des groupes phényle, pyridine ou pyrimidine substitués ou non substitués suivants, ou l'un des fragments ci-dessous: 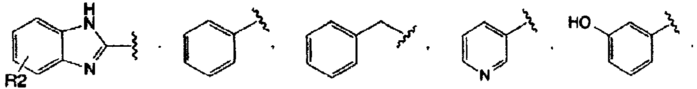 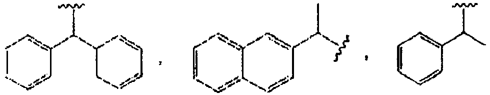 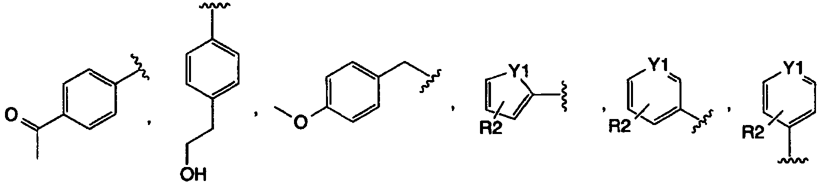
R2 est indépendamment au moins un groupe parmi hydroxyle, hydrogène, fluoro, chloro ou bromo; et
dans les cycles à cinq chaînons, Y1 est indépendamment CH₂, O, S ou NH, tandis que dans les cycles à six chaînons, Y1 est indépendamment CH ou N.

3. Composé destiné à être utilisé selon la revendication 1, dans lequel
L est H;
X est N;
Y est carbonyle;
Z est NH;
R₁ est choisi parmi l'un quelconque des groupes phényle, pyridine ou pyrimidine substitués ou non substitués suivants, ou l'un des fragments ci-dessous: 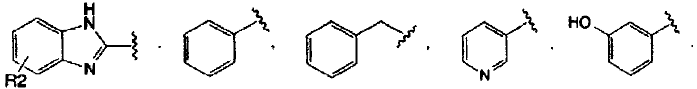 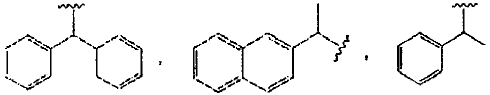 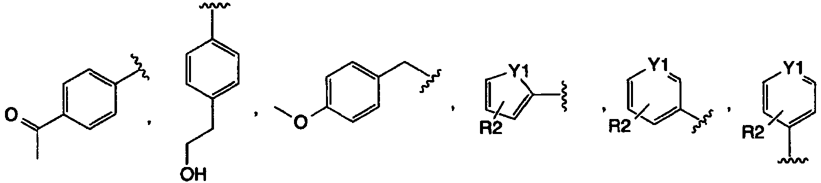
R₂ est indépendamment au moins un groupe parmi hydroxyle, hydrogène, fluoro, chloro ou bromo;
dans les cycles à cinq chaînons, Y₁ est indépendamment CH₂, O, S ou NH, tandis que dans les cycles à six chaînons, Y₁ est indépendamment CH ou N.

4. Composé destiné à être utilisé selon la revendication 1, dans lequel
L est H;
X est N;
Y est indépendamment H ou carbonyle;
Z est indépendamment S ou NH;
R₁ est choisi parmi les fragments suivants: 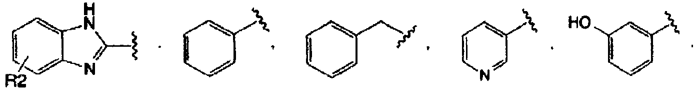 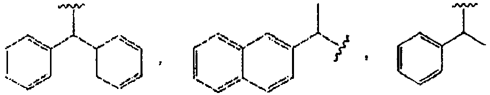 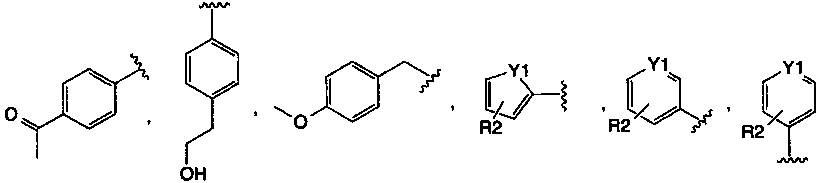
R₂ est indépendamment au moins un groupe parmi hydroxyle, hydrogène, fluoro, chloro ou bromo; et
dans les cycles à cinq chaînons, Y₁ est indépendamment CH₂, O, S ou NH, tandis que dans les cycles à six chaînons, Y₁ est indépendamment CH ou N.

5. Composé destiné à être utilisé selon l'une quelconque des revendications 1 à 4, ayant la formule: 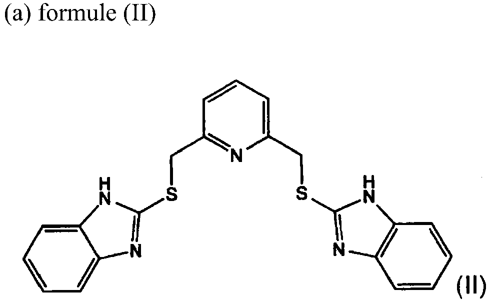
ou sel pharmaceutiquement acceptable de celui-ci, 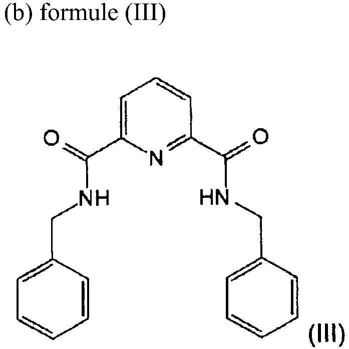
ou sel pharmaceutiquement acceptable de celui-ci; ou 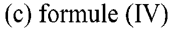 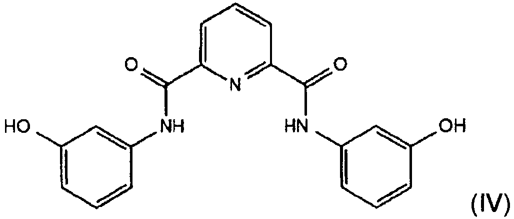
ou sel pharmaceutiquement acceptable de celui-ci.

6. Composé de formule II 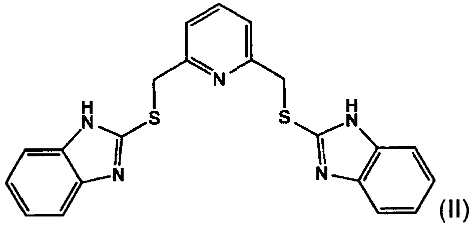
ou composé de formule III 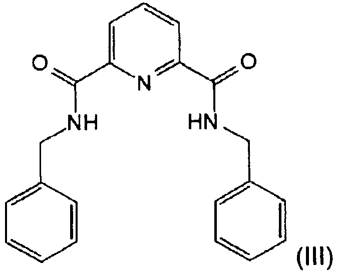
destiné à être utilisé dans le traitement du cancer.

7. Composé destiné à être utilisé selon l'une quelconque des revendications 1 à 6, destiné à être utilisé en association avec un ou plusieurs agents thérapeutiques supplémentaires, de préférence dans lequel les un ou plusieurs agents thérapeutiques supplémentaires sont choisis dans le groupe consistant en:
10-hydroxycamptothécine, 17-allylamino-geldanamycine, 2-méthoxy-antimycine A3, 3,4-dichloroisocoumarine, 4-hydroxyphénylrétinamide, acide 9-cis-rétinoïque, abiratérone, ado-trastuzumab, emtansine, adriamycine, afatinib, N-(3-chlorophényl)-6,7-diméthoxyquinazolin-4-amine, 2-amino-4-(1H-indol-5-yl)-1,1,3-tricyanobuta-1,3-diène, aldesleukine, alemtuzumab, amifostine, anastrozole, anisomycine, aphidicoline, trioxyde d'arsenic, asparaginase de Erwinia chrysanthemi, avastin, axitinib, sel trifluoroacétate de N-[2(S)-(2(R)-2-amino-3-mercaptopropylamino)-3-méthylbutyl]-L-phénylalanyl-L-méthionine, bacille de Calmette-Guérin, diglycidyléther de bisphénol A, bendamustine, bêta-lapachone, acide bétulinique, bévacizumab, bexarotène, bicalutamide, bisbenzimide, bléomycine, bortézomib, bosutinib, buséréline, busulfan, cabazitaxel, cabozantinib-S-malate, calpeptine, camptothécine, ester phénéthylique de l'acide caféique, capécitabine, caprelsa (vandétanib), carboplatine, carboplatine, carfilzomib, carmustine, cétuximab, chlorambucil, ciglitazone, cisplatine, clodronate, clofarabine, cometriq, crizotinib, curcumine, cyclo [Arg-Gly-Asp-D-Phe-Val], cycloheximide, cyclopamine, cyclophosphamide, cyclosporine A, cyprotérone, cytarabine, D12-prostaglandine J2, dabrafénib, dacarbazine, dactinomycine, dasatinib, daunorubicine, dégarélix, dénosumab, dexaméthasone, docétaxel, doxorubicine, ebselen, ellipticine, enzalutamide, épirubicine, erlotinib, étoposide, évérolimus, exémestane, fludarabine, fluorouracile, flutamide, acide folinique, fulvestrant, géfitinib, geldanamycine, gemcitabine, génistéine, gingérol, implant gliadel, gliotoxine, goséréline, 2-chloro-5-nitrobenzanilide, ester éthylique de l'acide 2-amino-6-bromo-α-cyano-3-(éthoxycarbonyl)-4H-1-benzopyrane-4-acétique, hinokitiol, hydroxyurée, sobuzoxane, idarubicine, ifosfamide, imatinib, indométacine, ipilimumab, irinotécan, ixabépilone, lanréotide, lapatinib, lénalidomide, létrozole, mésylate de lenvatinib, lenvima, leucovorine, leuprolide, lomustine, médroxyprogestérone, mégestrol, melphalan, mépesuccinate, mercaptopurine, mesna, méthotrexate, méthoxyvérapamil, carbobenzoxy-L-leucyl-L-leucyl-L-leucinal, mitomycine C, mitoxantrone, N,N-diméthylsphingosine, nélarabine, nilotinib, nivolumab, octréotide, ofatumumab, oligomycine A, omacétaxine, oxaliplatine, paclitaxel, pamidronate, panitumumab, pazopanib, pégaspargase, pémétrexed, pembrolizumab, pertuzumab, pifithrine, plérixafor, podophyllotoxine, pomalidomide, ponatinib, prednisone, 2,2-bis(hydroxyméthyl)-1-azabicyclo[2.2.2]octan-3-one, procarbazine, dichlorure de radium 223, raltitrexed, rapamycine, vaccin bivalent contre le virus du papillome humain (VPH) recombinant, vaccin quadrivalent contre le VPH recombinant, vaccin bivalent contre le VPH recombinant, vaccin, vaccin quadrivalent contre le VPH recombinant, interféron alpha-2b recombinant, régorafénib, resvératrol, acide rétinoïque tout-trans, rheumatrex, rituximab, rolipram, roscovitine, rottlerine, shikonine, sipuleucel-T, sirolimus, sorafénib, sphingosine, splitomycine, staurosporine, stilboestrol, streptozocine, 3-(4-diméthylaminobenzylidényl)-2-indolinone, 3-[[(4-diméthylamino)phényl]-méthylène]-1,3-dihydro-2H-indol-2-one, sulfure de sulindac, sunitinib, tamoxifène, témozolomide, temsirolimus, thalidomide, topotécan, torémifène, trametinib, trastuzumab, trichostatine A, trifluopérazine, acide 4-[(E)-2-(5,6,7,8-tétrahydro-5,5,8,8-tétraméthyl-2-naphtalényl)-1-propényl]benzoïque, 3,4-dihydroxy-α-cyanothiocinnamamide, (3-hydroxy-4-nitrobenzylidène)malono-nitrile, vandertanib, acide valproïque, vemurafenib, vérapamil, vinblastine, vincristine, vinorelbine, wortmannine, acide 4-chloro-6-(2,3-xylidino)-2-pyrimidinylthioacétique, ziv-aflibercept, acide zolédronique, leurs sels, et leurs combinaisons,
de préférence choisi dans le groupe consistant en: abiratérone, ado-trastuzumab, emtansine, afatinib, afinitor (évérolimus), anastrozole, avastin, bevacizumab, cabazitaxel, capécitabine, carboplatine, carmustine, cisplatine, crizotinib, cyclophosphamide, degarelix, denosumab, docétaxel, doxorubicine, enzalutamide, épirubicine, erlotinib, étoposide, évérolimus, exémestane, fluorouracile, fulvestrant, gefitinib, gemcitabine, ixabépilone, lapatinib, létrozole, leuprolide, lomustine, mégestrol, méthotrexate, mitomycine C, paclitaxel, pamidronate, pémétrexed, pertuzumab, prednisone, dichlorure de radium 223, sipuleucel-T, sunitinib, tamoxifène, témodar, témozolomide, topotécan, torémifène, trastuzumab, cabozantinib-S-malate, caprelsa (vandétanib), cometriq (cabozantinib-S-malate), chlorhydrate de doxorubicine, ipilimumab, mésylate de lenvatinib, nexavar (tosylate de sorafénib), nivolumab, vandétanib, yervoy (ipilimumab), leurs sels, et toute combinaison de ceux-ci.

8. Composés destinés à être utilisés selon la revendication 7, comprenant en outre un ou plusieurs agents thérapeutiques supplémentaires, qui sont choisis dans le groupe consistant en:
acide acétylsalicylique, diflunisal, salsalate, ibuprofène, dexibuprofène, naproxène, fénoprofène, kétoprofène, dexkétoprofène, flurbiprofène, oxaprozine, loxoprofène, indométacine, tolmétine, sulindac, étodolac, kétorolac, diclofénac, nabumétone, piroxicam, méloxicam, ténoxicam, droxicam, lomoxicam, isoxicam, acide méfénamique, acide méclofénamique, acide flufénamique, acide tolfénamique, célécoxib, rofécoxib, valdécosib, parécoxib, lumiracoxib, étoricoxib, firocoxib, nimésulide, licofélone, harpagide, clonixinate de lysine, leurs sels pharmaceutiquement acceptables, et leurs combinaisons, de préférence choisis dans le groupe consistant en célécoxib, rofécoxib, valdécosib, parécoxib, lumiracoxib, étoricoxib, firocoxib, leurs sels pharmaceutiquement acceptables, et leurs combinaisons.

9. Composé destiné à être utilisé selon l'une quelconque des revendications 1 à 8, dans lequel la maladie cancéreuse est choisie dans le groupe consistant en: la leucémie monocytaire aiguë, la leucémie myéloïde aiguë, la leucémie myélomonocytaire aiguë, la leucémie promyélocytaire aiguë, la leucémie à cellules T de l'adulte, le lymphome à cellules T de l'adulte, l'astrocytome, le cancer carcinoïde du poumon atypique, le carcinome basocellulaire, la leucémie lymphoblastique aiguë à cellules B, la leucémie/lymphome lymphoblastique aigu à cellules B, le cancer de la vessie, le cancer du cerveau, le cancer du sein, le cancer bronchique, le lymphome de Burkitt, le cancer des voies biliaires, le cancer primitif inconnu, le cancer du col de l'utérus, le syndrome myéloprolifératif chronique, le cancer du côlon, le lymphome diffus à grandes cellules, le cancer de l'endomètre, l'épendymome, le cancer de l'œsophage, le cancer de l'estomac, le gliome, le glioblastome, le cancer de la tête et du cou, l'hémagiopéricytome, le carcinome hépatocellulaire, le lymphome de Hodgkin, le sarcome de Kaposi, le cancer du rein, le carcinome neuroendocrinien à grandes cellules, la leucémie à grands lymphocytes granuleux, la leucémie, le cancer du foie, le cancer du poumon, le lymphome, le médulloblastome, le mélanome, le myélome multiple, le syndrome myélodysplasique, le cancer du nasopharynx, le neuroblastome, la tumeur à cellules NK, le lymphome non hodgkinien, le carcinome épidermoïde de l'œsophage, l'ostéosarcome, le cancer de l'ovaire, le cancer du pancréas, la leucémie à cellules T périphérique, la leucémie à plasmocytes primitive, le cancer de la prostate, le carcinome à cellules claires du rein, le rétinoblastome, le rhabdomyosarcome, le sarcome, carcinome pulmonaire à petites cellules, la leucémie lymphoblastique aiguë à cellules T, le lymphome lymphoblastique aigu à cellules T, le cancer du testicule, le thymome, le cancer de la thyroïde, le cancer de l'ouraque, le cancer de l'utérus, le cancer du vagin, et leurs combinaisons,
de préférence choisie dans le groupe consistant en la leucémie lymphoblastique aiguë à cellules B, le lymphome de Burkitt, le lymphome diffus à grandes cellules, le myélome multiple, la leucémie à plasmocytes primitive, le cancer carcinoïde du poumon atypique, le cancer de la vessie, le cancer du cerveau, le cancer du sein, le cancer du col de l'utérus, le cancer du côlon, le cancer de l'endomètre, le cancer de l'estomac, le glioblastome, le cancer de la tête et du cou, le carcinome hépatocellulaire, le carcinome neuroendocrinien à grandes cellules, le cancer du foie, le médulloblastome, le mélanome, le neuroblastome, le carcinome épidermoïde de l'œsophage, l'ostéosarcome, le cancer de l'ovaire, le cancer de l'hypophyse, le cancer de la prostate, le carcinome à cellules claires du rein, le rétinoblastome, le rhabdomyosarcome, le carcinome pulmonaire à petites cellules, l'adénocarcinome du côlon, l'adénocarcinome du poumon, l'adénocarcinome de la prostate, l'adénocarcinome de l'ouraque, l'adénocarcinome du vagin, l'adénocarcinome du sein, l'adénocarcinome de l'œsophage, l'adénocarcinome bronchique, l'adénocarcinome du pancréas, l'adénocarcinome gastro-intestinal, l'adénocarcinome de l'endomètre, l'adénocarcinome de l'ovaire, l'adénocarcinome de l'estomac, l'adénocarcinome du foie, l'adénocarcinome de la thyroïde, l'adénocarcinome de la tête et du cou, l'adénocarcinome de la vessie et l'adénocarcinome gastro-intestinal; et leurs combinaisons, de préférence dans lequel la maladie cancéreuse est l'adénocarcinome canalaire du pancréas, l'adénocarcinome du sein, l'adénocarcinome de la prostate, ou l'adénocarcinome du poumon, l'adénocarcinome de l'endomètre, l'adénocarcinome de l'ovaire, l'adénocarcinome de l'estomac, l'adénocarcinome du foie, l'adénocarcinome de la thyroïde, l'adénocarcinome de la tête et du cou, l'adénocarcinome de la vessie, ou l'adénocarcinome gastro-intestinal et leurs combinaisons.
